# DEMANDE DE BREVET EUROPEEN

(11) **EP 4 011 932 A1**
(43) Date de publication de la demande: **15.06.2022**
(21) Numéro de dépôt: 20306561.0
(22) Date de dépôt: 14.12.2020
(51) Int. Cl.: C08G 63/02, C08G 69/44, C08G 18/42

(54) **COMPOSÉ POLYMÈRE À PEPTIDES PENDANTS, PROCÉDÉ DE PRÉPARATION ET UTILISATIONS DE CELUI-CI**

(71) Demandeur: Genepep, 34430 Saint-Jean-de-Vedas (FR)
(72) Inventeur: JEBORS, Saïd, 34070 MONTPELLIER (FR); PUGET, Karine, 34430 SAINT JEAN DE VEDAS (FR)
(74) Mandataire: Cabinet Becker et Associés

(57) **Abrégé**

La présente invention concerne un composé polymère à peptides pendants, un procédé de préparation de celui-ci et son utilisation dans la fabrication d'un matériau ou d'un article.

## Description

### OBJET DE L'INVENTION

La présente invention concerne un composé polymère à peptides pendants, un procédé de préparation de celui-ci et son utilisation dans la fabrication d'un matériau ou d'un article.

### ARRIERE-PLAN DE L'INVENTION

Les polyesters et les polyamides font partie des polymères les plus utilisés dans la vie quotidienne. Ces polymères que l'on rencontre principalement sous la forme de fibres, démontrent notamment une grande solidité, résistance et stabilité, des propriétés qui sont essentielles dans bon nombre d'applications. Pour conférer certaines propriétés requises pour des applications spécifiques, il est généralement nécessaire d'insérer des groupements fonctionnels particuliers, parfois également en des positions particulières du polymère et des proportions particulières. Notamment, dans le domaine biomédical, la formation de polymères modifiés par l'introduction de molécules bioactives a suscité au cours de ces dernières années l'intérêt des scientifiques. En fonction du procédé mis au point, les peptides ajoutés au polymère peuvent être incorporés au sein même de la structure du polymère, ou bien seulement être adsorbés à la surface de celui-ci.

Par exemple, le brevet US 7709601 décrit un procédé dans lequel la fonctionnalisation du polymère par les peptides a lieu après la polymérisation, et il en résulte alors une adsorption des peptides à la surface du polymère.

La demande de brevet WO 2017/098018 décrit un procédé de polymérisation interfaciale d'un di-halogénure d'acyle avec un ou plusieurs acides aminés ou peptides jouant le rôle d'une diamine, ou bien avec une diamine substituée par un peptide. Des polyamides ayant des peptides incorporés au sein même du squelette polymérique ou bien pendants, et ayant des extrémités - CO₂H et/ou -NH₂ sont obtenus par un tel procédé. Ce type de polymère est particulièrement utile pour fabriquer des biomatériaux ayant notamment des propriétés antibactériennes. Il s'avère cependant qu'un tel procédé de polymérisation interfaciale ne permet pas d'accéder à une grande diversité de polymères, et est difficile à mettre en œuvre d'un point de vue industriel. Il subsiste donc un réel besoin de développer de nouveaux polymères portant des peptides, et pouvant être obtenus par un procédé simple permettant un bon contrôle de la répartition des peptides le long de la chaîne polymère.

### RESUME DE L'INVENTION

Dans ce contexte, les inventeurs ont développé un composé polymère, sur lequel pendent un ou plusieurs peptides. La structure du composé polymère peut être assimilée à une structure en peigne, dans laquelle le squelette polymérique est linéaire et constitué de motifs répétitifs, et chaque « branche » ou « dent » rattachée au squelette est constituée d'un peptide. Le procédé mis au point par les inventeurs consistent à mélanger, en milieu fondu, des monomères substitués par un peptide, et des monomères non substitués, dans des conditions permettant une répartition homogène et/ou contrôlée des peptides pendants sur le polymère ainsi obtenu. Une grande diversité de polymères a pu être préparée par ce procédé, en particulier des polyamides, des polyesters et des polyuréthanes. Le composé polymère obtenu a démontré son intérêt comme composant utilisé dans la fabrication d'un (bio)matériau ou d'un article, ayant notamment des propriétés antibactériennes.

L'invention a donc pour objet un composé polymère comprenant :
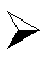 des motifs répétitifs de formule (I) : 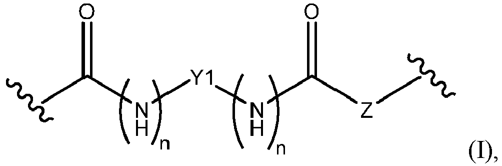 dans lequel
   ∘ n vaut 0 ou 1 ;
   ∘ Y1 représente un radical choisi parmi un (C₁-C₁₂)alkyle, un aryle et un aryle-(Ci-C₁₂)alkyle-aryle ; et
   ∘ Z représente un radical choisi parmi :
      ▪ un groupe représenté par la formule (la) suivante : 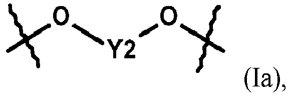 dans lequel Y2 représente un (C₁-C₁₂)alkyle, aryle, ou polyalkylène glycol ; et
      ▪ un groupe représenté par la formule (Ib) suivante : 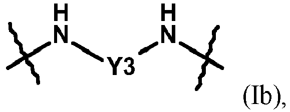
   dans lequel Y3 représente un (C₁-C₁₂)alkyle, aryle ou polyalkylène glycol ;
   et dans lequel au moins un de Y1, Y2, et Y3 est relié à un résidu peptidique via un résidu fonctionnel, le résidu fonctionnel et le résidu peptidique étant éventuellement séparés par un fragment espaceur ;
   ; et 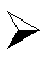
des motifs répétitifs de formule (II) : 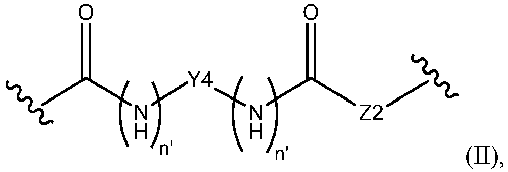 dans lequel
   ∘ n' vaut 0 ou 1 ;
   ∘ Y4 représente un radical choisi parmi un (C₁-C₁₂)alkyle, un aryle et un aryle-(C₁-C₁₂)alkyle-aryle ; et
   ∘ Z2 représente un radical choisi parmi :
      ▪ un groupe représenté par la formule (IIa) suivante : 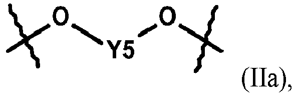 dans lequel Y5 représente un (C₁-C₁₂)alkyle, aryle, ou polyalkylène glycol ; et
      ▪ un groupe représenté par la formule (IIb) suivante : 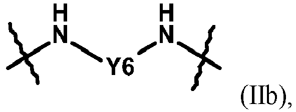 dans lequel Y6 représente un (C₁-C₁₂)alkyle, aryle, ou polyalkylène glycol,
         sous réserve que, lorsque n et n' valent 0 et que Y3 est relié à un résidu peptidique via un résidu fonctionnel, le polymère présente au moins une extrémité de formule (E10) ou (E40) : 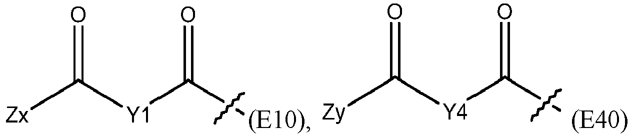 dans lesquelles, Zx et Zy représentent indépendamment un (C₁-C₁₂)alkyle-O- ou aryle-O-.

Elle a également pour objet un composé monomère représenté par la formule (III-1) suivante : 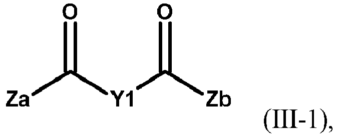 dans lequel
∘ Y1 représente un radical choisi parmi un (C₁-C₁₂)alkyle, un aryle, et un aryle-(C₁-C₁₂)alkyle-aryle ;
∘ Za représente un radical choisi parmi :
   ▪ un (C₁-C₁₂)alkyle-O-, un aryle-O-, un chlore ; et,
   ▪ un groupe représenté par la formule (IIIa) suivante : 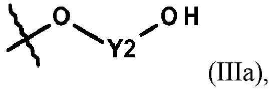 dans lequel Y2 représente un (C₁-C₁₂)alkyle, aryle, ou polyalkylène glycol ; et
∘ Zb représente un radical choisi parmi :
   ▪ un (C₁-C₁₂)alkyle-O-, un aryle-O-, un chlore ;
   ▪ un groupe représenté par la formule (IIIa) suivante : 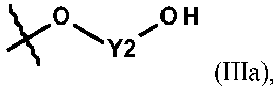 dans lequel Y2 représente un (C₁-C₁₂)alkyle, aryle ou polyalkylène glycol ; et
   ▪ un groupe représenté par la formule (IIIb) suivante : 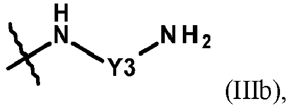 dans lequel Y3 représente un (C₁-C₁₂)alkyle, aryle, ou polyalkylène glycol ;
   et dans lequel au moins un de Y1, Y2, et Y3 est relié à un résidu peptidique via un résidu fonctionnel, le résidu fonctionnel et le résidu peptidique étant éventuellement séparés par un fragment espaceur,
   sous réserve que, lorsque Y3 est relié à un résidu peptidique via un résidu fonctionnel, Za est choisi parmi un (C₁-C₁₂)alkyle-O- et un aryle-O-.

Elle a également pour objet un composé monomère représenté par la formule (111-2) suivante :

HO-Y2-OH (III-2),

dans lequel Y2 représente un (C₁-C₁₂)alkyle, aryle ou polyalkylène glycol, où Y2 est relié à un résidu peptidique via un résidu fonctionnel, le résidu fonctionnel et le résidu peptidique étant éventuellement séparés par un fragment espaceur.

Elle a encore pour objet un procédé de préparation d'un composé polymère tel que défini ci-dessus, comprenant une réaction de polycondensation en milieu fondu, mettant enjeu :
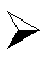 au moins un composé monomère choisi parmi :
   ∘ un composé monomère de formule Za-C(O)-Y1-C(O)-Zb, où :
      - Y1 représente un radical choisi parmi un (C₁-C₁₂)alkyle, un aryle et un aryle-(C₁-C₁₂)alkyle-aryle ;
      - Za représente un radical choisi parmi un (C₁-C₁₂)alkyle-O-, un aryle-O-, un chlore, et un groupe de formule (IIIa) où Y2 représente un (C₁-C₁₂)alkyle, aryle, ou polyalkylène glycol,
      - Zb représente un radical choisi parmi un (C₁-C₁₂)alkyle-O-, un aryle-O-, un chlore, un groupe de formule (IIIa) où Y2 représente un (C₁-C₁₂)alkyle, aryle, ou polyalkylène glycol, et un groupe de formule (IIIb) où Y3 représente un (C₁-C₁₂)alkyle, aryle, ou polyalkylène glycol ;
   ∘ OCN-Y1-NCO, où Y1 représente un (C₁-C₁₂)alkyle, aryle, ou un aryle-(C₁-C₁₂)alkyle-aryle,
   ∘ HO-Y2-OH où Y2 représente un (C₁-C₁₂)alkyle, aryle, ou polyalkylène glycol, et
   ∘ H₂N-Y3-NH₂ où Y3 représente un (C₁-C₁₂)alkyle, aryle, ou polyalkylène glycol,
   où au moins un de Y1, Y2, et Y3 est relié à un résidu peptidique via un résidu fonctionnel, le résidu fonctionnel et le résidu peptidique étant éventuellement séparés par un fragment espaceur;
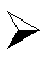 et au moins un composé monomère choisi parmi :
   ∘ un composé monomère représenté par la formule (V) suivante : 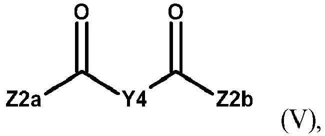 dans lequel
      - Y4 représente un radical choisi parmi un (C₁-C₁₂)alkyle, un aryle, et un aryle-(C₁-C₁₂)alkyle-aryle ;
      - Z2a représente un radical choisi parmi :
         ▪ un (C₁-C₁₂)alkyle-O-, un aryle-O-, un chlore ; et
         ▪ un groupe représenté par la formule (Va) suivante : 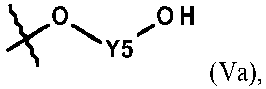 dans lequel Y5 représente un (C₁-C₁₂)alkyle, aryle ou polyalkylène glycol ; et
      - Z2b représente un radical choisi parmi :
         ▪ un (C₁-C₁₂)alkyle-O-, un aryle-O-, un chlore ;
         ▪ un groupe représenté par la formule (Va) suivante : 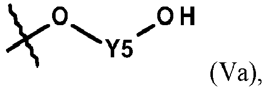 dans lequel Y5 représente un (C₁-C₁₂)alkyle, aryle ou polyalkylène glycol ; et
         ▪ un groupe représenté par la formule (Vb) suivante : 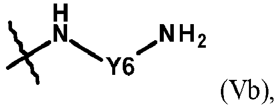 dans lequel Y6 représente un (C₁-C₁₂)alkyle, aryle, ou polyalkylène glycol,
   ∘ OCN-Y4-NCO, où Y4 représente un (C₁-C₁₂)alkyle, aryle ou aryle-(C₁-C₁₂)alkyle-aryle,
   ∘ HO-Y5-OH, où Y5 représente un (C₁-C₁₂)alkyle, aryle, ou polyalkylène glycol, et
   ∘ H₂N-Y6-NH₂, où Y6 représente un (C₁-C₁₂)alkyle, aryle, ou polyalkylène glycol.

Elle concerne également une utilisation d'un composé polymère tel que défini ci-dessus, dans la fabrication d'un matériau ou d'un article.

Elle concerne en outre un matériau comprenant un composé polymère tel que défini ci-dessus.

Elle concerne en outre un article comprenant un composé polymère tel que défini ci-dessus.

### DESCRIPTION DES FIGURES

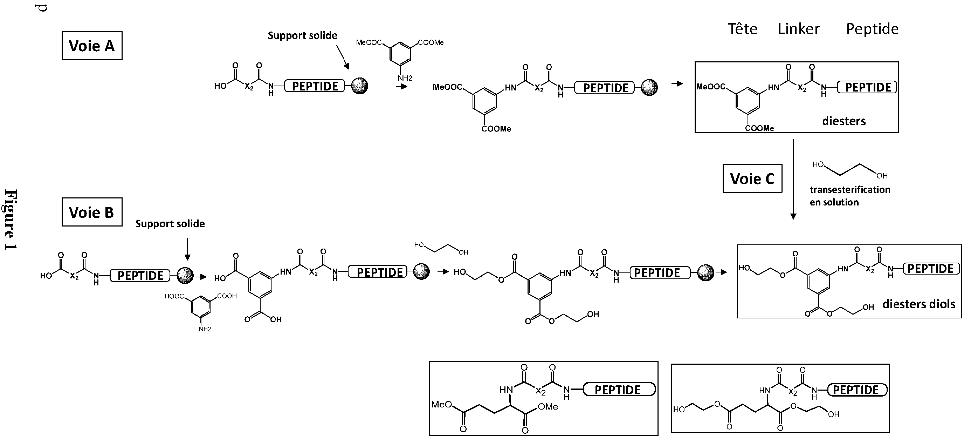

La Figure 1 représente un schéma de différentes synthèses de monomères diméthyl isophthalate substitués par un peptide (Voie A) ou bis(2-hydroxyéthyl) isophthalate substitués par un peptide (Voies B et C) (X2 = (C₁-C₁₂)alkyle, aryle, ou polyalkylène glycol).
La Figure 2 représente un schéma de différentes synthèses de monomères de type amino ester monoamide substitués par un peptide (X2 = (C₁-C₁₂)alkyle, aryle, ou polyalkylène glycol).
La Figure 3 représente un schéma de synthèse sur résine 2-chlorotrityle de monomères de type amino ester monoamide substitués par un peptide.
La Figure 4 représente un schéma illustrant les différentes étapes d'un test antibactérien (norme ISO 22196:2011) sur un échantillon de 5 cm x 5 cm.
La Figure 5 représente le pourcentage de bactéries mesuré par le test antibactérien (norme ISO 22196:2011) avec le poly(butylène adipate-co-téréphthalate) (PBAT) et le poly(butylène adipate-co-téréphthalate-co-isophthalatopeptide) (PBAT-co-peptide).

### DESCRIPTION DETAILLEE

### Définitions :

Par "alkyle", on entend un groupe hydrocarboné aliphatique saturé, linéaire ou ramifié. L'expression « (C₁-C₁₂)alkyle » désigne un alkyle renfermant de 1 à 12 atomes de carbone, notamment un groupe méthyle, éthyle, propyle, isopropyle, butyle, isobutyle, pentyle, hexyle, heptyle, octyle, nonyle, décyle, undécyle, ou dodécyle. Des exemples de groupements (C₁-C₁₂)alkyle préférés sont notamment des groupes (C₁-C₆)alkyle tels que les groupements méthyle, éthyle, propyle, butyle, pentyle, et hexyle. De préférence, un groupe (C₁-C₁₂)alkyle est un groupe (C₁-C₆)alkyle.

Par "aryle", on entend un groupe carbocyclique aromatique de 6 à 14 chaînons, mono- ou polycyclique. Des exemples de groupements aryle sont le phényle et le naphtyle, de préférence le phényle.

Par « aryle-(C₁-C₁₂)alkyle-aryle », on entend un groupe divalent constitué d'un alkyle ayant 1 à 12 atomes de carbone rattaché à deux aryles, où chaque liaison avec lesdits aryles implique le même carbone ou deux carbones différents de l'alkyle et où chacun des deux aryles est rattaché au reste de la molécule. Un aryle-(C₁-C₁₂)alkyle-aryle est de préférence un aryle-(C₁-C₆)alkyle-aryle tel que le groupe phényle-méthyle-phényle, qui peut en particulier être représenté comme suit :

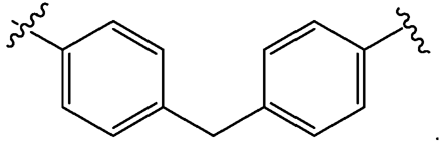

Par « polyalkylène glycol » (ou « polyoxyalkylène »), on entend un groupe d'oligomère ou polymère d'un alkylène glycol, tel que le polyéthylène glycol, le polypropylène glycol, ou polybutylène glycol. En particulier, le groupe polyalkylène glycol est de formule -[(CHR⁵)ᵢ-O]ₖ-(CHR⁵)ᵢ- où :
- chaque R⁵ est indépendamment un hydrogène ou un alkyle en C₁-C₆ (de préférence un hydrogène),
- i est un entier de 1 à 12 (de préférence de 2 à 6), et k est un entier de 1 à 250 (de préférence de 2 à 50, mieux encore de 2 à 20).
Le groupe polyéthylène glycol est en particulier de formule -[(CH₂)₂-O]ₖ-(CH₂)₂- avec k un entier de 1 à 250 (de préférence de 2 à 50, mieux encore de 2 à 20).
Le groupe polypropylène glycol est en particulier de formule -[CH(CH₃)-CH₂-O]ₖ-CH(CH₃)-CH₂- avec k un entier de 1 à 250 (de préférence de 2 à 50, mieux encore de 2 à 20).
Le groupe polybutylène glycol (ou polytétrahydrofurane ou polytétraméthylène glycol) est en particulier de formule -[(CH₂)₄-O]ₖ-(CH₂)₄- avec k un entier de 1 à 250 (de préférence de 2 à 50, mieux encore de 2 à 20).

Les groupes alkyle et aryle tels que définis ci-dessus peuvent également être mono ou poly substitués par des groupes incluant notamment les alkyles, les cycloalkyles, hétérocycloalkyles, les aryles, les hétéroaryles, les perfluoroalkyles, les alcoxy, les alkylthio, les alkylamino, les halogènes, un cyano, un nitro, etc.
Par « cycloalkyle », on entend un groupe alkyle mono-, bi- ou tri-cyclique, ponté ou non. Des exemples de cycloalkyle sont notamment cyclopropyle, cyclobutyle, cyclopentyle, cyclohexyle, cycloheptyle, cyclooctyle, cyclodécyle, ou cyclododécyle.
Par « hétérocycloalkyle », on entend un groupe hydrocarboné, mono-, bi- ou tri-cyclique, ponté ou non, saturé ou insaturé, et comprenant au moins un hétéroatome, tel que N (azacycloalkyle), O (oxacycloalkyle) ou S (thiacycloalkyle). Des exemples de hétérocycloalkyle sont notamment un oxirane, oxétane, 3-dioxolane, benzo-[1,3]-dioxolyle, pyrazolinyle, pyranyle, thiomorpholinyle, pyrazolidinyle, pipéridyle, pipérazinyle, 1,4-dioxanyle, imidazolinyle, pyrrolinyle, pyrrolidinyle, pipéridinyle, imidazolidinyle, morpholinyle, 1,4-dithianyle, pyrrolidinyle, quinolizinyle, oxozolinyle, oxazolidinyle, isoxazolinyle, isoxazolidinyle, thiazolinyle, thiazolidinyle, isothiazolinyle, isothiazolidinyle, dihydropyranyle, ou tétrahydrofuranyle..
Par « hétéroaryle », on entend un groupe aromatique monocyclique ou polycyclique, comprenant au moins un hétéroatome tel que l'azote, l'oxygène ou le soufre. Particulièrement, un hétéroaryle désigne un pyridinyle, thiazolyle, thiophényle, furanyle, pyrrolyle, imidazolyle, triazolyle, tétrazolyle, benzofuranyle, thianaphthalényle, indolyle, indolinyle, quinolinyle, isoquinolinyle, benzimidazolyle, triazinyle, thianthrényle, isobenzofuranyle, phénoxanthinyle, isothiazolyle, isoxazolyle, pyrazinyle, pyridazinyle, indolizinyle, isoindolyle, indazolyle, purinyle, phtalazinyle, naphthyridinyle, quinoxalinyle, quinazolinyle, cinnolinyle, ptéridinyle, carbazolyle, β-carbolinyle, phénanthridinyle, acridinyle, pyrimidinyle, phénanthrolinyle, phénazinyle, phénothiazinyle, furazanyle, phenoxazinyle, benzotriazolyle, benzoxazolyle, benzisoxazolyle, oxindolyle, benzothiényle, benzothiazolyle, s-triazinyle, oxazolyle, ou thiofuranyle.
Par « alkyloxy » ou « alcoxy », on entend un groupe -O-alkyle où le groupe alkyle est tel que défini ci-dessus. Des exemples d'alcoxy sont notamment un groupe méthoxy, éthoxy, propyle, isopropoxy, butoxy, *iso*-butoxy, *tert*-butoxy, pentoxy, ou hexyloxy.
Par « alkylthio », on entend un groupe -S-(alkyle), où le groupe alkyle est tel que défini ci-dessus. Un exemple d'alkylthio est notamment un méthylthio.
Par « alkylamino », on entend un groupe -NH-(alkyle) ou -N(alkyle)₂ où le groupe alkyle est tel que défini ci-dessus. Des exemples d'alkylamino sont notamment méthylamino, éthylamino, ou diméthylamino.
Par « perfluoroalkyle », on entend un groupe alkyle dans lequel les hydrogènes ont été remplacés par un fluor. Un exemple de perfluoroalkyle est notamment CF₃.
Par « halogène », on entend un atome de fluor, chlore, brome ou iode.

Par « solvant », on entend aussi bien un solvant organique qu'un solvant inorganique, ou encore un mélange de ceux-ci. Des exemples de solvants organiques sont notamment, les hydrocarbures aliphatiques, cycliques ou acycliques, tels que le cyclohexane ou le pentane, les hydrocarbures aromatiques tels que le benzène, le toluène ou le xylène, les hydrocarbures halogénés tels que le dichlorométhane, le chloroforme ou le chlorobenzène, les solvants azotés tels que l'acétonitrile ou la triéthylamine, les solvants oxygénés, en particulier les cétones telles que l'acétone, les éthers tels que le diéthyléther, le tert-butylméthyléther (TBME), le tétrahydrofurane (THF), les alcools tels que le méthanol ou l'éthanol, les esters tels que l'acétate d'éthyle, ou les amides tels que le diméthylformamide (DMF), le diméthyl acétamide (DMAc), les solvants soufrés tels que le diméthylsulfoxyde (DMSO), et un mélange de ceux-ci. Un exemple de solvant inorganique est notamment l'eau.

### COMPOSE POLYMERE

L'invention a pour objet un composé polymère comprenant (de préférence, constitué) :
> des motifs répétitifs de formule (I) : 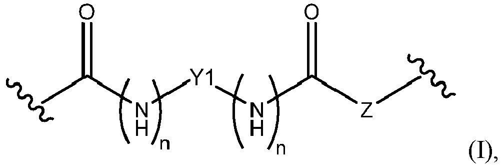 dans lequel
   ∘ n vaut 0 ou 1 ;
   ∘ Y1 représente un radical choisi parmi un (C₁-C₁₂)alkyle, un aryle, et un aryle-(C₁-C₁₂)alkyle-aryle ; et
   ∘ Z représente un radical choisi parmi :
      ▪ un groupe représenté par la formule (la) suivante : 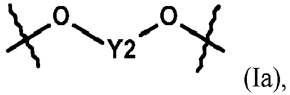 dans lequel Y2 représente un (C₁-C₁₂)alkyle, aryle ou polyalkylène glycol ; et
      ▪ un groupe représenté par la formule (Ib) suivante : 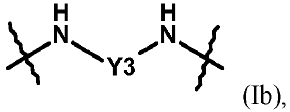 dans lequel Y3 représente un (C₁-C₁₂)alkyle, aryle, ou polyalkylène glycol (de préférence un (C₁-C₁₂)alkyle ou aryle) ; et dans lequel au moins un de Y1, Y2, et Y3 est relié à un résidu peptidique via un résidu fonctionnel, le résidu fonctionnel et le résidu peptidique étant éventuellement séparés par un fragment espaceur ;
         ; et 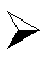
des motifs répétitifs de formule (II) : 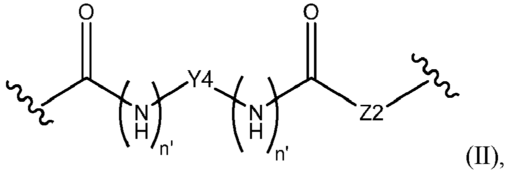 dans lequel
   ∘ n' vaut 0 ou 1 ;
   ∘ Y4 représente un radical choisi parmi un (C₁-C₁₂)alkyle, un aryle, et un aryle-(C₁-C₁₂)alkyle-aryle ; et
   ∘ Z2 représente un radical choisi parmi :
      ▪ un groupe représenté par la formule (IIa) suivante : 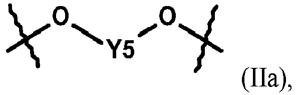 dans lequel Y5 représente un (C₁-C₁₂)alkyle, aryle ou polyalkylène glycol ; et
      ▪ un groupe représenté par la formule (IIb) suivante : 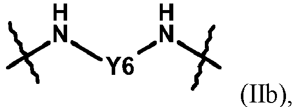 dans lequel Y6 représente un (C₁-C₁₂)alkyle, aryle, ou polyalkylène glycol (de préférence, un (C₁-C₁₂)alkyle ou aryle),
         sous réserve que, lorsque n et n' valent 0 et que Y3 est relié à un résidu peptidique via un résidu fonctionnel, le polymère présente au moins une extrémité de formule (E10) ou (E40) : 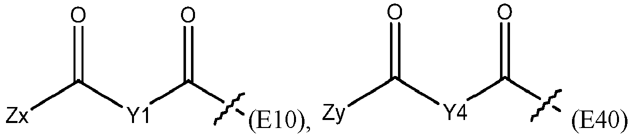 dans lesquelles, Zx et Zy représentent indépendamment un (C₁-C₁₂)alkyle-O- ou aryle-O-.

Le composé polymère selon l'invention comprend (de préférence, est constitué) des motifs répétitifs dont une partie est substitué par des peptides (motifs de formule (I)). La structure du composé polymère peut être assimilée à une structure en peigne, dans laquelle le squelette polymérique est linéaire et constitué de motifs répétitifs, et chaque « branche » ou « dent » rattachée au squelette est constituée d'un peptide. On peut ainsi parler d'un composé polymère à peptides pendants.

Plus particulièrement, le composé polymère est de type polyester, polyamide, polyuréthane, ou une combinaison de ceux-ci.

Les motifs répétitifs de formule (I) du composé polymère de l'invention peuvent être identiques ou différents les uns des autres.
Les motifs répétitifs de formule (II) du composé polymère de l'invention peuvent être identiques ou différents les uns des autres.

Dans la présente invention, les indices n d'un motif répétitif de formule (I) sont identiques entre eux. De la même manière, les indices n' d'un motif répétitif de formule (II) sont identiques entre eux. De préférence, les indices n et n', respectivement des motifs répétitifs de formule (I) et (II) du composé polymère, sont identiques entre eux.
Dans un mode de réalisation particulier, le composé polymère comprend (de préférence, est constitué) des motifs répétitifs de formule (I) dans lequel n = 0 et des motifs répétitifs de formule (II) dans lequel n'= 0.
Dans un autre mode de réalisation particulier, le composé polymère comprend (de préférence, est constitué) des motifs répétitifs de formule (I) dans lequel n = 1 et des motifs répétitifs de formule (II) dans lequel n' = 1.

Dans certains modes de réalisation, d'autres motifs répétitifs peuvent être présents dans le composé polymère de l'invention. De préférence, les motifs répétitifs de formule (I) et (II) représentent au moins 30 %, au moins 40 %, au moins 50 %, au moins 60 %, au moins 70 %, au moins 80 %, au moins 90 %, au moins 95 % en nombre par rapport au nombre total de motifs répétitifs constituant le composé polymère de l'invention. Dans un mode particulier, le composé polymère de l'invention n'est constitué que de motifs répétitifs de formule (I) et (II), c'est-à-dire que les motifs répétitifs de formule (I) et (II) représentent 100 % en nombre par rapport au nombre total de motifs répétitifs constituant le composé polymère de l'invention.

Les différentes caractéristiques physico-chimiques du composé polymère selon l'invention, telles que la masse molaire moyenne en nombre ou en masse, la polydispersité, la composition chimique, la pureté, la structure chimique, peuvent être déterminées par différentes techniques bien connues de l'homme de l'art, telles que la calorimétrie différentielle à balayage, l'analyse élémentaire, la chromatographie d'exclusion stérique, la spectrométrie de masse, et/ou la spectroscopie RMN

Par « composé polymère », on entend une ou plusieurs macromolécules (ou chaîne polymère), identiques ou différentes, comprenant des motifs répétitifs (ou unités monomériques) reliés les uns aux autres par des liaisons covalentes, et de manière linéaire, de telle sorte que ces motifs forment une chaîne.

Le symbole 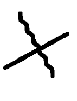 représente le point d'attache entre deux motifs ou deux fragments de motifs dans la chaîne polymère, ou plus généralement le point d'attache entre deux groupes moléculaires. Ce point d'attache se traduit physiquement par une liaison covalente.

Les motifs répétitifs représentés avec un symbole 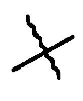 à chaque extrémité s'enchaînent de la gauche vers la droite. L'extrémité gauche d'un motif donné est reliée à l'extrémité droite du motif adjacent précédent dans la chaîne polymère et l'extrémité droite du motif donné est reliée à l'extrémité gauche du motif adjacent suivant dans la chaîne polymère.

Selon un mode de réalisation particulier, le rapport du nombre de motifs répétitifs de formule (I) au nombre total de motifs répétitifs (formule (I) et formule (II)) est compris entre 0,0001/100 et 50/100, de préférence entre 0,001/100 et 10/100, mieux encore entre 0,005/100 et 1/100, voire entre 0,0075/100 et 0,1/100.

Selon un mode de réalisation particulier, la masse molaire moyenne en nombre du composé polymère selon l'invention est comprise entre 1000 et 150000 g/mol, de préférence entre 5000 et 15000 g/mol.
Selon un mode de réalisation particulier, la masse molaire moyenne en masse du composé polymère selon l'invention est comprise entre 1000 et 250000 g/mol, de préférence entre 5000 et 35000 g/mol.
La masse molaire en masse ou en nombre peut en particulier être déterminée par chromatographie par exclusion stérique, ou tout autre technique adaptée connue de l'homme du métier.

Selon un mode de réalisation particulier, la polydispersité du composé polymère selon l'invention est comprise entre 1 et 5, de préférence entre 1 et 3, mieux encore entre 1 et 2.

Au sein d'une même chaîne du composé polymère selon l'invention, les motifs répétitifs de formule (I), les motifs répétitifs de formule (II) (et les éventuels autres motifs répétitifs) peuvent notamment s'enchaîner :
- de manière aléatoire : la probabilité de trouver un motif répétitif de formule (I) ou (II) en un point donné de la chaîne est indépendante de la nature des motifs adjacents. Un exemple de chaîne ou partie de chaîne aléatoire peut notamment être -A-A-B-A-B-A-A-B-B-B-A-B-B-A- ;
- de manière périodique et, en particulier, de manière alternée : les motifs répétitifs de formule (I) et (II) s'enchaînent selon un ordre séquentiel régulier, par exemple en alternance. Un exemple de chaîne ou partie de chaîne périodique peut notamment être -A-B-A-B-A-B-A-B-A-B-; ou
- par blocs. Un exemple de chaîne ou partie de chaîne en blocs peut notamment être -A-A-A-A-A-A-A-B-B-B-B-B-B-B-B-;
où A représente un motif répétitif de formule (I) et B représente un motif répétitif de formule (II).

Il est bien entendu que, lorsque le composé polymère comprend plusieurs chaînes, les motifs répétitifs de formule (I) et de formule (II) peuvent s'enchaîner de manière aléatoire au sein d'une même chaîne, mais également d'une chaîne à l'autre, de telle sorte que la répartition des motifs répétitifs de formule (I) et de formule (II) entre les chaînes est aléatoire.

Chaque chaîne du composé polymère selon l'invention présente deux extrémités. Selon un mode particulier, chaque extrémité de chaque chaîne est représentée indépendamment par une formule choisie parmi les formules (E1) à (E6) suivantes : 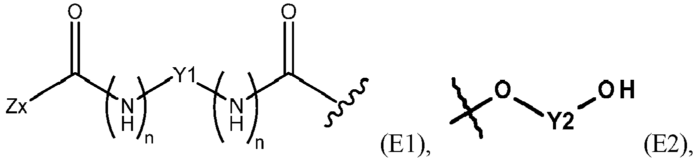 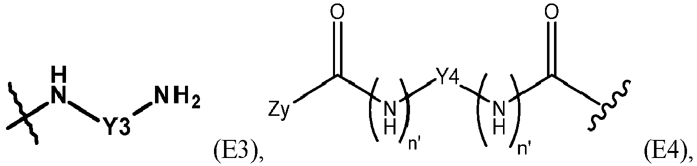 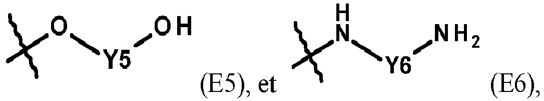 dans lesquelles, n, n', Y1, Y2, Y3, Y4, Y5, et Y6 sont tels que définis ci-dessus et, Zx et Zy représentent indépendamment un hydroxy, un chlore, un (C₁-C₁₂)alkyle-O- ou un aryle-O- (de préférence, un chlore, un (C₁-C₁₂)alkyle-O- ou un aryle-O-, mieux encore un (C₁-C₁₂)alkyle-O- ou un aryle-O), et une forme salifiée de celles-ci.

Il est bien entendu que les extrémités de chaîne polymère telles que représentées par les formules (E1), (E2), (E3), (E4), (E5), (E6), (E10) et (E40), peuvent inclure un fragment de motif répétitif de formule (I) ou (II) constituant la chaîne polymère.

Par « forme salifiée », on entend des sels d'acides ou basiques du composé ou groupe spécifié. Les sels d'acide comprennent, mais ne sont pas limités à, des sels de chlorhydrate, bromhydrate, iodhydrate, nitrate, sulfate, bisulfate, phosphate, phosphate acide, isonicotinate, acétate, lactate, salicylate, citrate, tartrate, pantothénate, bitartrate, ascorbate, succinate, maléate, gentisinate, fumarate, gluconate, glucaronate, saccharate, formate, benzoate, glutamate, méthanesulfonate, éthanesulfonate, benzènesulfonate, et para-toluènesulfonate. Des sels de base comprennent, mais ne sont pas limités à, des sels d'aluminium, calcium, lithium, magnésium, potassium, sodium, zinc, et diéthanolamine.

Selon un mode de réalisation dans lequel Y3 est relié à un résidu peptidique via un résidu fonctionnel, le composé polymère selon l'invention présente au moins une extrémité (en particulier, constitué d'au moins une chaîne ayant au moins une de ses deux extrémités, et de préférence, chaque chaîne ayant au moins une de ses deux extrémités) de formule (E10) ou (E40) : 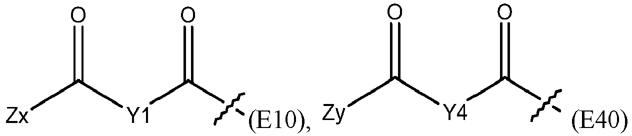 dans lesquelles, Y1 et Y4 sont tels que définis ci-dessus et, Zx et Zy représentent indépendamment un (C₁-C₁₂)alkyle-O- ou aryle-O-.

Dans le motif de formule (I), au moins un de Y1, Y2, et Y3 est relié à un résidu peptidique via un résidu fonctionnel, le résidu fonctionnel et le résidu peptidique étant éventuellement séparés par un fragment espaceur.

On entend par « résidu peptidique », un peptide dépourvu d'un ou plusieurs atomes ou groupe(s) d'atomes (par exemple, -H ou -OH) d'un de ses groupes fonctionnels. Le résidu peptidique résulte typiquement de la réaction de ce groupe fonctionnel avec une fonction de couplage reliée au reste du motif de formule (I) (ou un espaceur), plus particulièrement reliée à au moins un de Y1, Y2, et Y3.
Des exemples de groupes fonctionnels de peptide sont notamment -NH₂ ou -CO₂H.
Les groupes fonctionnels du résidu peptidique sont typiquement -NH- et -CO-.

On entend par « résidu fonctionnel », une fonction de couplage reliée au reste du motif de formule (I), plus particulièrement reliée à au moins un de Y1, Y2, et Y3, et dépourvue d'un ou plusieurs atomes ou groupe(s) d'atomes (par exemple, -H ou -OH). Le résidu fonctionnel résulte typiquement de la réaction de cette fonction de couplage avec un groupe fonctionnel d'un peptide (ou d'un espaceur).
Des exemples de fonctions de couplage sont notamment -NH₂ ou -CO₂H.
Les résidus fonctionnels sont typiquement -NH- et -CO-.

On entend par « fragment espaceur », un fragment chimique qui sépare le motif répétitif de formule (I), plus précisément le résidu fonctionnel, et le résidu peptidique. Ce fragment est dépourvu d'un ou plusieurs atomes ou groupe(s) d'atomes (par exemple, -H ou -OH) au niveau d'une ou plusieurs de ces groupes fonctionnels. Le fragment espaceur résulte de la réaction entre d'un groupe fonctionnel d'un espaceur avec une fonction de couplage et d'un autre groupe fonctionnel de cet espaceur avec un peptide.

Selon une première forme d'exécution de l'invention, le résidu fonctionnel et le résidu peptidique (ou plus précisément le groupe fonctionnel du résidu peptidique) ne sont pas séparés par un fragment espaceur. Dans une telle forme d'exécution, la fonction de couplage et le groupe fonctionnel d'un peptide sont avantageusement choisis de sorte qu'ils aient une réactivité complémentaire. Par exemple,
- lorsque la fonction de couplage est un groupe -NH₂, alors le groupe fonctionnel du peptide est avantageusement -CO₂H ou un dérivé de ce groupe (ester, chlorure d'acyle...), réagissant ainsi pour former un fragment -(résidu fonctionnel)-(groupe fonctionnel du résidu peptidique)- sous la forme d'un amide (-NH-CO-) ; et
- lorsque la fonction de couplage est un groupe -CO₂H ou un dérivé de ce groupe (ester, chlorure d'acyle...), alors le groupe fonctionnel du peptide est avantageusement -NH₂, réagissant ainsi pour former un fragment -(résidu fonctionnel)-(groupe fonctionnel du résidu peptidique)- sous la forme d'un amide (-CO-NH-).

De manière préférée, le résidu fonctionnel et le groupe fonctionnel du résidu peptidique sont respectivement -CO- et -NH-, ou -NH- et -CO-, formant ainsi un amide (-CO-NH- ou -NH-CO-).

Selon une deuxième forme d'exécution de l'invention, le résidu fonctionnel et le résidu peptidique (ou plus précisément un groupe fonctionnel du résidu peptidique) sont séparés par un fragment espaceur. Dans une telle forme d'exécution :
- la fonction de couplage réagit avec un premier groupement fonctionnel d'un espaceur (il résulte que le résidu fonctionnel est relié au fragment espaceur), et
- un groupe fonctionnel du peptide réagit avec un second groupement fonctionnel de l'espaceur (il résulte que le résidu peptidique est relié au fragment espaceur).
Les différents groupes ou résidus fonctionnels sont choisis de sorte qu'ils aient une réactivité complémentaire.
Par exemple :
- lorsque la fonction de couplage ou le groupe fonctionnel du peptide est un groupe -NH₂, alors le groupement fonctionnel de l'espaceur est avantageusement -CO₂H ou un dérivé de -CO₂H (ester, chlorure d'acyle...), réagissant ainsi pour former un amide (-NH-CO-) ; et
- lorsque la fonction de couplage ou le groupe fonctionnel du peptide est un groupe -CO₂H ou un dérivé de -CO₂H (ester, chlorure d'acyle...), alors le groupe fonctionnel de l'espaceur est avantageusement -NH₂, réagissant ainsi pour former un amide (-CO-NH-).

Selon un mode de réalisation particulier, le fragment espaceur est de formule -(X1)-(X2)-(X3)-, dans laquelle X1 et X3 représentent chacun indépendamment -NH- ou -CO-, et X2 représente un (C₁-C₁₂)alkyle, aryle ou polyalkylène glycol.

Selon un mode particulier, X1 et X3 représentent -CO-. Dans un tel mode de réalisation, le résidu fonctionnel et le groupe fonctionnel du résidu peptidique sont avantageusement -NH-. Selon un autre mode particulier, X1 représente -NH- et X3 représente -CO-. Dans un tel mode de réalisation, le résidu fonctionnel et le groupe fonctionnel du résidu peptidique sont avantageusement -CO- et -NH-, respectivement. On considère ici arbitrairement que X1 est relié au résidu fonctionnel et X3 est relié au résidu peptidique.

Selon un autre mode particulier, X2 représente un (C₁-C₆)alkyle, mieux encore un méthyle, un propyle (e.g. 1,3-propyle) ou un pentyle (e.g. 1,5-pentyle).

Selon un mode de réalisation particulier, Y1 et/ou Y3 sont reliés à un résidu peptidique via un résidu fonctionnel, le résidu fonctionnel et le résidu peptidique étant éventuellement séparés par un fragment espaceur.

Selon un autre mode de réalisation particulier, Y2 est relié à un résidu peptidique via un résidu fonctionnel, le résidu fonctionnel et le résidu peptidique étant éventuellement séparés par un fragment espaceur.

Selon un mode de réalisation préféré, Y1 est relié à un résidu peptidique via un résidu fonctionnel, le résidu fonctionnel et le résidu peptidique étant éventuellement séparés par un fragment espaceur.

Selon un autre mode de réalisation préféré, Y3 est relié à un résidu peptidique via un résidu fonctionnel, le résidu fonctionnel et le résidu peptidique étant éventuellement séparés par un fragment espaceur.

Selon un mode de réalisation particulier, Y1 est choisi parmi un éthyle, un propyle, un butyle, un pentyle, un hexyle, un phényle, et un phényle-méthyle-phényle.
Selon un autre mode de réalisation particulier, Y2 est choisi parmi un éthyle, un propyle, un butyle, un pentyle, un hexyle, un phényle, un polyéthylène glycol, polypropylène glycol, et un polybutylène glycol.
Selon un autre mode de réalisation particulier, Y3 est choisi parmi un éthyle, un propyle, un butyle, un pentyle, un hexyle, un phényle, un polyéthylène glycol, polypropylène glycol, et un polybutylène glycol.
Selon un autre mode de réalisation particulier, Y4 est choisi parmi un éthyle, un propyle, un butyle, un pentyle, un hexyle, un phényle, et un phényle-méthyle-phényle.
Selon un autre mode de réalisation particulier, Y5 est choisi parmi un éthyle, un propyle, un butyle, un pentyle, un hexyle, un phényle, un polyéthylène glycol, un polypropylène glycol et un polybutylène glycol.

Selon un autre mode de réalisation particulier, Y6 est choisi parmi un éthyle, un propyle, un butyle, un pentyle, un hexyle, un phényle, un polyéthylène glycol, polypropylène glycol, et un polybutylène glycol.

Les groupes Y1 à Y6 sont des groupes divalents, c'est-à-dire qu'ils sont rattachés au reste de la molécule par deux liaisons, chaque liaison impliquant le même carbone (dudit groupe divalent) ou deux carbones différents (dudit groupe divalent).

De manière préférée, un groupe divalent de type « alkyle » est rattaché au reste de la molécule par les carbones aux extrémités de la chaîne alkyle.

Un groupe divalent de type « propyle » inclut notamment le 1,1-propyle, le 1,2-propyle, et le 1,3-propyle, et de préférence le 1,3-propyle.

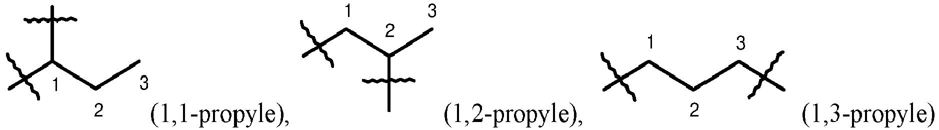

Un groupe divalent de type « éthyle » préféré est le 1,2-éthyle.
Un groupe divalent de type« butyle » préféré est le 1,4-butyle.
Un groupe divalent de type « pentyle » préféré est le 1,5-pentyle.
Un groupe divalent de type« hexyle » préféré est le 1,6-hexyle.

Un groupe divalent de type« phényle » inclut notamment le 1,2-phényle, 1,3-phényle et le 1,4-phényle, de manière préférée le 1,3-phényle et le 1,4 phényle, et mieux encore, le 1,4 phényle.

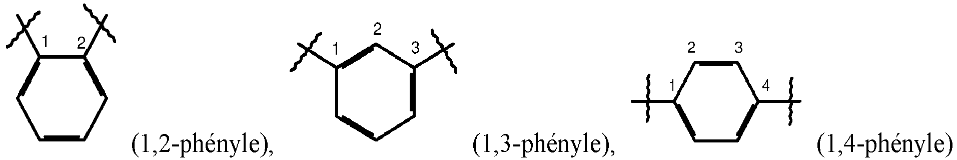

De manière préférée, un groupe divalent de type « polyalkylène glycol » est rattaché au reste de la molécule par les atomes aux extrémités de la chaîne polyalkylène glycol.

Selon un mode de réalisation particulier, Y1 est choisi parmi un 1,2-éthyle, un 1,3-propyle, un 1,4-butyle, un 1,5-pentyle, un 1,6-hexyle, un 1,3-phényle, un 1,4-phényle, et un phényle-méthyle-phényle.
Selon un autre mode de réalisation particulier, Y2 est choisi parmi un 1,2-éthyle, un 1,3-propyle, un 1,4-butyle, un 1,5-pentyle, un 1,6-hexyle, un 1,3-phényle, un 1,4-phényle, un polyéthylène glycol, un polypropylène glycol et un polybutylène glycol.
Selon un autre mode de réalisation particulier, Y3 est choisi parmi un 1,2-éthyle, un 1,3-propyle, un 1,4-butyle, un 1,5-pentyle, un 1,6-hexyle, un 1,3-phényle, un 1,4-phényle, un polyéthylène glycol, un polypropylène glycol et un polybutylène glycol.
Selon un autre mode de réalisation particulier, Y4 est choisi parmi un 1,2-éthyle, un 1,3-propyle, un 1,4-butyle, un 1,5-pentyle, un 1,6-hexyle, un 1,3-phényle, un 1,4-phényle, et un phényle-méthyle-phényle.
Selon un autre mode de réalisation particulier, Y5 est choisi parmi un 1,2-éthyle, un 1,3-propyle, un 1,4-butyle, un 1,5-pentyle, un 1,6-hexyle, un 1,3-phényle, un 1,4-phényle, un polyéthylène glycol, un polypropylène glycol et un polybutylène glycol.
Selon un autre mode de réalisation particulier, Y6 est choisi parmi un 1,2-éthyle, un 1,3-propyle, un 1,4-butyle, un 1,5-pentyle, un 1,6-hexyle, un 1,3-phényle, un 1,4-phényle, un polyéthylène glycol, un polypropylène glycol et un polybutylène glycol.

Le résidu fonctionnel (ou la fonction de couplage) peut être rattaché en n'importe quelle position dudit groupe divalent Y1, Y2, ou Y3, formant ainsi une structure « Y-(résidu fonctionnel) », où Y est au moins un de Y1, Y2 ou Y3 relié à un résidu peptidique via le résidu fonctionnel (et éventuellement un fragment espaceur).

Des exemples de structures « Y-(résidu fonctionnel) », sont notamment : 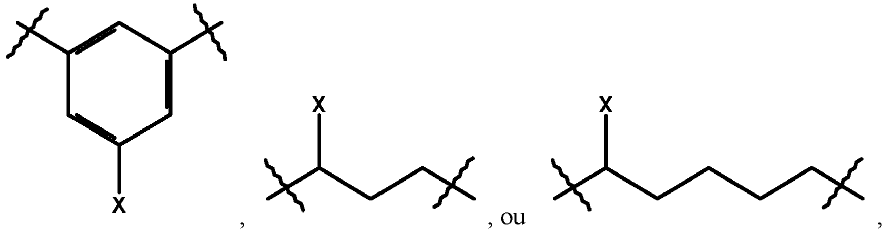 où X représente le résidu fonctionnel, notamment -CO- ou -NH-.

Selon un mode réalisation où Y est Y1, la structure « Y-(résidu fonctionnel)» est avantageusement 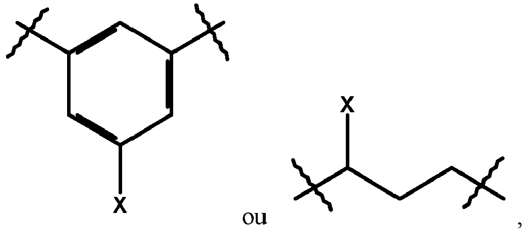 où X représente le résidu fonctionnel -CO- ou -NH-, de préférence -NH-. Dans un tel mode de réalisation, on préfère que la structure « Y-(résidu fonctionnel) » soit : 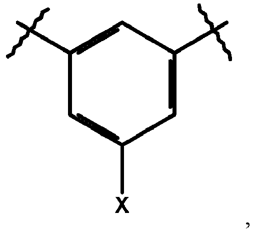 où X représente le résidu fonctionnel, notamment -CO- ou -NH-, de préférence -NH-.

Selon un mode réalisation où Y est Y3, la structure « Y-(résidu fonctionnel) » est avantageusement : 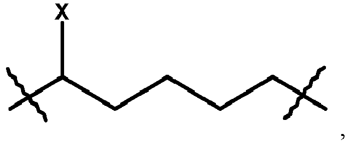 où X représente le résidu fonctionnel -CO- ou -NH-, de préférence -CO-.

Selon un mode de réalisation particulier, Y1 et Y4 sont identiques.
Selon un autre mode de réalisation particulier, Y2 et Y5 sont identiques.
Selon un autre mode de réalisation particulier, Y3 et Y6 sont identiques.

### Peptide

Par « peptide », on entend un homo- ou hétéro- oligomère ou polymère d'acides aminés reliés entre eux par des liaisons peptidiques (c'est-à-dire des liaisons de type amide). Plus particulièrement, le peptide peut répondre à la formule suivante : 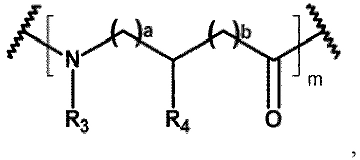 dans laquelle,
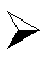 m est un entier compris entre 2 et 200, plus particulièrement entre 2 et 50, encore plus particulièrement entre 2 et 10 ;
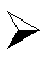 R3 représente un atome d'hydrogène, ou un (C₁-C₆)alkyle éventuellement substitué par un aryle ;
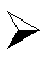 R4 représente une chaîne latérale d'acide aminé, notamment d'acide aminé naturel, d'acide aminé non naturel, d'acide aminé modifié ou protégé ;
   ou, R3 et R4, pris ensemble avec le groupe -N-(CH₂)ₐ-CH- auquel ils sont rattachés, peuvent former un cycle ; et
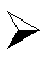 a et b sont des entiers choisis de telle sorte que la somme (a+b) est un entier de 0 à 10.

Selon un mode de réalisation particulier, le peptide est une protéine.
Selon un autre mode de réalisation particulier, le peptide est un seul acide aminé (naturel ou non naturel, modifié ou non modifié).

On préfère que ledit peptide soit un peptide bioactif.

En particulier, le peptide bioactif peut être un peptide antimicrobien, un peptide favorisant l'adhésion et/ou la prolifération cellulaire, un peptides anti-inflammatoire, pro-cicatriciel, ou favorisant l'ostéo-intégration.

Des exemples de tels peptides sont notamment RGD, DGEA, GHK, les peptides magainines, les peptides de séquence BBXB où B est un acide aminé basique et X est un acide aminé non basique, les peptides de séquence pro-collagéniques de type Gly-Pro-X ou Gly-X-Hyp (où X désigne n'importe quel acide aminé), des peptides dont la séquence est issue de la Ténascine X.

De préférence, ledit peptide est un peptide antimicrobien, et plus particulièrement un peptide antimicrobien cationique. Un peptide antimicrobien (ou peptide antimicrobien cationique) comprend généralement des acides aminés chargés positivement, tels que l'arginine ou la lysine, et des acides aminés hydrophobes, tels que la phénylalanine, la leucine ou l'isoleucine. Un tel peptide peut comprendre en outre d'autres acides aminés.

Dans un mode de réalisation particulier, le peptide est choisi parmi Gly-Phe-Arg, Arg-Phe (également noté RF), Phe-Arg (également noté FR), Arg-Phe-Arg-Phe (également noté RFRF ou (RF)₂), Phe-Arg-Phe-Arg (également noté FRFR ou (FR)₂), Arg-Phe-Arg-Phe-Arg-Phe (également noté RFRFRF ou (RF)₃), Phe-Arg-Phe-Arg-Phe-Arg (également noté FRFRFR ou (FR)₃), Arg-Phe-Arg-Phe-Arg-Phe-Arg-Phe (également noté RFRFRFRF ou (RF)₄), Phe-Arg-Phe-Arg-Phe-Arg-Phe-Arg (également noté FRFRFRFR ou (FR)₄), et Arg-Arg-Palm (également noté RR-Palm ou (R)₂-Palm).

Dans un mode de réalisation préféré, le peptide est Arg-Phe-Arg-Phe-Arg-Phe-Arg-Phe, Phe-Arg-Phe-Arg-Phe-Arg-Phe-Arg ou Arg-Arg-Palm. Plus préférentiellement, le peptide est Arg-Phe-Arg-Phe-Arg-Phe-Arg-Phe, ou Phe-Arg-Phe-Arg-Phe-Arg-Phe-Arg. Mieux encore, le peptide est Arg-Phe-Arg-Phe-Arg-Phe-Arg-Phe.

L'abréviation à 3 lettres « Gly » ou à une lettre « G » correspond à la glycine. L'abréviation à 3 lettres « Phe » ou à une lettre « F » correspond à la phénylalanine. L'abréviation à 3 lettres « Arg » ou à une lettre « R » correspond à l'arginine. L'abréviation à 3 lettres « Lys » ou à une lettre « K » correspond à la lysine. L'abréviation à 3 lettres « Asp » ou à une lettre « D » correspond à l'acide aspartique (ou aspartate). L'abréviation à 3 lettres « Ala » ou à une lettre « A » correspond à l'alanine. L'abréviation à 3 lettres « Glu » ou à une lettre « E » correspond à l'acide glutamique (ou glutamate). L'abréviation à 3 lettres « His » ou à une lettre « H » correspond à l'histidine. L'abréviation à 3 lettres « Pro » ou à une lettre « P » correspond à la proline. L'abréviation à 3 lettres « Hyp » correspond à l'hydroxyproline. L'abréviation « Palm » correspond à un résidu palmityle (pouvant être représenté par la formule -(CH₂)₁₅-CH₃).

Pour les peptides décrits dans la présente demande, la convention d'écriture des séquences place l'extrémité C-terminale à la droite, la séquence étant écrite de gauche à droite, de l'extrémité N-terminale à l'extrémité C-terminale.

Dans le peptide Arg-Arg-Palm, le résidu palmityle est typiquement relié au C-terminale de Arg par une fonction amine. Le peptide Arg-Arg-Palm peut être représenté comme suit :

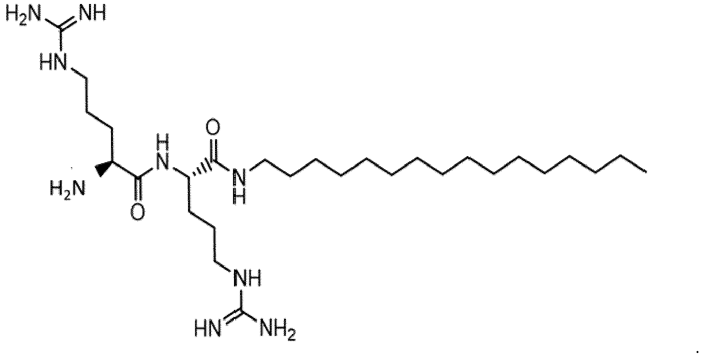

Dans le peptide Arg-Phe-Arg-Phe-Arg-Phe-Arg-Phe, l'extrémité C-terminale peut en particulier être reliée à une fonction amine. Le peptide Arg-Phe-Arg-Phe-Arg-Phe-Arg-Phe peut en particulier être représenté comme suit :

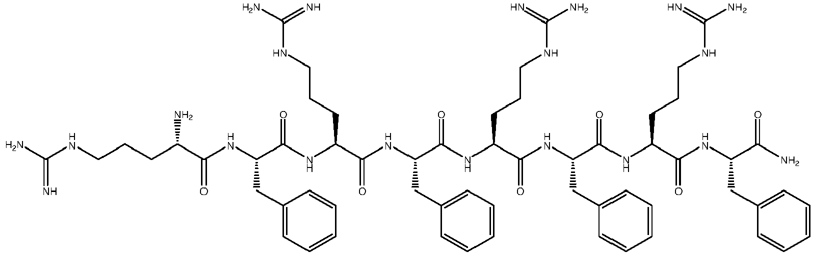

Dans le peptide Phe-Arg-Phe-Arg-Phe-Arg-Phe-Arg, l'extrémité N-terminale peut en particulier être reliée à une fonction acétyle. Le peptide Phe-Arg-Phe-Arg-Phe-Arg-Phe-Arg peut en particulier être représenté comme suit :

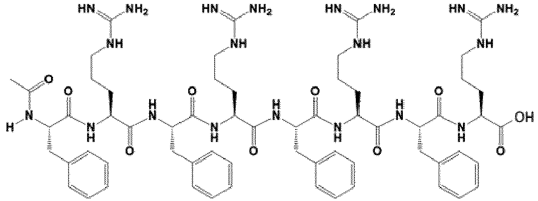

Le peptide, ou un résidu peptidique de celui-ci, peut être relié au monomère ou polymère de l'invention (éventuellement via un fragment espaceur) par son extrémité N-terminale, C-terminale, ou par n'importe quel autre groupement fonctionnel porté par le peptide. De préférence, le peptide, ou un résidu peptidique de celui-ci, est relié au monomère ou polymère de l'invention (éventuellement via un fragment espaceur) par son extrémité N-terminale ou C-terminale, mieux encore par son extrémité N-terminale.

Dans un mode particulier, le peptide Arg-Phe-Arg-Phe-Arg-Phe-Arg-Phe ou un résidu peptidique de celui-ci, est relié au monomère ou polymère de l'invention (éventuellement via un fragment espaceur) par son extrémité N-terminale.
Dans un autre mode particulier, le peptide Phe-Arg-Phe-Arg-Phe-Arg-Phe-Arg ou un résidu peptidique de celui-ci, est relié au monomère ou polymère de l'invention (éventuellement via un fragment espaceur) par son extrémité C-terminale.

Le peptide peut être préparé par toute technique connue de l'homme du métier. Avantageusement, le peptide est préparé par synthèse peptidique sur support solide. Le support solide peut notamment être une silice, une alumine, du verre, une résine de type polystyrène, une résine de type polyéthylène glycol. Le support solide peut être un support fonctionnalisé par un groupe chimique adapté à la synthèse peptidique, par exemple une résine fonctionnalisée par un groupe 4-((2,4-diméthoxyphényl)(Fmoc-amino)méthyl)phénoxyalkyle (e.g. Résine Rink-Amide^{®}) ou par un groupe chlorure de 2-chlorotrityle. En variante, le peptide peut être synthétisé en solution. Les peptides décrits dans le présente demande peuvent être sous une forme protégée ou non (de préférence non protégée). Les groupements protecteurs des peptides sont bien connus de l'homme du métier, et sont par exemple le groupe Pbf (pentaméthylbenzofuranylsulfonyle), Boc (tert-butoxycarbonyle), ou Fmoc (fluorénylméthoxycarbonyle).

### Polyesters

Selon un mode de réalisation particulier, le composé polymère de l'invention est un polyester comprenant (de préférence constitué) des motifs répétitifs de formule (I) dans laquelle :
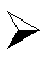 n=0;
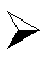 Z est un groupe représenté par la formule (la) suivante : 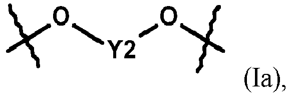 dans lequel Y2 représente un (C₁-C₁₂)alkyle, aryle ou polyalkylène glycol ; et
et des motifs répétitifs de formule (II) dans laquelle :
> n'=0;
> Z2 est un groupe représenté par la formule (IIa) suivante : 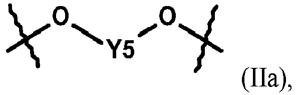 dans lequel Y5 représente un (C₁-C₁₂)alkyle, aryle ou polyalkylène glycol,
   et dans lequel au moins un de Y1 et Y2, de préférence Y1, est relié à un résidu peptidique via un résidu fonctionnel, le résidu fonctionnel et le résidu peptidique étant éventuellement séparés par un fragment espaceur.

Dans un tel mode de réalisation, le résidu peptidique est avantageusement un résidu du peptide Arg-Phe-Arg-Phe-Arg-Phe-Arg-Phe, Phe-Arg-Phe-Arg-Phe-Arg-Phe-Arg, ou Arg-Arg-Palm, mieux encore Arg-Phe-Arg-Phe-Arg-Phe-Arg-Phe.

Selon un mode de réalisation particulier, Y1, Y2, Y4 et Y5 sont choisis indépendamment parmi un (C₁-C₆)alkyle et un phényle.
Selon un mode de réalisation plus particulier, Y1, Y2, Y4 et Y5 sont choisis indépendamment parmi un éthyle, un propyle, un butyle, un pentyle, un hexyle, et un phényle, de préférence parmi un 1,2-éthyle, 1,3-propyle, 1,4-butyle, 1,5-pentyle, 1,6-hexyle, 1,2-phényle, 1,3-phényle et 1,4-phényle. Selon un mode de réalisation préféré, Y1 représente un phényle (notamment un 1,3-phényle) ou un propyle (notamment un 1,3-propyle).
Selon un autre mode de réalisation préféré, la structure « Y-(résidu fonctionnel) », où Y est Y1 et/ou Y2, de préférence Y1, est : 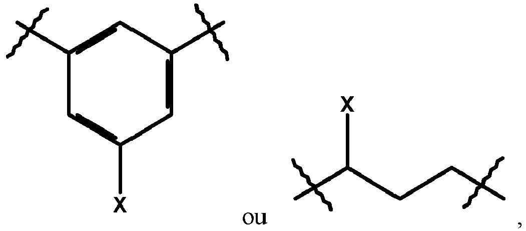 où X représente le résidu fonctionnel, notamment -CO- ou -NH-, de préférence -NH-, qui lui-même est relié au résidu peptidique, le résidu fonctionnel et le résidu peptidique étant éventuellement séparés par un fragment espaceur.

Dans un mode de réalisation particulier, Y2 et Y5 sont identiques. On préfère que Y2 et Y5 représentent un (C₁-C₆)alkyle, plus préférentiellement un 1,2-éthyle ou un 1,4-butyle, mieux encore un 1,4-butyle.

Dans un mode de réalisation préféré, le composé polymère de l'invention comprend :
- des motifs répétitifs de formule (I) dans lequel n=n'=0, Y1 représente un 1,3-propyle ou 1,3-phényle (de préférence, un 1,3-phényle) et Z est un groupe de formule (la) dans lequel Y2 représente un 1,2-éthyle ou 1,4-butyle (de préférence un 1,4-butyle); où Y1 est relié à un résidu peptidique via un résidu fonctionnel, le résidu fonctionnel et le résidu peptidique étant éventuellement séparés par un fragment espaceur ;
- des motifs répétitifs de formule (II) dans lequel Y4 est un 1,2-éthyle ou un 1,4-butyle (de préférence un 1,4-butyle), et Z2 est un groupe de formule (IIa) dans lequel Y5 représente un 1,2-éthyle ou 1,4-butyle (de préférence un 1,4-butyle) ; et
- éventuellement, des motifs répétitifs de formule (II) dans lequel Y4 est un 1,4-phényle, et Z2 est un groupe de formule (IIa) dans lequel Y5 représente un 1,2-éthyle ou 1,4-butyle (de préférence un 1,4-butyle).
Dans un tel mode de réalisation préféré, le résidu fonctionnel est avantageusement -NH-, et le fragment espaceur est avantageusement de formule -(X1)-(X2)-(X3)- où X1 et X3 représentent -CO- et X2 est un (C₁-C₆)alkyle, tel qu'un 1,3-propyle.
Dans un tel mode de réalisation préféré, le résidu peptidique est avantageusement un résidu du peptide Arg-Phe-Arg-Phe-Arg-Phe-Arg-Phe, Phe-Arg-Phe-Arg-Phe-Arg-Phe-Arg, ou Arg-Arg-Palm, mieux encore Arg-Phe-Arg-Phe-Arg-Phe-Arg-Phe.

### Polyamides

Selon un autre mode de réalisation particulier, le composé polymère de l'invention est un polyamide comprenant (de préférence, constitué) des motifs répétitifs de formule (I) dans laquelle :
∘ n=0;
∘ Z est un groupe représenté par la formule (Ib) suivante : 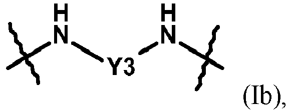 dans lequel Y3 représente un (C₁-C₁₂)alkyle, aryle, ou polyalkylène glycol (de préférence, un (C₁-C₁₂)alkyle, ou aryle) ;
et des motifs répétitifs de formule (II) dans laquelle :
∘ n' =0
∘ Z2 est un groupe représenté par la formule (IIb) suivante : 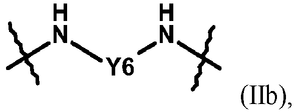 dans lequel Y6 représente un (C₁-C₁₂)alkyle, aryle, ou polyalkylène glycol (de préférence, un (C₁-C₁₂)alkyle ou aryle), et
   dans lequel au moins un de Y1 et Y3 est relié à un résidu peptidique via un résidu fonctionnel, le résidu fonctionnel et le résidu peptidique étant éventuellement séparés par un fragment espaceur.
Dans un tel mode de réalisation, le résidu peptidique est avantageusement un résidu du peptide Arg-Phe-Arg-Phe-Arg-Phe-Arg-Phe, Phe-Arg-Phe-Arg-Phe-Arg-Phe-Arg, ou Arg-Arg-Palm, mieux encore Arg-Phe-Arg-Phe-Arg-Phe-Arg-Phe ou Phe-Arg-Phe-Arg-Phe-Arg-Phe-Arg.

Selon un mode de réalisation particulier, Y1, Y3, Y4 et Y6 sont choisis indépendamment parmi un (C₁-C₆)alkyle et un phényle.
Selon un mode de réalisation plus particulier, Y1, Y3, Y4 et Y6 sont choisis indépendamment parmi un éthyle, un propyle, un butyle, un pentyle, un hexyle, et un phényle, de préférence parmi un 1,2-éthyle, 1,3-propyle, 1,4-butyle, 1,5-pentyle, 1,6-hexyle, 1,2-phényle, 1,3-phényle et 1,4-phényle.

Selon un mode de réalisation préféré, Y1 est relié à un résidu peptidique via un résidu fonctionnel, le résidu fonctionnel et le résidu peptidique étant éventuellement séparés par un fragment espaceur.
Selon ce mode préféré, la structure « Y-(résidu fonctionnel) », où Y est Y1, est avantageusement : 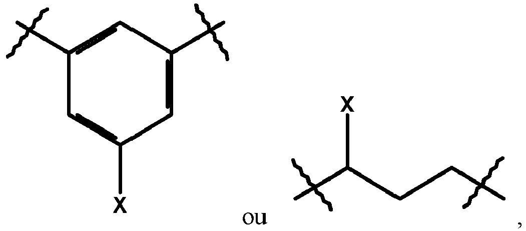 où X représente le résidu fonctionnel, notamment -CO- ou -NH-, de préférence NH, et de préférence, 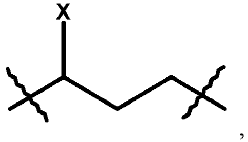 où X représente le résidu fonctionnel -NH-.

Selon un autre mode de réalisation préféré, Y3 est relié à un résidu peptidique via un résidu fonctionnel, le résidu fonctionnel et le résidu peptidique étant éventuellement séparés par un fragment espaceur.
Selon ce mode préféré, la structure « Y-(résidu fonctionnel) », où Y est Y3, est avantageusement : 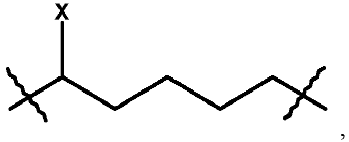 où X représente le résidu fonctionnel -CO- ou -NH-, de préférence -CO-.
Selon ce mode préféré, le composé polymère qui est un polyamide présente au moins une extrémité (en particulier, constitué d'au moins une chaîne ayant au moins une de ses deux extrémités, et de préférence, chaque chaîne ayant au moins une de ses deux extrémités) de formule (E10) ou (E40) : 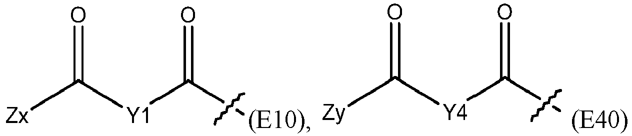 dans lesquelles, Y1 et Y4 sont tels que définis ci-dessus et, Zx et Zy représentent indépendamment un (C₁-C₁₂)alkyle-O- ou aryle-O-.

Dans un mode de réalisation particulier, Y3 et Y6 sont identiques. On préfère que Y3 et Y6 représentent un (C₁-C₆)alkyle.

### Polyuréthanes

Selon un autre mode de réalisation particulier, le composé polymère de l'invention est un polyuréthane comprenant (de préférence, constitué) des motifs répétitifs de formule (I) dans laquelle :
∘ n = 1 ;
∘ Y1 représente un radical choisi parmi un (C₁-C₁₂)alkyle, un aryle, et un aryle-(Ci-C₁₂)alkyle-aryle ; et
∘ Z représente un radical choisi parmi :
   ▪ un groupe représenté par la formule (la) suivante : 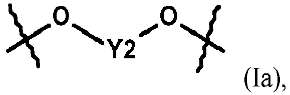 dans lequel Y2 représente un (C₁-C₁₂)alkyle, aryle ou polyalkylène glycol ; et
   ▪ un groupe représenté par la formule (Ib) suivante : 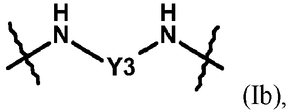 dans lequel Y3 représente un (C₁-C₁₂)alkyle, aryle, ou polyalkylène glycol ;
      dans lequel au moins un de Y2 et Y3 est relié à un résidu peptidique via un résidu fonctionnel, le résidu fonctionnel et le résidu peptidique étant éventuellement séparés par un fragment espaceur;
      ; et 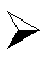
   des motifs répétitifs de formule (II) dans lequel :
∘ n' = 1 ;
∘ Y4 représente un radical choisi parmi un (C₁-C₁₂)alkyle, un aryle, et un aryle-(C₁-C₁₂)alkyle-aryle ; et
∘ Z2 représente un radical choisi parmi :
   ▪ un groupe représenté par la formule (IIa) suivante : 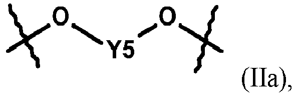 dans lequel Y5 représente un (C₁-C₁₂)alkyle, aryle ou polyalkylène glycol ; et
   ▪ un groupe représenté par la formule (IIb) suivante : 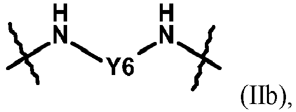 dans lequel Y6 représente un (C₁-C₁₂)alkyle, aryle, ou polyalkylène glycol.

Dans un tel mode de réalisation, le résidu peptidique est avantageusement un résidu du peptide Arg-Phe-Arg-Phe-Arg-Phe-Arg-Phe, Phe-Arg-Phe-Arg-Phe-Arg-Phe-Arg, ou Arg-Arg-Palm, mieux encore Arg-Phe-Arg-Phe-Arg-Phe-Arg-Phe.

Selon un mode particulier, Y1 et Y4 sont identiques et sont de préférence un aryle-(C₁-C₁₂)alkyle-aryle tel qu'un phényle-méthyle-phényle.
Selon un autre mode particulier, Y3 et Y6 sont choisis indépendamment parmi un (C₁-C₁₂)alkyle et aryle.
Selon un mode de réalisation particulier, Y1 et Y4 sont choisis indépendamment parmi un (C₁-C₆)alkyle, un phényle-méthyle-phényle et un phényle, et Y2, Y3, Y5, et Y6 sont choisis indépendamment parmi un (C₁-C₆)alkyle, un phényle, un polyéthylène glycol, un polypropylène glycol, et un polybutadiène glycol.
Selon un mode de réalisation particulier, Y1, Y2, Y3, Y4, Y5, et Y6 sont choisis indépendamment parmi un (C₁-C₆)alkyle et un phényle.
Selon un mode de réalisation plus particulier, Y1, Y2, Y3, Y4, Y5, et Y6 sont choisis indépendamment parmi un éthyle, un propyle, un butyle, un pentyle, un hexyle, et un phényle, de préférence parmi un 1,2-éthyle, 1,3-propyle, 1,4-butyle, 1,5-pentyle, 1,6-hexyle, 1,2-phényle, 1,3-phényle et 1,4-phényle.

Dans un autre mode de réalisation particulier, le composé polymère comprend :
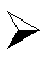 de motifs répétitifs de formule (I) dans laquelle :
   ∘ n vaut 0 ;
   ∘ Y1 représente un radical choisi parmi un (C₁-C₁₂)alkyle, un aryle, et un aryle-(C₁-C₁₂)alkyle-aryle (de préférence parmi un (C₁-C₆)alkyle et un aryle, mieux encore Y1 est un phényle) ; et
   ∘ Z représente un groupe représenté par la formule (la) suivante : 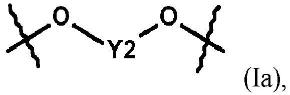 dans lequel Y2 représente un (C₁-C₁₂)alkyle, aryle, ou polyalkylène glycol (de préférence un (C₁-C₆)alkyle, ou polyalkylène glycol, mieux un (C₁-C₆)alkyle) ; et
      dans lequel Y1 est relié à un résidu peptidique via un résidu fonctionnel, le résidu fonctionnel et le résidu peptidique étant éventuellement séparés par un fragment espaceur ; et
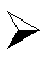 de motifs répétitifs de formule (II) dans laquelle :
   ∘ n' vaut 1 ;
   ∘ Y4 représente un radical choisi parmi un (C₁-C₁₂)alkyle, un aryle et un aryle-(C₁-C₁₂)alkyle-aryle (de préférence un aryle-(C₁-C₁₂)alkyle-aryle, tel qu'un phényle-méthyle-phényle); et
   ∘ Z2 représente un groupe représenté par la formule (IIa) suivante : 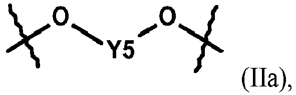 dans lequel Y5 représente un (C₁-C₁₂)alkyle, aryle, ou polyalkylène glycol (de préférence un (C₁-C₆)alkyle ou polyalkylène glycol).

Dans un tel mode, les motifs de formule (II) peuvent en particulier être une combinaison :
- de motifs répétitifs de formule (II) tels que définis ci-dessus où Y5 représente un (C₁-C₁₂)alkyle ; et
- de motifs répétitifs de formule (II) tels que définis ci-dessus où Y5 représente un polyalkylène glycol.

Dans un autre mode de réalisation, le composé polymère de l'invention comprend en outre d'autres motifs monomériques ou polymériques connus de l'homme du métier. En particulier, le composé polymère peut comprendre un bloc constitué de motifs de formule (I) et (II) tels que définis ci-dessus, et un ou plusieurs autres blocs polymériques constitués de motifs répétitifs connus de l'homme du métier, tels qu'un bloc polycaprolactone.

Dans un mode de réalisation, le composé polymère comprend des motifs de formule (I) du composé polymère dans laquelle :
- n = 0 ou 1, de préférence 0, et
- le groupe Y1 est relié à un résidu peptidique via un résidu fonctionnel, le résidu fonctionnel et le résidu peptidique étant séparé par un fragment espaceur, selon l'une quelconque des formules (Y1-M1) et (Y1-M2) : 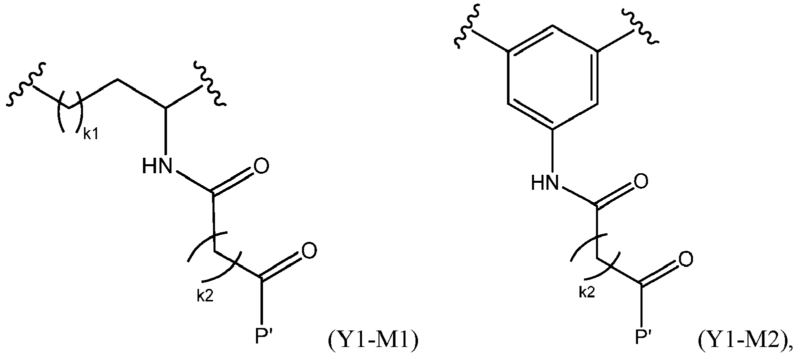 formules dans chacune desquelles :
- k1 est un entier de 1 à 8, de préférence de 1 à 4, mieux k1 vaut 1 ;
- chaque k2 est un entier de 1 à 8, de préférence de 1 à 6, mieux k2 vaut 3 ; et
- P' représente un résidu peptidique, de préférence un résidu du peptide Arg-Phe-Arg-Phe-Arg-Phe-Arg-Phe, du peptide Phe-Arg-Phe-Arg-Phe-Arg-Phe-Arg, ou du peptide Arg-Arg-Palm, mieux encore un résidu du peptide Arg-Phe-Arg-Phe-Arg-Phe-Arg-Phe.
Dans une tel mode de réalisation, Z est de préférence un groupe de formule (Ia) où Y2 représente un (C₁-C₁₂)alkyle (de préférence, un (C₁-C₆)alkyle tel qu'un éthyle ou butyle).

Dans un autre mode de réalisation particulier, le composé polymère comprend des motifs de formule (I) du composé polymère dans laquelle :
- n = 0 ou 1, de préférence 0, et
- le groupe Y1 est relié à un résidu peptidique via un résidu fonctionnel, le résidu fonctionnel et le résidu peptidique étant séparé par un fragment espaceur, selon la formule (Y1-M3) suivante : 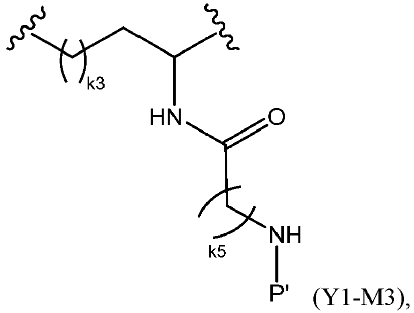
dans laquelle :
- k3 est un entier de 1 à 8, de préférence de 1 à 4, mieux k3 vaut 1 ;
- k5 est un entier de 1 à 8, de préférence de 1 à 6, mieux k5 vaut 5 ; et
- P' représente un résidu peptidique, de préférence un résidu du peptide Arg-Phe-Arg-Phe-Arg-Phe-Arg-Phe, Phe-Arg-Phe-Arg-Phe-Arg-Phe-Arg ou du peptide Arg-Arg-Palm, mieux encore du peptide Phe-Arg-Phe-Arg-Phe-Arg-Phe-Arg.
Dans une tel mode de réalisation, Z est de préférence un groupe de formule (Ib) où Y3 représente un (C₃-C₁₀)alkyle en (de préférence un (C₃-C₈)alkyle tel qu'un hexyle).

Dans un autre mode de réalisation particulier, le composé polymère comprend des motifs de formule (I) du composé polymère dans laquelle :
- n = 0 ou 1 ; de préférence 1, et
- Z est un groupe de formule (la) où Y2 est relié à un résidu peptidique via un résidu fonctionnel, le résidu fonctionnel et le résidu peptidique étant séparé par un fragment espaceur, selon la formule (Y2-M4) : 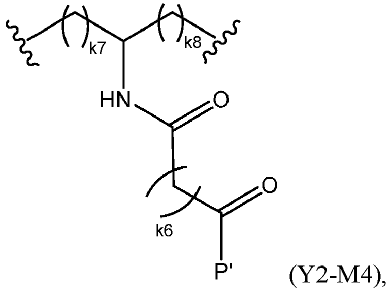
dans laquelle :
- k6 est un entier de 1 à 8, de préférence de 1 à 4, mieux k6 vaut 1 ;
- k7 est un entier de 1 à 8, de préférence de 1 à 4, mieux k7 vaut 1 ;
- k8 est un entier de 1 à 8, de préférence de 1 à 4, mieux k8 vaut 1 ; et
- P' représente un résidu peptidique, de préférence un résidu du peptide Arg-Phe-Arg-Phe-Arg-Phe-Arg-Phe, Phe-Arg-Phe-Arg-Phe-Arg-Phe-Arg, ou du peptide Arg-Arg-Palm.

### COMPOSE MONOMERE

L'invention concerne également un composé monomère représenté par la formule (III-1) suivante : 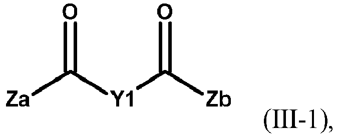 dans lequel
∘ Y1 représente un radical choisi parmi un (C₁-C₁₂)alkyle, un aryle, et un aryle-(C₁-C₁₂)alkyle-aryle ;
∘ Za représente un radical choisi parmi :
   ▪ un (C₁-C₁₂)alkyle-O-, un aryle-O-, un chlore ; et,
   ▪ un groupe représenté par la formule (IIIa) suivante : 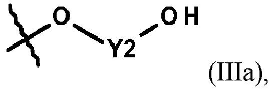 dans lequel Y2 représente un (C₁-C₁₂)alkyle, aryle ou polyalkylène glycol (de préférence un (C₁-C₁₂)alkyle ou aryle) ; et
∘ Zb représente un radical choisi parmi :
   ▪ un (C₁-C₁₂)alkyle-O-, un aryle-O-, un chlore ;
   ▪ un groupe représenté par la formule (IIIa) suivante : 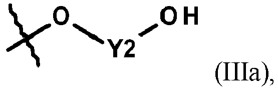 dans lequel Y2 représente un (C₁-C₁₂)alkyle, aryle, ou polyalkylène glycol (de préférence un (C₁-C₁₂)alkyle ou aryle) ; et
   ▪ un groupe représenté par la formule (IIIb) suivante : 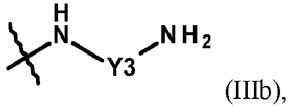 dans lequel Y3 représente un (C₁-C₁₂)alkyle, aryle, ou polyalkylène glycol (de préférence un (C₁-C₁₂)alkyle ou aryle) ;
   et dans lequel au moins un de Y1, Y2, et Y3 est relié à un résidu peptidique via un résidu fonctionnel, le résidu fonctionnel et le résidu peptidique étant éventuellement séparés par un fragment espaceur,
   sous réserve que, lorsque Y3 est relié à un résidu peptidique via un résidu fonctionnel, le résidu fonctionnel et le résidu peptidique étant éventuellement séparés par un fragment espaceur, Za est choisi parmi un (C₁-C₁₂)alkyle-O- et un aryle-O-.

Dans un mode de réalisation particulier, le résidu peptidique est un résidu du peptide Arg-Phe-Arg-Phe-Arg-Phe-Arg-Phe, Phe-Arg-Phe-Arg-Phe-Arg-Phe-Arg, ou Arg-Arg-Palm, mieux encore Arg-Phe-Arg-Phe-Arg-Phe-Arg-Phe.

Dans un mode de réalisation particulier, ledit fragment espaceur est de formule -(X1)-(X2)-(X3)-, dans laquelle X1 et X3 représentent chacun indépendamment -NH- ou -CO-, et X2 représente un (C₁-C₁₂)alkyle, aryle, ou polyalkylène glycol.

Selon un mode de réalisation particulier, Y1 représente un radical choisi parmi un (C₁-C₁₂)alkyle et un aryle.
Selon un mode de réalisation particulier, Y1 à Y3 sont chacun choisis indépendamment parmi un éthyle, un propyle, un butyle, un pentyle, un hexyle et un phényle, et de préférence parmi un 1,2-éthyle, 1,3-propyle, 1,4-butyle, 1,5-pentyle, 1,6-hexyle, 1,2-phényle, 1,3-phényle et 1,4-phényle.

Selon un mode de réalisation particulier, Za et Zb représentent chacun indépendamment un chlore, un (C₁-C₁₂)alkyle-O-, tel qu'un méthyle-O- ou un éthyle-O-, ou un aryle-O-, tel qu'un phényle-O-, de préférence un (C₁-C₁₂)alkyle-O- ou un aryle-O- (mieux encore, un (C₁-C₁₂)alkyle-O-), et Y1 est relié à un résidu peptidique via un résidu fonctionnel, le résidu fonctionnel et le résidu peptidique étant éventuellement séparés par un fragment espaceur. Dans un tel mode de réalisation, Za et Zb sont avantageusement identiques.

Selon un autre mode de réalisation, Za et Zb sont un groupe représenté par la formule (IIIa) suivante : 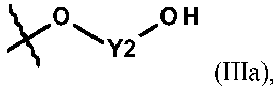 dans lequel Y2 représente un (C₁-C₁₂)alkyle, aryle ou polyalkylène glycol, et au moins un de Y1 et Y2, de préférence Y1, est relié à un résidu peptidique via un résidu fonctionnel, le résidu fonctionnel et le résidu peptidique étant éventuellement séparés par un fragment espaceur. Dans un tel mode de réalisation, Za et Zb sont avantageusement identiques.

Selon un mode particulier, Y1 est un (C₁-C₆)alkyle ou un phényle.
Selon un mode de réalisation préféré, Y1 est choisi parmi un éthyle, un propyle, un butyle, un pentyle, un hexyle, et un phényle, de préférence parmi un 1,2-éthyle, 1,3-propyle, 1,4-butyle, 1,5-pentyle, 1,6-hexyle, 1,2-phényle, 1,3-phényle et 1,4-phényle. De manière plus préférée, Y1 représente un phényle (notamment un 1,3-phényle) ou un propyle (notamment un 1,3-propyle).

Selon un mode de réalisation particulier, Y2 est un (C₁-C₆)alkyle et un phényle.
Selon un mode de réalisation préféré, Y2 est choisi parmi un éthyle, un propyle, un butyle, un pentyle, un hexyle, et un phényle, de préférence parmi un 1,2-éthyle, 1,3-propyle, 1,4-butyle, 1,5-pentyle, 1,6-hexyle, 1,2-phényle, 1,3-phényle et 1,4-phényle.
Selon un mode de réalisation préféré, Y1 est relié à un résidu peptidique via un résidu fonctionnel, le résidu fonctionnel et le résidu peptidique étant éventuellement séparés par un fragment espaceur.
Selon un autre mode de réalisation préféré, la structure « Y-(résidu fonctionnel) », où Y est Y1 et/ou Y2, de préférence Y1, est : 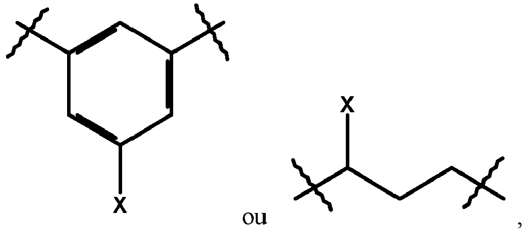 où X représente le résidu fonctionnel, notamment -CO- ou -NH-, de préférence -NH-, lui-même relié à un résidu peptidique, le résidu fonctionnel et le résidu peptidique étant éventuellement séparés par un fragment espaceur.

Dans un mode de réalisation préféré, le composé monomère est un composé de formule (III-1), dans lequel :
- Y1 est un 1,3-propyle ou un 1,3-phényle (de préférence un 1,3-phényle) ; et
- Za et Zb, identiques ou différents (de préférence identiques), représentent un méthyle-O- ou un groupe de formule (IIIa) dans lequel Y2 est un 1,2-éthyle ou un 1,4-butyle (de préférence un 1,4-butyle);
et dans lequel Y1 est relié à un résidu peptidique via un résidu fonctionnel, le résidu fonctionnel et le résidu peptidique étant éventuellement séparés par un fragment espaceur.
Dans un tel mode de réalisation préféré, le résidu fonctionnel est avantageusement -NH-, et le fragment espaceur est avantageusement de formule -(X1)-(X2)-(X3)- où X1 et X3 représentent -CO- et X2 est un (C₁-C₆)alkyle, mieux un 1,1-méthyle ou un 1,3-propyle.
Dans un tel mode de réalisation préféré, le résidu peptidique est avantageusement un résidu du peptide Arg-Phe-Arg-Phe-Arg-Phe-Arg-Phe, Phe-Arg-Phe-Arg-Phe-Arg-Phe-Arg, ou Arg-Arg-Palm, mieux encore Arg-Phe-Arg-Phe-Arg-Phe-Arg-Phe.
De préférence, Za et Zb représentent un méthyle-O-.

Selon un autre mode de réalisation, le composé monomère est représenté par la formule (III-1), dans laquelle :
∘ Za est un (C₁-C₁₂)alkyle-O-, un aryle-O-, ou un chlore, de préférence un (C₁-C₁₂)alkyle-O- ou un aryle-O- ; et
∘ Zb est un groupe représenté par la formule (IIIb) suivante : 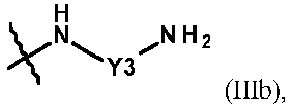 dans lequel Y3 représente un (C₁-C₁₂)alkyle, aryle, ou polyalkylène glycol (de préférence un (C₁-C₁₂)alkyle ou aryle),
   où au moins un de Y1 et Y3 est substitué par un peptide et sous réserve que, lorsque Y3 est relié à un résidu peptidique via un résidu fonctionnel, Za est choisi parmi un (C₁-C₁₂)alkyle-O- et un aryle-O-.

Selon un mode de réalisation particulier, Y1 est un (C₁-C₆)alkyle ou un phényle.
Selon un mode de réalisation plus particulier, Y1 est choisi parmi un éthyle, un propyle, un butyle, un pentyle, un hexyle, et un phényle, de préférence parmi un 1,2-éthyle, 1,3-propyle, 1,4-butyle, 1,5-pentyle, 1,6-hexyle, 1,2-phényle, 1,3-phényle et 1,4-phényle.
Selon un mode de réalisation particulier, Y3 est un (C₁-C₆)alkyle ou un phényle.
Selon un mode de réalisation plus particulier, Y3 est choisi parmi un éthyle, un propyle, un butyle, un pentyle, un hexyle, et un phényle, de préférence parmi un 1,2-éthyle, 1,3-propyle, 1,4-butyle, 1,5-pentyle, 1,6-hexyle, 1,2-phényle, 1,3-phényle et 1,4-phényle.
Selon un mode de réalisation préféré, Y1 est relié à un résidu peptidique via un résidu fonctionnel, le résidu fonctionnel et le résidu peptidique étant éventuellement séparés par un fragment espaceur.
Selon ce mode préféré, la structure « Y-(résidu fonctionnel) », où Y est Y1 et/ou Y3, de préférence Y1, est avantageusement : 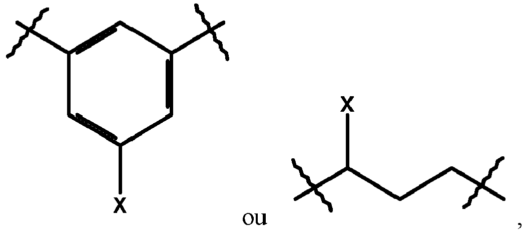 où X représente le résidu fonctionnel, notamment -CO- ou -NH-, de préférence NH, et de préférence, 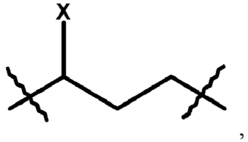 où X représente le résidu fonctionnel -NH-,
ledit résidu fonctionnel étant relié à un résidu peptidique, et le résidu fonctionnel et le résidu peptidique étant éventuellement séparés par un fragment espaceur.

Selon un autre mode de réalisation préféré, Y3 est relié à un résidu peptidique via un résidu fonctionnel, le résidu fonctionnel et le résidu peptidique étant éventuellement séparés par un fragment espaceur.
Selon ce mode préféré, la structure « Y-(résidu fonctionnel) », où Y est Y3, est avantageusement 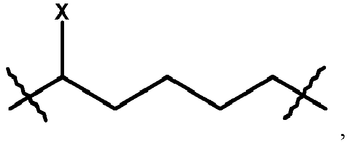 où X représente le résidu fonctionnel -CO- ou -NH-, de préférence -CO-, lui-même relié à un résidu peptidique, le résidu fonctionnel et le résidu peptidique étant éventuellement séparés par un fragment espaceur.

Dans un autre mode de réalisation préféré, le composé monomère est un composé de formule (III-1), dans lequel :
- Y1 est un 1,3-propyle ;
- Za représente un méthyle-O ; et
- Zb représente un groupe représenté par la formule (IIIb) dans lequel Y3 représente un 1,6-hexyle;
et dans lequel Y1 est relié à un résidu peptidique via un résidu fonctionnel, le résidu fonctionnel et le résidu peptidique étant éventuellement séparés par un fragment espaceur. Dans un tel mode de réalisation préféré, le résidu fonctionnel est avantageusement -NH-, et le fragment espaceur est avantageusement de formule -(X1)-(X2)-(X3)- où X1 et X3 représentent -CO- et -NH- respectivement, et X2 est un (C₁-C₆)alkyle, mieux un 1,5-pentyle.
Dans un tel mode de réalisation préféré, le résidu peptidique est avantageusement un résidu du peptide Arg-Phe-Arg-Phe-Arg-Phe-Arg-Phe, Phe-Arg-Phe-Arg-Phe-Arg-Phe-Arg, ou Arg-Arg-Palm, mieux encore Arg-Phe-Arg-Phe-Arg-Phe-Arg-Phe.

La présente invention a également pour objet un composé monomère représenté par la formule (III-2) suivante :

HO-Y2-OH (III-2),

dans lequel Y2 représente un (C₁-C₁₂)alkyle, aryle ou polyalkylène glycol, où Y2 est relié à un résidu peptidique via un résidu fonctionnel, le résidu fonctionnel et le résidu peptidique étant éventuellement séparés par un fragment espaceur.

On préfère que le résidu peptidique soit un résidu du peptide Arg-Phe-Arg-Phe-Arg-Phe-Arg-Phe, Phe-Arg-Phe-Arg-Phe-Arg-Phe-Arg, ou Arg-Arg-Palm, mieux encore Arg-Phe-Arg-Phe-Arg-Phe-Arg-Phe.

Dans un mode de réalisation particulier, le monomère de formule (III-1) est choisi parmi les formules (M1) et (M2) suivantes : 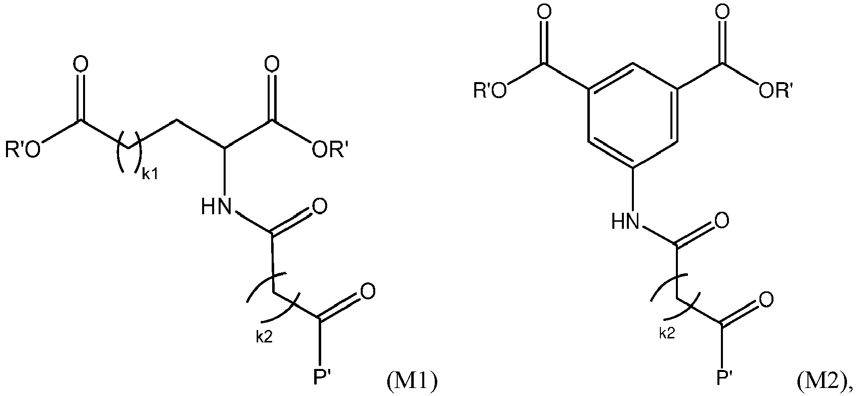 formules dans chacune desquelles :
- k1 est un entier de 1 à 8, de préférence de 1 à 4, mieux k1 vaut 1 ;
- chaque k2 est un entier de 1 à 8, de préférence de 1 à 6, mieux k2 vaut 3 ;
- chaque R' représente indépendamment un (C₁-C₆)-alkyle (de préférence, un méthyle) ou un hydroxy-(C₁-C₁₂)alkyle (de préférence, un hydroxy-(C₁-C₆)alkyle tel qu'un 2-hydroxyéthyle ou un 4-hydroxybutyle) ;
- P' représente un résidu peptidique, de préférence un résidu du peptide Arg-Phe-Arg-Phe-Arg-Phe-Arg-Phe, Phe-Arg-Phe-Arg-Phe-Arg-Phe-Arg, ou du peptide Arg-Arg-Palm, mieux encore un résidu du peptide Arg-Phe-Arg-Phe-Arg-Phe-Arg-Phe.

Dans un autre mode de réalisation particulier, le monomère de formule (III-1) est représenté par la formule (M3) suivante : 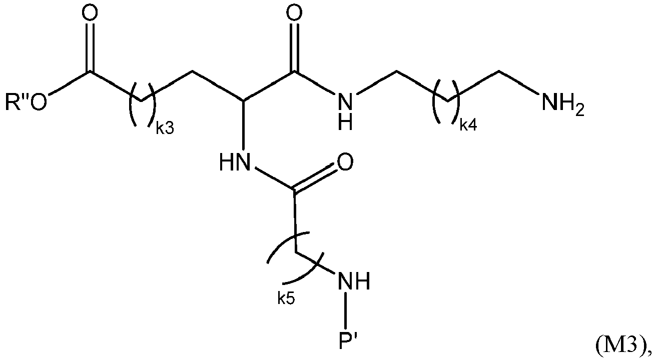 dans laquelle :
- k3 est un entier de 1 à 8, de préférence de 1 à 4, mieux k3 vaut 1 ;
- k4 est un entier de 1 à 8, de préférence de 1 à 4, mieux k4 vaut 4 ;
- k5 est un entier de 1 à 8, de préférence de 1 à 6, mieux k5 vaut 5 ;
- R" est un (C₁-C₆)-alkyle, de préférence un méthyle ;
- P' représente un résidu peptidique, de préférence un résidu du peptide Arg-Phe-Arg-Phe-Arg-Phe-Arg-Phe, Phe-Arg-Phe-Arg-Phe-Arg-Phe-Arg, ou du peptide Arg-Arg-Palm, mieux encore du peptide Phe-Arg-Phe-Arg-Phe-Arg-Phe-Arg.

Dans un autre mode de réalisation particulier, le monomère de formule (111-2) est représenté par la formule (M4) suivante : 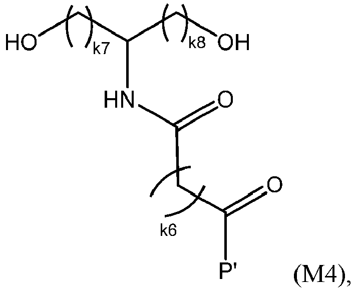 dans laquelle :
- k6 est un entier de 1 à 8, de préférence de 1 à 4, mieux k6 vaut 1 ;
- k7 est un entier de 1 à 8, de préférence de 1 à 4, mieux k7 vaut 1 ;
- k8 est un entier de 1 à 8, de préférence de 1 à 4, mieux k8 vaut 1 ;
- P' représente un résidu peptidique, de préférence un résidu du peptide Arg-Phe-Arg-Phe-Arg-Phe-Arg-Phe, Phe-Arg-Phe-Arg-Phe-Arg-Phe-Arg, ou du peptide Arg-Arg-Palm.

Selon un mode de réalisation particulier, le composé monomère selon l'invention est greffé sur un support solide, tel qu'une silice, une alumine, du verre, une résine de type polystyrène, une résine de type polyéthylène glycol. Selon un mode de réalisation, le composé monomère est rattaché au support solide via le résidu peptidique.

### Procédé de préparation du composé polymère

Dans la présente demande, la formule 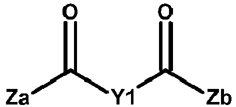 peut également être écrite : Za-C(O)-Y1-C(O)-Zb.

Un autre objet de l'invention concerne un procédé de préparation d'un composé polymère selon l'invention, comprenant une réaction de polycondensation en milieu fondu, mettant enjeu : 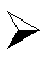
au moins un composé monomère choisi parmi :
   ∘ un composé monomère de formule Za-C(O)-Y1-C(O)-Zb, où :
      - Y1 représente un radical choisi parmi un (C₁-C₁₂)alkyle, un aryle et un aryle-(C₁-C₁₂)alkyle-aryle ;
      - Za représente un radical choisi parmi un (C₁-C₁₂)alkyle-O-, un aryle-O-, un chlore, et un groupe de formule (IIIa) : 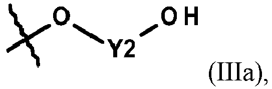 où Y2 représente un (C₁-C₁₂)alkyle, aryle, ou polyalkylène glycol ;
      - Zb représente un radical choisi parmi un (C₁-C₁₂)alkyle-O-, un aryle-O-, un chlore, un groupe de formule (IIIa) et un groupe de formule (IIIb) ; 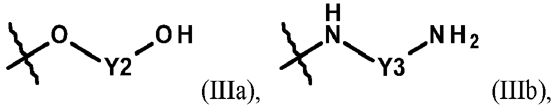 où Y2 représente un (C₁-C₁₂)alkyle, aryle, ou polyalkylène glycol, et Y3 représente un (C₁-C₁₂)alkyle, aryle, ou polyalkylène glycol ;
   ∘ OCN-Y1-NCO, où Y1 représente un (C₁-C₁₂)alkyle, aryle, ou un aryle-(C₁-C₁₂)alkyle-aryle ;
   ∘ HO-Y2-OH, où Y2 représente un (C₁-C₁₂)alkyle, aryle ou polyalkylène glycol, et
   ∘ H₂N-Y3-NH₂, où Y3 représente un (C₁-C₁₂)alkyle, aryle, ou polyalkylène glycol ;
   où au moins un de Y1, Y2, et Y3 est relié à un résidu peptidique via un résidu fonctionnel, le résidu fonctionnel et le résidu peptidique étant éventuellement séparés par un fragment espaceur; 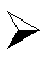
et au moins un composé monomère choisi parmi :
   ∘ un composé monomère représenté par la formule (V) suivante : 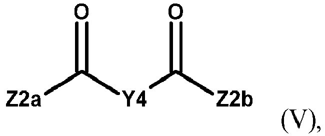 dans lequel
      - Y4 représente un radical choisi parmi un (C₁-C₁₂)alkyle, un aryle et un aryle-(C₁-C₁₂)alkyle-aryle ;
      - Z2a représente un radical choisi parmi :
         ▪ un (C₁-C₁₂)alkyle-O-, un aryle-O-, un chlore ; et
         ▪ un groupe représenté par la formule (Va) suivante : 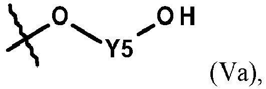 dans lequel Y5 représente un (C₁-C₁₂)alkyle, aryle ou polyalkylène glycol; et
      - Z2b représente un radical choisi parmi :
         ▪ un (C₁-C₁₂)alkyle-O-, un aryle-O-, un chlore ;
         ▪ un groupe représenté par la formule (Va) suivante : 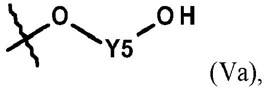 dans lequel Y5 représente un (C₁-C₁₂)alkyle, aryle ou polyalkylène glycol ; et
         ▪ un groupe représenté par la formule (Vb) suivante : 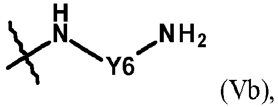 dans lequel Y6 représente un (C₁-C₁₂)alkyle, aryle, ou polyalkylène glycol,
   ∘ OCN-Y4-NCO, où Y4 représente un (C₁-C₁₂)alkyle, aryle, ou un aryle-(C₁-C₁₂)alkyle-aryle,
   ∘ HO-Y5-OH, où Y5 représente un (C₁-C₁₂)alkyle, aryle ou polyalkylène glycol et
   ∘ H₂N-Y6-NH₂, où Y6 représente un (C₁-C₁₂)alkyle, aryle, ou polyalkylène glycol.

Dans le procédé de l'invention, Y3 et Y6 sont de préférence un (C₁-C₁₂)alkyle ou aryle.

Dans le procédé de l'invention, on préfère que, lorsque Y3 est relié à un résidu peptidique via un résidu fonctionnel, le résidu fonctionnel et le résidu peptidique étant éventuellement séparés par un fragment espaceur, Za, Zb, Z2a et Z2b sont indépendamment choisis parmi un (C₁-C₁₂)alkyle-O- et un aryle-O-.

Dans un mode de réalisation particulier, le résidu peptidique est avantageusement un résidu du peptide Arg-Phe-Arg-Phe-Arg-Phe-Arg-Phe, Phe-Arg-Phe-Arg-Phe-Arg-Phe-Arg, ou Arg-Arg-Palm, mieux encore Arg-Phe-Arg-Phe-Arg-Phe-Arg-Phe.

Dans un mode de réalisation particulier, ledit au moins un composé monomère de formule Za-C(O)-Y1-C(O)-Zb mis enjeu dans le procédé selon l'invention est un composé monomère de formule (III-1) tel que défini ci-dessus.

Dans un autre mode de réalisation particulier, ledit au moins un composé monomère de formule HO-Y2-OH mis enjeu dans le procédé selon l'invention est un composé monomère de formule (III-2) tel que défini ci-dessus.

Les composés monomères mis en jeu pour préparer le composé polymère de l'invention sont choisis de telle sorte qu'ils peuvent réagir ensemble selon une réaction de polycondensation. Par « réaction de polycondensation », on entend une réaction de polymérisation par addition d'un nucléophile sur un électrophile, éventuellement avec élimination d'un groupement partant. Des exemples de réaction de polycondensation sont notamment :
- la polycondensation par estérification ou transestérification : des fonctions esters sont créées, par exemple par réaction entre une fonction alcool et une fonction dérivée d'acide carboxylique telle qu'un ester, anhydride, ou chlorure d'acyle ;
- la polycondensation par couplage amide : réaction entre une fonction amine et une fonction dérivée d'acide carboxylique telle qu'un ester, anhydride, ou chlorure d'acyle ;
- la polycondensation par réaction entre une fonction alcool ou amine, et une fonction isocyanate (une polycondensation mettant en jeu une fonction isocyanate peut également être appelée « polyaddition »).

Selon un mode de réalisation particulier, le procédé comprend une réaction de polycondensation en milieu fondu, mettant en jeu :
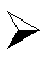 au moins un composé monomère de formule (III-1), dans lequel Za et Zb représentent un groupe de formule (IIIa), où au moins un de Y1 et Y2, de préférence Y1, est relié à un résidu peptidique via un résidu fonctionnel, le résidu fonctionnel et le résidu peptidique étant éventuellement séparés par un fragment espaceur ;
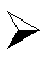 et au moins un composé monomère de formule (V) tel que défini ci-dessus dans lequel Z2a et Z2b représentent un groupe de formule (Va).

Selon un autre mode de réalisation particulier, le procédé comprend une réaction de polycondensation en milieu fondu, mettant en jeu:
- au moins un composé monomère de formule (III-1), dans lequel Za et Zb représentent chacun indépendamment un (C₁-C₁₂)alkyle-O-, aryle-O-, ou chlore, de préférence un (C₁-C₁₂)alkyle-O- ou aryle-O- ;
- au moins un HO-Y2-OH ;
   où au moins un de Y1 et Y2, de préférence Y1, est relié à un résidu peptidique via un résidu fonctionnel, le résidu fonctionnel et le résidu peptidique étant éventuellement séparés par un fragment espaceur ;
- au moins un composé monomère de formule (V), dans lequel Z2a et Z2b représentent chacun un (C₁-C₁₂)alkyle-O-, aryle-O-, ou chlore, de préférence un (C₁-C₁₂)alkyle-O- ou aryle-O- ; et,
- au moins un HO-Y5-OH.

Selon un mode particulier, Y1, Y2, Y4, et Y5 représentent chacun indépendamment un (C₁-C₁₂)alkyle, tel qu'un éthyle, un propyle, un butyle, un pentyle, ou un hexyle.
Selon un autre mode particulier, Y1, Y2, Y4, et Y5 représentent chacun indépendamment un aryle, tel qu'un phényle.
Selon un autre mode particulier, Y1 et Y4 sont identiques.
Selon un autre mode particulier, Y2 et Y5 sont identiques. On préfère que Y2 et Y5 représentent un (C₁-C₆)alkyle, plus préférentiellement un 1,2-éthyle ou un 1,4-butyle, mieux encore un 1,4-butyle.

Selon un mode de réalisation particulier, Y1, Y2, Y4 et Y5 sont choisis indépendamment parmi un (C₁-C₆)alkyle et un phényle.

Selon un mode de réalisation plus particulier, Y1, Y2, Y4 et Y5 sont choisis indépendamment parmi un éthyle, un propyle, un butyle, un pentyle, un hexyle, et un phényle, de préférence parmi un 1,2-éthyle, 1,3-propyle, 1,4-butyle, 1,5-pentyle, 1,6-hexyle, 1,2-phényle, 1,3-phényle et 1,4-phényle.

Selon un mode de réalisation particulier, Y1 et/ou Y2 sont choisis indépendamment parmi un (C₁-C₆)alkyle et un phényle, notamment parmi un éthyle, un propyle, un butyle, un pentyle, un hexyle, et un phényle, de préférence parmi un 1,2-éthyle, 1,3-propyle, 1,4-butyle, 1,5-pentyle, 1,6-hexyle, 1,2-phényle, 1,3-phényle et 1,4-phényle.
Selon un mode de réalisation préféré, Y1 est relié à un résidu peptidique via un résidu fonctionnel, le résidu fonctionnel et le résidu peptidique étant éventuellement séparés par un fragment espaceur.
Selon un mode de réalisation préféré, Y1 représente un phényle (notamment un 1,3-phényle) ou un propyle (notamment un 1,3-propyle).
Selon un autre mode de réalisation préféré, la structure « Y-(résidu fonctionnel) », où Y est Y1 et/ou Y2, de préférence Y1, est : 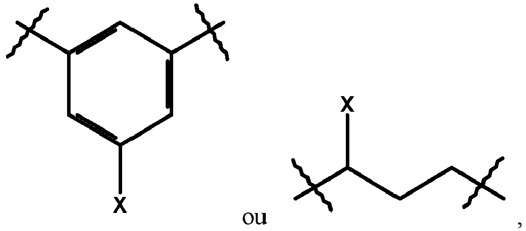 où X représente le résidu fonctionnel, notamment -CO- ou -NH-, de préférence -NH-, lui-même relié à un résidu peptidique, le résidu fonctionnel et le résidu peptidique étant éventuellement séparés par un fragment espaceur.

Selon un autre mode de réalisation plus particulier, le procédé comprend une réaction de polycondensation en milieu fondu, mettant enjeu :
- un composé monomère de formule (III-1), dans lequel Y1 représente un (C₁-C₁₂)alkyle ou un aryle, et Za et Zb représentent un (C₁-C₁₂)alkyle-O- ;
   où Y1 est relié à un résidu peptidique via un résidu fonctionnel, le résidu fonctionnel et le résidu peptidique étant éventuellement séparés par un fragment espaceur ;
- au moins un composé monomère de formule (V), dans lequel Y4 est un (C₁-C₁₂)alkyle ou un aryle, et Z2a et Z2b représentent un (C₁-C₁₂)alkyle-O-; et,
- au moins un composé de formule HO-(C₁-C₁₂)alkyle-OH ou HO-aryle-OH, de préférence de formule HO-(C₁-C₁₂)alkyle-OH.
Dans un tel mode de réalisation particulier, le résidu fonctionnel est avantageusement -NH-, et le fragment espaceur est avantageusement de formule -(X1)-(X2)-(X3)- où X1 et X3 représentent -CO- et X2 est un (C₁-C₆)alkyle.

De préférence, le procédé comprend une réaction de polycondensation en milieu fondu, mettant en jeu :
- un composé monomère de formule (III-1), dans lequel Y1 représente un 1,3-propyle ou un 1,3-phényle (mieux, un 1,3-phényle), Za et Zb représentent un méthyle-O- ;
   où Y1 est relié à un résidu peptidique via un résidu fonctionnel, le résidu fonctionnel et le résidu peptidique étant éventuellement séparés par un fragment espaceur ;
- au moins un composé monomère de formule (V), dans lequel Y4 est un 1,4-phényle, 1,2-éthyle ou un 1,4-butyle, Z2a et Z2b représentent un méthyle-O- ; et,
- de l'éthane-1,2-diol ou du butane-1,4-diol, de préférence du butane-1,4-diol.
Dans un tel mode de réalisation particulier, le résidu fonctionnel est avantageusement -NH-, et le fragment espaceur est avantageusement de formule -(X1)-(X2)-(X3)- où X1 et X3 représentent -CO- et X2 est un (C₁-C₆)alkyle, mieux un 1,1-méthyle ou un 1,3-propyle.

Selon un autre mode de réalisation particulier, le procédé comprend une réaction de polycondensation en milieu fondu, mettant en jeu :
∘ au moins un composé monomère de formule (III-1), dans lequel :
   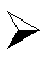 Za est un (C₁-C₁₂)alkyle-O-, un aryle-O-, ou un chlore, de préférence un (C₁-C₁₂)alkyle-O- ou aryle-O- ; et
   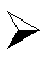 Zb est un groupe de formule (IIIb) ;
   où au moins un de Y1 et Y3, de préférence Y1 ou Y3, est substitué par un peptide et sous réserve que, lorsque Y3 est relié à un résidu peptidique via un résidu fonctionnel, le résidu fonctionnel et le résidu peptidique étant éventuellement séparés par un fragment espaceur, Za est choisi parmi un (C₁-C₁₂)alkyle-O- et un aryle-O-,
∘ et au moins un composé monomère de formule (V) tel que défini ci-dessus dans lequel :
   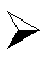 Z2a est un (C₁-C₁₂)alkyle-O-, un aryle-O-, ou un chlore, de préférence un (C₁-C₁₂)alkyle-O- ou aryle-O- ; et
   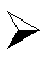 Z2b est un groupe de formule (Vb) ;
   sous réserve que, lorsque Y3 est relié à un résidu peptidique via un résidu fonctionnel, le résidu fonctionnel et le résidu peptidique étant éventuellement séparés par un fragment espaceur, Za, Zb, Z2a et Z2b sont indépendamment choisis parmi un (C₁-C₁₂)alkyle-O- et un aryle-O-. Dans un tel mode particulier, ledit au moins un composé monomère de formule Za-C(O)-Y1-C(O)-Zb peut en particulier être un monomère de formule (M3) tel que défini ci-dessus.

Selon un autre mode de réalisation particulier, le procédé comprend une réaction de polycondensation en milieu fondu, mettant enjeu :
- au moins un composé monomère de formule Za-C(O)-Y1-C(O)-Zb, dans lequel Za et Zb représentent chacun indépendamment un (C₁-C₁₂)alkyle-O-, aryle-O-, ou un chlore, de préférence un (C₁-C₁₂)alkyle-O- ou aryle-O- ;
- au moins un H₂N-Y3-NH₂ ;
   où au moins un de Y1 et Y3, de préférence Y1 ou Y3, est relié à un résidu peptidique via un résidu fonctionnel, le résidu fonctionnel et le résidu peptidique étant éventuellement séparés par un fragment espaceur ;
- au moins un composé monomère de formule (V), dans lequel Z2a et Z2b représentent chacun un (C₁-C₁₂)alkyle-O-, aryle-O-, ou un chlore, de préférence un (C₁-C₁₂)alkyle-O- ou aryle-O- ; et,
- au moins un H₂N-Y6-NH₂ ;
   sous réserve que, lorsque Y3 est relié à un résidu peptidique via un résidu fonctionnel, le résidu fonctionnel et le résidu peptidique étant éventuellement séparés par un fragment espaceur, Za, Zb, Z2a et Z2b sont indépendamment choisis parmi un (C₁-C₁₂)alkyle-O- et un aryle-O-.

Selon un mode particulier, Y1, Y3, Y4, et Y6 représentent chacun indépendamment un (C₁-C₁₂)alkyle, tel qu'un éthyle, un propyle, un butyle, un pentyle, ou un hexyle.
Selon un autre mode particulier, Y1, Y3, Y4, et Y6 représentent chacun indépendamment un aryle, tel qu'un phényle.
Selon un autre mode particulier, Y1 et Y4 sont identiques.
Selon un autre mode particulier, Y3 et Y6 sont identiques.

Selon un mode de réalisation particulier, Y1, Y3, Y4 et Y6 sont choisis indépendamment parmi un (C₁-C₆)alkyle et un phényle.
Selon un mode de réalisation plus particulier, Y1, Y3, Y4 et Y6 sont choisis indépendamment parmi un éthyle, un propyle, un butyle, un pentyle, un hexyle, et un phényle, de préférence parmi un 1,2-éthyle, 1,3-propyle, 1,4-butyle, 1,5-pentyle, 1,6-hexyle, 1,2-phényle, 1,3-phényle et 1,4-phényle.

Selon un mode de réalisation particulier, Y1 et/ou Y3 sont choisis indépendamment parmi un éthyle, un propyle, un butyle, un pentyle, un hexyle, et un phényle, de préférence parmi un 1,2-éthyle, 1,3-propyle, 1,4-butyle, 1,5-pentyle, 1,6-hexyle, 1,2-phényle, 1,3-phényle et 1,4-phényle.

Selon un mode de réalisation préféré, Y1 est relié à un résidu peptidique via un résidu fonctionnel, le résidu fonctionnel et le résidu peptidique étant éventuellement séparés par un fragment espaceur.
Selon ce mode préféré, la structure « Y-(résidu fonctionnel) », où Y est Y1, est avantageusement : 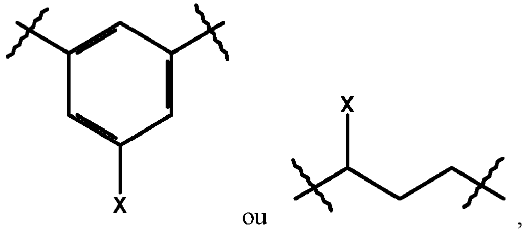 où X représente le résidu fonctionnel, notamment -CO- ou -NH-, de préférence NH, et de préférence, 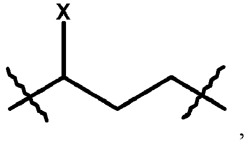 où X représente le résidu fonctionnel -NH-,
ledit résidu fonctionnel étant relié à un résidu peptidique, et le résidu fonctionnel et le résidu peptidique étant éventuellement séparés par un fragment espaceur.

Selon un autre mode de réalisation préféré, Y3 est relié à un résidu peptidique via un résidu fonctionnel, le résidu fonctionnel et le résidu peptidique étant éventuellement séparés par un fragment espaceur.
Selon ce mode préféré, la structure « Y-(résidu fonctionnel) », où Y est Y3, est avantageusement 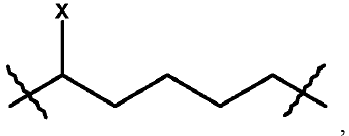 où X représente le résidu fonctionnel -CO- ou -NH-, de préférence -CO-, lui-même relié à un résidu peptidique, le résidu fonctionnel et le résidu peptidique étant éventuellement séparés par un fragment espaceur.

Dans un autre mode de réalisation particulier, le procédé comprend une réaction de polycondensation en milieu fondu, mettant en jeu :
- au moins un composé OCN-Y1-NCO où Y1 représente un (C₁-C₁₂)alkyle, aryle ou aryle-(C₁-C₁₂)alkyle-aryle (de préférence un aryle-(C₁-C₁₂)alkyle-aryle),
- au moins un composé HO-Y2-OH où Y2 représente un (C₁-C₁₂)alkyle, aryle, ou polyalkylène glycol,
- éventuellement, au moins un composé H₂N-Y3-NH₂ où Y3 représente un (C₁-C₁₂)alkyle, aryle, ou polyalkylène glycol,
- au moins un composé OCN-Y4-NCO, où Y4 représente un (C₁-C₁₂)alkyle, aryle, ou aryle-(C₁-C₁₂)alkyle-aryle (de préférence un aryle-(C₁-C₁₂)alkyle-aryle),
- au moins un composé HO-Y5-OH, où Y5 représente un (C₁-C₁₂)alkyle, aryle ou polyalkylène glycol, et
- éventuellement H₂N-Y6-NH₂, où Y6 représente un (C₁-C₁₂)alkyle, aryle, ou polyalkylène glycol ;
où Y2 et/ou Y3, de préférence Y2 uniquement, est relié à un résidu peptidique via un résidu fonctionnel, le résidu fonctionnel et le résidu peptidique étant éventuellement séparés par un fragment espaceur.

Dans un mode de réalisation plus particulier, le procédé comprend une réaction de polycondensation en milieu fondu, mettant en jeu :
- au moins un composé OCN-Y1-NCO où Y1 représente un (C₁-C₁₂)alkyle, aryle ou un aryle-(C₁-C₁₂)alkyle-aryle,
- au moins un composé de formule (111-2) tel que défini ci-dessus,
- éventuellement, au moins un composé H₂N-Y3-NH₂ où Y3 représente un (C₁-C₁₂)alkyle, aryle, ou polyalkylène glycol,
- au moins un composé OCN-Y4-NCO, où Y4 représente un (C₁-C₁₂)alkyle, aryle, ou un aryle-(C₁-C₁₂)alkyle-aryle),
- au moins un composé HO-Y5-OH, où Y5 représente un (C₁-C₁₂)alkyle, aryle, ou polyalkylène glycol,
- éventuellement H₂N-Y6-NH₂, où Y6 représente un (C₁-C₁₂)alkyle, aryle, ou polyalkylène glycol
où Y2 est relié à un résidu peptidique via un résidu fonctionnel, le résidu fonctionnel et le résidu peptidique étant éventuellement séparés par un fragment espaceur.

On préfère que Y1 et Y4 soient identiques.

Selon un mode de réalisation particulier, Y1 est choisi parmi un 1,2-éthyle, un 1,3-propyle, un 1,4-butyle, un 1,5-pentyle, un 1,6-hexyle, un 1,3-phényle, un 1,4-phényle, et un phényle-méthyle-phényle.
Selon un autre mode de réalisation particulier, Y2 est choisi parmi un 1,2-éthyle, un 1,3-propyle, un 1,4-butyle, un 1,5-pentyle, un 1,6-hexyle, un 1,3-phényle, un 1,4-phényle, un polyéthylène glycol et un polybutylène glycol.
Selon un autre mode de réalisation particulier, Y3 est choisi parmi un 1,2-éthyle, un 1,3-propyle, un 1,4-butyle, un 1,5-pentyle, un 1,6-hexyle, un 1,3-phényle, un 1,4-phényle.
Selon un autre mode de réalisation particulier, Y4 est choisi parmi un 1,2-éthyle, un 1,3-propyle, un 1,4-butyle, un 1,5-pentyle, un 1,6-hexyle, un 1,3-phényle, un 1,4-phényle, et un phényle-méthyle-phényle.
Selon un autre mode de réalisation particulier, Y5 est choisi parmi un 1,2-éthyle, un 1,3-propyle, un 1,4-butyle, un 1,5-pentyle, un 1,6-hexyle, un 1,3-phényle, un 1,4-phényle, un polyéthylène glycol et un polybutylène glycol.
Selon un autre mode de réalisation particulier, Y6 est choisi parmi un 1,2-éthyle, un 1,3-propyle, un 1,4-butyle, un 1,5-pentyle, un 1,6-hexyle, un 1,3-phényle, et un 1,4-phényle.

Dans un autre mode de réalisation, le procédé comprend une réaction de polycondensation en milieu fondu, mettant en jeu :
- au moins un composé monomère de formule (III-1), dans lequel Za et Zb représentent un groupe de formule (IIIa), où au moins un de Y1 et Y2, de préférence Y1, est relié à un résidu peptidique via un résidu fonctionnel, le résidu fonctionnel et le résidu peptidique étant éventuellement séparés par un fragment espaceur,
- au moins un OCN-Y4-NCO, où Y4 représente un (C₁-C₁₂)alkyle, aryle ou aryle-(C₁-C₁₂)alkyle-aryle (de préférence un aryle-(C₁-C₁₂)alkyle-aryle),
- au moins un HO-Y5-OH, où Y5 représente un (C₁-C₁₂)alkyle, aryle, ou polyalkylène glycol, et
- éventuellement au moins un H₂N-Y6-NH₂, où Y6 représente un (C₁-C₁₂)alkyle, aryle, ou polyalkylène glycol.
De préférence, ledit au moins un HO-Y5-OH est un mélange d'un HO-Y5-OH, où Y5 représente un (C₁-C₁₂)alkyle (par exemple un 1,4-butyle) et d'un HO-Y5-OH, où Y5 représente un polyalkylène glycol (de préférence, polybutylène glycol).

Dans un autre mode de réalisation, le procédé comprend :
(a) une première étape de réaction de polycondensation en milieu fondu pour former un premier composé polymère, mettant en jeu :
   - au moins un composé monomère de formule (III-1), dans lequel Za et Zb représentent chacun indépendamment un (C₁-C₁₂)alkyle-O-, aryle-O-, ou chlore, de préférence un (C₁-C₁₂)alkyle-O- ou aryle-O- ;
   - au moins un HO-Y2-OH, où Y2 représente un (C₁-C₁₂)alkyle, aryle ou polyalkylène glycol ; où au moins un de Y1 et Y2, de préférence Y1, est relié à un résidu peptidique via un résidu fonctionnel, le résidu fonctionnel et le résidu peptidique étant éventuellement séparés par un fragment espaceur ; et
   - au moins un composé monomère de formule (V), dans lequel Z2a et Z2b représentent chacun un (C₁-C₁₂)alkyle-O-, aryle-O-, ou chlore, de préférence un (C₁-C₁₂)alkyle-O- ou aryle-O- ; et,
   - au moins un HO-Y5-OH où Y5 représente un (C₁-C₁₂)alkyle, aryle ou polyalkylène glycol ;
(b) une deuxième étape de réaction de polycondensation en milieu fondu pour former un deuxième composé polymère, mettant en jeu :
   - le premier composé polymère obtenu à l'étape (a) ;
   - au moins un composé OCN-Y4-NCO, où Y4 représente un (C₁-C₁₂)alkyle, aryle, ou aryle-(C₁-C₁₂)alkyle-aryle (de préférence un aryle-(C₁-C₁₂)alkyle-aryle),
   - éventuellement au moins un HO-Y5-OH, où Y5 représente un (C₁-C₁₂)alkyle, aryle ou polyalkylène glycol, et
   - éventuellement au moins un H₂N-Y6-NH₂, où Y6 représente un (C₁-C₁₂)alkyle, aryle, ou polyalkylène glycol.

Dans le procédé selon l'invention, le rapport en poids des composés monomères reliés à un résidu peptidique à la totalité des composés monomères est avantageusement compris entre 0,0001/100 et 50/100, de préférence entre 0,001/100 et 10/100, mieux encore entre 0,005/100 et 1/100, voire entre 0,0075/100 et 0,1/100.

Les monomères mis en jeu dans la réaction de polycondensation sont mélangés sans solvant, ou mis en solution dans un solvant, tel que le DMF, le DMSO, ou le dichlorométhane, ou un mélange de ceux-ci. Dans ces conditions, le composé polymère généralement précipite, et peut être isolé, notamment par filtration. La réaction de polycondensation peut être réalisée en présence d'un catalyseur et/ou d'une base, généralement soluble dans le(s) solvant(s) de réaction. Des exemples de bases sont notamment la di-isopropyléthylamine (DIPEA) ou encore la triéthylamine. Un exemple de catalyseur est notamment un complexe de titane (IV) tel que le tétrabutanolate de titane le dilaurate de dioctyl d'étain, les catalyseurs à base de bismuth, ou encore les catalyseurs aminés tertiaires tels que le 1,8-diazabicyclo[5.4.0]undec-7-ène (DBU).

Dans un mode de réalisation particulier, la réaction de polycondensation du procédé de préparation du composé polymère est mise en œuvre par :
(i) mise en contact des composés monomères mis en jeu dans la réaction de polycondensation (c'est-à-dire, les composés monomères de formule Za-C(O)-Y1-C(O)-Zb, HO-Y2-OH, de formule (V), H₂N-Y3-NH₂, HO-Y5-OH, H₂N-Y6-NH₂) en présence d'un catalyseur de polycondensation (notamment un complexe de titane (IV) tel que le tétrabutanolate de titane) ;
(ii) chauffage du mélange obtenu à l'étape (i) à une température comprise entre 100 °C et 200 °C, sous une pression comprise entre 500 mbar et 1,5 bar ;
(iii) chauffage du mélange obtenu l'étape (ii) à une température comprise entre 100 °C et 200 °C, sous une pression comprise entre 0,1 mbar et 200 mbar ; et
(iv) récupération du composé polymère, par exemple par précipitation au moyen d'un ou plusieurs solvants organiques adaptés (tels qu'un mélange de dichlorométhane et de méthanol) et filtration.
Les étapes (i) à (iii) sont avantageusement mises en œuvre sous atmosphère inerte (par exemple, sous atmosphère d'azote ou argon).

Le procédé de l'invention peut être mis en œuvre soit de manière discontinue, par exemple dans un réacteur fermé, ou de manière continue, par exemple par extrusion réactive. Le composé polymère de l'invention peut être mis en forme au moyen de toute technique connue de l'homme du métier. Le composé polymère de l'invention peut être mis en forme pendant le procédé de polymérisation, ou après le procédé de polymérisation, en particulier s'il est obtenu sous la forme de granules ou plaques. Le composé polymère peut en particulier être préparés sous la forme de fils, fibres, ou encore d'un article moulé.

### Procédé de préparation du composé monomère

Le composé monomère selon l'invention est avantageusement préparé par un procédé sur support solide.
Selon une première forme d'exécution, le procédé de préparation d'un monomère selon l'invention comprend :
(a) la fourniture d'un peptide greffé sur un support solide ;
(b) éventuellement la réaction d'un groupe fonctionnel du peptide greffé de l'étape (a) avec un premier groupe fonctionnel d'un espaceur ;
(c) la réaction d'une fonction de couplage reliée à un composé A de formule choisie parmi les formules suivantes :

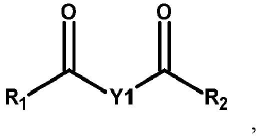

   HO-Y2-OH,

   ou

   H₂N-Y3-NH₂,

   dans lesquelles
   Y1 représente un (C₁-C4₁₂)alkyle, un aryle ou aryle-(C₁-C₁₂)alkyle-aryle,
   Y2 représente un (C₁-C4₁₂)alkyle, un aryle, ou polyalkylène glycol,
   Y3 représente un (C₁-C4₁₂)alkyle, un aryle, ou polyalkylène glycol,
   R₁ et R₂ représentent chacun indépendamment, un chlore, un hydroxy, un (C₁-C₁₂)alkyle-O-, ou un aryle-O-, et
   ladite fonction de couplage est reliée à Y1, Y2 ou Y3 ;
   avec un groupe fonctionnel du peptide greffé de l'étape (a) ou avec un second groupe fonctionnel de l'espaceur installé à l'étape (b) ; et,
(d) l'élimination du support solide du composé ainsi obtenu ;
   le procédé comprenant éventuellement une étape (x) de réaction d'au moins un composé B de formule choisie parmi les formules suivantes :

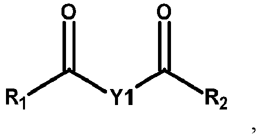

   HO-Y2-OH,

   ou

   H₂N-Y3-NH₂,

   dans lesquelles
   Y1 représente un (C₁-C4₁₂)alkyle, un aryle ou aryle-(C₁-C₁₂)alkyle-aryle,
   Y2 représente un (C₁-C4₁₂)alkyle, un aryle, ou polyalkylène glycol,
   Y3 représente un (C₁-C4₁₂)alkyle, un aryle, ou polyalkylène glycol,
   R₁ et R₂ représentent chacun indépendamment, un chlore, un hydroxy, un (C₁-C₁₂)alkyle-O-, ou un aryle-O-,
   avec le composé obtenu à l'étape (c) ou (d) ; et
   dans lequel les composés A et B sont choisis de telle sorte qu'ils peuvent réagir ensemble selon une réaction de condensation.

Par « réaction de condensation », on entend une réaction d'addition d'un nucléophile sur un électrophile, avec élimination d'une groupement partant. La réaction de condensation est typiquement une réaction d'addition-élimination. Des exemples de réaction de condensation sont notamment :
- l'estérification : réaction entre une fonction alcool et une fonction acide carboxylique ou une fonction dérivée d'acide carboxylique telle qu'un ester, anhydride, ou chlorure d'acyle ; ou
- le couplage amide : réaction entre une fonction amine et une fonction acide carboxylique ou une fonction dérivée d'acide carboxylique telle qu'un ester, anhydride, ou chlorure d'acyle.

Selon un mode particulier, le procédé comprend :
(a) la fourniture d'un peptide greffé sur un support solide ;
(b) éventuellement la réaction d'un groupe fonctionnel du peptide greffé de l'étape (a) avec un premier groupe fonctionnel d'un espaceur ;
(c) la réaction d'une fonction de couplage reliée à un composé A de formule : 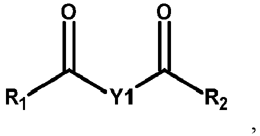 dans laquelle
   Y1 représente un (C₁-C4₁₂)alkyle, un aryle, ou aryle-(C₁-C₁₂)alkyle-aryle, R₁ et R₂ représentent chacun indépendamment, un chlore, un hydroxy, un (C₁-C₁₂)alkyle-O-, ou un aryle-O-, et
   où ladite fonction de couplage est reliée à Y1 ;
   avec un groupe fonctionnel du peptide greffé de l'étape (a) ou avec un second groupe fonctionnel de l'espaceur installé à l'étape (b) ;
(d) l'élimination du support solide du composé obtenu à l'étape (c) ou (x) ;
   le procédé comprenant éventuellement, une étape (x) de réaction d'un composé B de formule

   HO-Y2-OH,

   ou

   H₂N-Y3-NH₂,

   dans lesquelles
   Y2 est un (C₁-C4₁₂)alkyle, un aryle, ou polyalkylène glycol, et
   Y3 est un (C₁-C4₁₂)alkyle, un aryle, ou polyalkylène glycol,
   avec le composé obtenu à l'étape (c) ou (d) ; et
   , dans lequel les composés A et B sont choisis de telle sorte qu'ils peuvent réagir ensemble selon une réaction de condensation.

Selon un mode particulier, le composé B est de formule HO-Y2-OH. Dans un tel mode particulier, les groupes R₁ et R₂ du composé A sont avantageusement identiques, et choisis parmi un (C₁-C₁₂)alkyle-O- et un aryle-O, de préférence un (C₁-C₁₂)alkyle-O- tel qu'un méthyle-O-.

Selon un autre mode particulier, le composé B est de formule H₂N-Y3-NH₂. Dans un tel mode particulier, les groupes R₁ et R₂ du composé A sont choisis parmi un hydroxy, (C₁-C₁₂)alkyle-O- et un aryle-O, et au moins un de R₁ et R₂ est un (C₁-C₁₂)alkyle-O- ou un aryle-O. Dans un tel mode de réalisation, on préfère qu'un de R₁ et R₂ soit un hydroxy, et l'autre de R₁ et R₂ soit un (C₁-C₁₂)alkyle-O- ou un aryle-O, de préférence un (C₁-C₁₂)alkyle-O- tel qu'un méthyle-O-.

Y1 peut en particulier être choisi parmi un (C₁-C₆)alkyle, notamment parmi un éthyle, un propyle, un butyle, un pentyle, un hexyle, et un phényle, de préférence parmi un 1,2-éthyle, 1,3-propyle, 1,4-butyle, 1,5-pentyle, 1,6-hexyle, 1,2-phényle, 1,3-phényle et 1,4-phényle. Plus particulièrement, Y1 peut être un phényle (notamment un 1,3-phényle) ou un propyle (notamment un 1,3-propyle).
Y2 peut en particulier être choisi parmi un éthyle, un propyle, un butyle, un pentyle, un hexyle, un phényle, un polyéthylène glycol, un polypropylène glycol, et un polybutylène glycol, de préférence parmi un 1,2-éthyle, 1,3-propyle, 1,4-butyle, 1,5-pentyle, 1,6-hexyle, 1,2-phényle, 1,3-phényle et 1,4-phényle, mieux encore Y2 est un 1,2-éthyle ou un 1,4-butyle.
La structure « Y1-(fonction de couplage) » est de préférence : 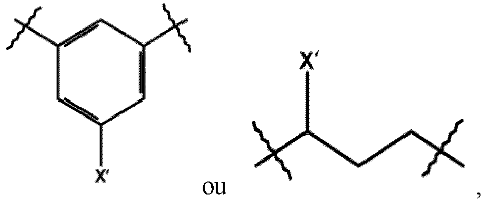 où X' représente la fonction de couplage, notamment -CO₂H (ou un dérivé de CO₂H) ou -NH₂, de préférence -NH₂.

Selon un mode de réalisation particulier, l'étape (x) n'est pas mise en œuvre. Dans un tel mode de réalisation, l'étape (d) comprend l'élimination du support solide du composé obtenu à l'étape (c).
Selon un autre mode de réalisation, l'étape (x) est mise en œuvre après l'étape (c) et avant l'étape (d). Dans un tel mode de réalisation, l'étape (x) comprend la réaction dudit composé B avec le composé obtenu à l'étape (c), et l' étape (d) comprend l'élimination du support solide du composé obtenu à l'étape (x).
Selon un autre mode de réalisation, l'étape (x) est mise en œuvre après l'étape (d). Dans un tel mode de réalisation, l'étape (d) comprend l'élimination du support solide du composé obtenu à l'étape (c), et l'étape (x) comprend la réaction dudit composé B avec le composé obtenu à l'étape (d).

Selon un autre mode particulier, le procédé comprend :
(a) la fourniture d'un peptide greffé sur un support solide ;
(b) éventuellement la réaction d'un groupe fonctionnel du peptide greffé de l'étape (a) avec un premier groupe fonctionnel d'un espaceur ;
(c) la réaction d'une fonction de couplage reliée à un composé A de formule HO-Y2-OH, ou H₂N-Y3-NH₂,
   dans lesquelles
   Y2 est un (C₁-C4₁₂)alkyle, un aryle, ou polyalkylène glycol, et
   Y3 est un (C₁-C4₁₂)alkyle, un aryle, ou polyalkylène glycol,
   ladite fonction de couplage est reliée à Y2 ou Y3 ;
   avec un groupe fonctionnel du peptide greffé de l'étape (a) ou avec un second groupe fonctionnel de l'espaceur installé à l'étape (b) ;
   (x) la réaction d'un composé B de formule : 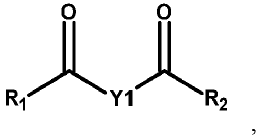 dans lesquelles
   Y1 représente un (C₁-C4₁₂)alkyle, un aryle ou aryle-(C₁-C₁₂)alkyle-aryle,
   Y2 représente un (C₁-C4₁₂)alkyle, un aryle, ou polyalkylène glycol,
   Y3 représente un (C₁-C4₁₂)alkyle, un aryle, ou polyalkylène glycol,
   R₁ et R₂ représentent chacun indépendamment, un chlore, un hydroxy, un (C₁-C₁₂)alkyle-O-, ou un aryle-O-,
   avec le composé obtenu à l'étape (c) ; et
(d) l'élimination du support solide du composé obtenu à l'étape (x) ;
   dans lequel les composés A et B sont choisis de telle sorte qu'ils peuvent réagir ensemble selon une réaction de condensation.

Selon une seconde forme d'exécution, le procédé de préparation d'un monomère selon l'invention comprend :
(a') le greffage sur un support solide d'un composé A' de formule

   HO-Y2-OH

   ou

   H₂N-Y3-NH₂,

   dans lesquelles Y2 est un (C₁-C4₁₂)alkyle, un aryle, ou polyalkylène glycol et Y3 est un (C₁-C4₁₂)alkyle, un aryle, ou polyalkylène glycol ;
(b') la réaction de condensation d'un composé B' de formule : 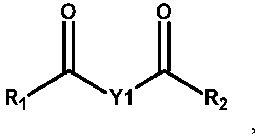 dans laquelle
   Y1 représente un (C₁-C4₁₂)alkyle, un aryle ou aryle-(C₁-C₁₂)alkyle-aryle,
   R₁ et R₂ représentent chacun indépendamment, un chlore, un hydroxy, un (Ci-C₁₂)alkyle-O-, ou un aryle-O-,
   avec le composé A' greffé à l'étape (a') ;
   où une fonction de couplage est reliée à au moins un de Y1, Y2 et Y3, de préférence reliée à Y1;
(c') la réaction de ladite fonction de couplage reliée à au moins un de Y1, Y2 et Y3, avec un groupe fonctionnel d'un peptide ou avec un premier groupe fonctionnel d'un espaceur ;
(d') éventuellement, la réaction d'un second groupe fonctionnel de l'espaceur installé à l'étape (c') avec un groupe fonctionnel d'un peptide ; et
(e') l'élimination du support solide du composé obtenu à l'étape (c') ou (d').

Selon un mode préféré, le composé A' est de formule H₂N-Y3-NH₂ dans laquelle Y3 représente un (C₁-C₁₂)alkyle (de préférence un 1,6-hexyle), et le composé B' est de formule : 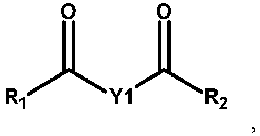 dans laquelle Y1 représente un (C₁-C₁₂)alkyle (de préférence un 1,3-propyle), et R₁ et R₂ représentent chacun indépendamment un (C₁-C₁₂)alkyle-O- (de préférence un méthyle-O-) ou un hydroxy (de préférence, l'un de R₁ et R₂ est un hydroxy et l'autre est un (C₁-C₁₂)alkyle-O-). Dans un tel mode, la fonction de couplage est avantageusement reliée à Y1, et est de préférence -NH₂ ou une forme protégée de -NH₂ (par exemple -NH-Fmoc). Un exemple de structure « Y1-(fonction de couplage) » est : 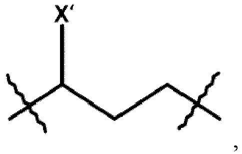 où X' représente la fonction de couplage -NH₂ ou une forme protégée de celle-ci, telle que - NHFmoc.

Selon un mode particulier, l'espaceur est de formule X-(X2)-X', dans laquelle X et X' représentent indépendamment -NH₂, -CO₂H ou un dérivé de -CO₂H (notamment un ester (e.g. -CO₂R où R est un alkyle ou aryle) ou un halogénure d'acyle), ou forment ensemble un anhydride, et X2 représente un (C₁-C₁₂)alkyle, aryle, ou un polyalkylène glycol, de préférence un (C₁-C₆)alkyle, mieux encore un méthyle ou propyle (e.g. 1,3-propyle).

Selon un mode particulier, X et X' sont un -CO₂H, un dérivé de -CO₂H, ou forment ensemble un anhydride.
Selon un autre mode particulier, X est -NH₂ et X' est un -CO₂H ou un dérivé de -CO₂H.

Selon un mode particulier, R₁ et R₂ représentent indépendamment un (C₁-C₁₂)alkyle-O- ou un aryle-O-, mieux encore un (C₁-C₁₂)alkyle-O-, tel qu'un méthyle-O-. Selon un autre mode particulier, R₁ et R₂ sont identiques.

Le support solide peut notamment être une silice, une alumine, du verre, une résine de type polystyrène, une résine de type polyéthylène glycol. Le support solide peut être un support fonctionnalisé par un groupe chimique adapté à la synthèse peptidique, par exemple une résine fonctionnalisée par un groupe 4-((2,4-diméthoxyphényl)(Fmoc-amino)méthyl)phénoxyalkyle (e.g. Résine Rink-Amide^{®}) ou par un groupe chlorure de 2-chlorotrityle.

La réaction de chacune des étapes (b), (c), (x), (b'), (c') et (d') est avantageusement une réaction de condensation, et est de préférence mise en en œuvre en présence d'une base et éventuellement d'un agent de couplage.
Des exemples de bases sont notamment une amine, telle que la diisopropyléthylamine (DIEA), ou une pyridine telle que la diméthylaminopyridine (DMAP).
Des exemples non-limitatifs d'agents de couplage sont notamment ceux utilisés en synthèse peptidique, tels le N-éthyl-N'carbodiimide (EDC), hexafluorophosphate d'O-(7-azabenzotriazol-1-yl)-tétraméthyluronium (HATU), ou hexafluorophosphate de benzotriazole-tétraméthyluronium (HBTU).
Les conditions réactionnelles tels que la quantité d'agent de couplage ou de base, la température, et le solvant peuvent être judicieusement choisies par l'homme du métier.

L'élimination du support à l'étape (d) ou à l'étape (e') peut être notamment réalisée en présence d'un acide, tel que l'acide trifluoroacétique (TFA), et d'un silane, tel que le triisopropylsilane (TIS) dans un solvant adapté.

Dans un mode de réalisation particulier, les composés de formule H₂N-Y3-NH₂ ou HO-Y2-OH, plus particulièrement H₂N-Y3-NH₂, peuvent être utilisés sous une forme protégée. Par « forme protégée », on entend une forme du composé dans laquelle une ou plusieurs fonctions réactives ont été converties en une fonction non réactive en introduisant un groupement protecteur (réaction de protection).
Des exemples de groupements protecteurs pour une fonction réactive NH₂ ou OH sont notamment le groupement Boc (tert-butoxycarbonyle), Fmoc (fluorénylméthoxycarbonyle), NVoc (nitrovératryloxycarbonyle), Alloc (allyloxycarbonyle), Troc (trichloroéthoxycarbonyle), Cbz (carboxybenzyle), trityle, THP (tétrahydropyranyle), ou un groupe silylé tel que TBS (tert-butyldiméthylsilyle) ou TMS (triméthylsilyle).

Un groupement protecteur adapté et des conditions pour la protection d'une ou plusieurs fonctions réactives peuvent être judicieusement choisis par l'homme de l'art.

La déprotection consiste à retirer le groupement protecteur d'une ou plusieurs fonctions réactives, dans le but de restaurer sa réactivité. La déprotection peut être mise en œuvre pendant ou entre n'importe quelle étape du procédé de préparation du composé monomère. Les conditions pour la déprotection d'une ou plusieurs fonctions réactives protégées peuvent être judicieusement choisis par l'homme de l'art.

Selon un mode particulier, plusieurs fonctions réactives d'un composé de formule H₂N-Y3-NH₂ ou HO-Y2-OH (plus particulièrement H₂N-Y3-NH₂) sont protégées, et les groupements protecteurs correspondants sont identiques ou différents les uns des autres. On préfère que ces groupements protecteurs soient différents, voire orthogonaux les uns par rapport aux autres. L'orthogonalité permet de protéger ou déprotéger sélectivement différentes fonctions d'un même composé. Deux groupements protecteurs orthogonaux sont notamment Boc et Fmoc.

### Utilisations

La présente invention concerne également une utilisation d'un composé polymère de l'invention dans la fabrication d'un matériau.

Un autre objet de l'invention est un matériau comprenant un composé polymère de l'invention.

Dans un mode de réalisation particulier, ledit matériau est un biomatériau, et de préférence un matériau ou biomatériau antibactérien.
Dans un autre mode de réalisation, ledit matériau est un matériau de détection, plus précisément un matériau qui réagit à une modification environnementale, tel qu'un biocapteur. Dans un tel mode de réalisation, la présence de peptides greffés sur le composé polymère, notamment de peptides bioactifs, permet au matériau d'être utilisé comme matériau de détection tel qu'un biocapteur.
Dans un autre mode de réalisation, ledit matériau est résorbable, voire biorésorbable.
Ledit matériau peut être notamment un matériau antibactérien, anti-inflammatoire, biocide et/ou fongicide.

La présente invention concerne également une utilisation d'un composé polymère de l'invention dans la fabrication d'un article.

Un autre objet de l'invention est un article comprenant un composé polymère de l'invention, tel qu'un dispositif médical ou un tissu.

Ledit article a avantageusement des propriétés antibactériennes, anti-inflammatoires, biocides et/ou fongicides.
Ledit article peut notamment être un fil, une fibre, un tissu, un filtre pour les fluides corporels, une suture, un cathéter, un pansement ou un dispositif implantable. Des exemples de tissus sont notamment une chaussette, une semelle, un bas, une chemise, un maillot ou une blouse.
Ledit article peut notamment être un bouchon, un joint, une capsule, un couvercle, ou un robinet. Ledit article peut être tout ou partie de bouteilles, de flacons, de pots, de boîtes, de bidons, de barriques, de cuves et de divers récipients utilisés pour le conditionnement et/ou le stockage de produits alimentaires, diététiques, cosmétiques, dermatologiques ou pharmaceutiques. Ledit article peut être un article adapté pour la conservation et/ou la distribution de produits pharmaceutiques (par exemple, les cuillers de sirop, les seringues pour administrer par exemple un sirop, les plaquettes de comprimés ou gélules, les poches de perfusion, les tubes, canules, pompes et flacons) ou alimentaires (par exemple, les barquettes, opercules et films d'emballage).
Ledit article ou ledit matériau selon l'invention peut être utilisé pour fabriquer un équipement de bloc opératoire, un équipement de pièce stérile, un instrument chirurgical, ou une paillasse (plan de travail) de laboratoire.

### EXEMPLES

L'invention sera mieux comprise à la lumière des exemples suivants, qui sont donnés à titre purement illustratif et n'ont pas pour but de limiter la portée de l'invention, définie par les revendications annexées.

### A. Préparation des monomères substitués par un peptide

Les peptides sont synthétisés sur support solide en utilisant un synthétiseur de peptides de type Symphony X (Protein Technologies, Inc., USA) en stratégie Fmoc/tert-butyl utilisant le bullage d'azote comme méthode d'agitation pour les cycles de couplages et de déprotection des groupements Fmoc en position N-terminal.
Les synthèses sont réalisées à une échelle de 0.25 mmol sur de la résine Fmoc-Rink-Amide polystyrène (481 mg/ 0.52 mmol/g) ou chlorure de 2-chlorotrityle (892 mg, 0,28 mmol/g). Le cycle standard de déprotection-couplage pour chaque résidu comprend six étapes : Lavage de la résine avec 5 ml de DMF (3 x 30 sec). Déprotection du groupement Fmoc avec 5 ml de pipéridine à 20% dans du DMF (3 x 3 min). Lavage de la résine avec 5 mL de DMF (3 x 30 sec). Couplage du résidu Fmoc-AA pendant 60 min en utilisant de l'HATU et de la DIEA comme agent de couplage. À la fin du cycle de couplage, une étape de capping est réalisée avec 5 mL d'Ac2O à 10% dans du DMF pendant 7 min, puis la résine est lavée 5 mL de DMF (3 x 30 sec).
Le clivage de la résine (Fmoc-Rink-Amide) est réalisé 2 x 60 minutes dans un cocktail de clivage (TFA/TIS/H2O 95/2,5/2,5) pour les séquences déprotégées et 2 x 60 minutes dans un cocktail de clivage (AcOH/TFE/DCM 10/20/70) pour les séquences protégées (résine chlorure de 2-chlorotrityle). Une fois la résine filtrée, la solution est évaporée sous vide et le peptide est précipité dans du diéthyl éther. Le peptide précipité est centrifugé (3500 RCF) et le surnageant est éliminé. (X3).Le peptide sous forme de sels de TFA est alors solubilisé dans un mélange H2O/ACN avant d'être congelé et lyophilisé.
Les peptides sont analysés par UPLC et spectrométrie de masse ESI-MS, équipé d'une colonne BEH C18 (WATERS), 150^{∗}2,1 mm (150 × 2,1 mm) (débit : 0,6 ml/min). Les solvants A et B étant à 0,1 % de TFA dans l'eau et à 0,1 % de TFA dans l'acétonitrile.
La purification des peptides est effectuée sur un appareil WATERS HPLC 4000 équipé d'un détecteur UV 486 et d'une colonne Vydac Denali 10 µm C18 120 Å (310 x 25 mm) à un débit de 50 ml/min. Les solvants utilisés étant à 0,1 % de TFA dans l'eau (tampon A) et à 0,1 % de TFA dans l'acétonitrile (tampon B).

### Exemple 1 : Préparation de monomères diméthyl isophthalate substitués par un peptide

### a) Peptide : H-(RF)4-NH₂

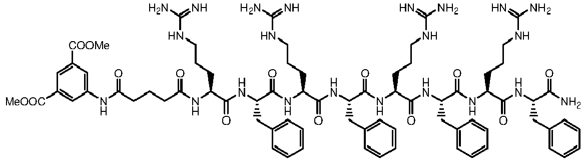

Le peptide antibactérien H-(RF)4-NH₂ est synthétisé sur une résine Rink Amide (charge 0,52 mmol/g, échelle de synthèse : 0.25 mmol) en stratégie Fmoc/tBu. Chaque couplage est suivi d'une déprotection du groupement Fmoc en N-ter. Le peptide est ensuite fonctionnalisé sur support après introduction d'un espaceur (ou « linker ») glutarique dans le DCM pendant 15 minutes en utilisant l'anhydride glutarique (5éq) en présence de DIEA (7 éq). La résine est lavée (3*DMF, 1 *MeOH et 1 *DCM). L'amino diméthylisophthalate est couplé dans le DMF en présence de 3éq. d'EDC.HCl et 1éq. de DMAP. La réaction est laissée sous agitation pendant une nuit puis la résine est lavée (3*DMF, 1*MeOH et 1*DCM) et clivée dans un mélange DCM/ TFA/TIS/H2O 50/47.5/2.5/2.5 pendant 2X1 h. La solution de « clivage » est concentrée sous pression réduite puis le peptide antibactérien est précipité dans l'éther diéthylique et enfin purifié par HPLC préparative (Colonne Vydac Denali 10 µm C18 120 Å (310 x 25 mm) utilisant un gradient de 20 à 60% de tampon B en 40 min.).
**ESI-MS** (*m*/z): [M+H]⁺ théorique C₇₅H₁₀₃N₂₂O₁₄: 1536.78, expérimental: 1536.84
**HPLC** (temps de rétention, min) : 3,24

### b) Peptide H-(R)₂-Palm

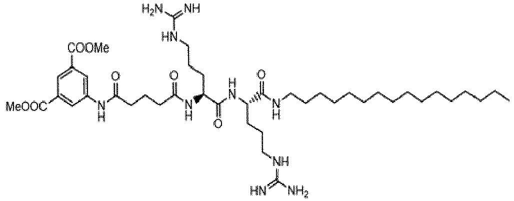

Le peptide antibactérien H-(R)₂-Palm est synthétisé sur une résine Rink (charge 0,52 mmol/g, échelle de synthèse : 0.25 mmol) préalablement fonctionnalisé par un benzotriazole en stratégie Fmoc/tBu. Chaque couplage est suivi d'une déprotection du groupement Fmoc en N-ter. Préparation de la résine :
La résine Rink-amide est préalablement traitée à la pipéridine (20% dans le DMF) et lavée (3*DMF, 1 *MeOH et 1 *DCM). L'acide 4-amino 3-nitrobenzoique est couplé dans le DMF en présence de 3éq. D'HATU et 6 éq. de DIEA. La réaction est laissée sous agitation 2h puis la résine est lavée (3*DMF, 1 *MeOH et 1 *DCM), capée a l'anhydride acétique (15 ml d'une solution à 10% v/v dans le DCM) puis lavée (2*DCM, 3*DMF, 1 *MeOH et 1 *DCM). La réduction du groupe nitro est réalisée dans une solution de 5g de SnCl2 (2H₂O) et 900uL DBU pour 10 ml de DMF qui est ajoutée à la résine sous bullage à l'azote pour 10 minutes. La réaction est agitée pendant 15h, seringue ouverte et lavée (3*DMF, 3*DCM).
L'introduction de la séquence peptidique -(R)₂- est réalisée en stratégie Fmoc/tBu. Chaque couplage est suivi d'une déprotection du groupement Fmoc en N-ter. Le peptide est ensuite fonctionnalisé sur support après introduction d'un espaceur (ou « linker ») glutarique dans le DCM pendant 15 minutes en utilisant l'anhydride glutarique (5éq) en présence de DIEA (7 éq). La résine est lavée (3*DMF, 1 *MeOH et 1 *DCM). L'amino diméthylisophthalate est couplé dans le DMF en présence de 3éq. d'EDC.HCl et 1éq. de DMAP. La réaction est laissée sous agitation pendant une nuit puis la résine est lavée (3*DMF, 1 *MeOH et 1 *DCM).

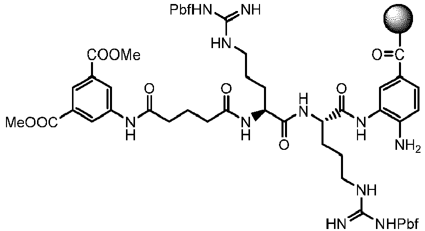

La résine est alors traitée par l'isoamyl nitrite (10 équivalents) pendant 90 minutes (lavage DCM x5).

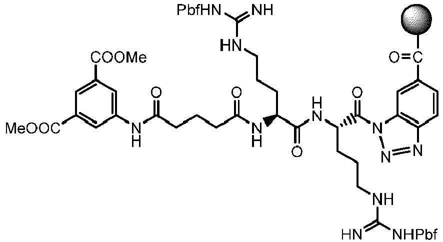

Le nucléophile (4 équivalents / dans ce cas l'hexadécylamine) en solution dans le DCM en présence de DIEA (8 équivalents) est bullé à l'azote 20 minutes avant la fin de la cyclisation, puis est introduit immédiatement sur la résine. La réaction est agitée induisant le clivage du peptide de la résine.

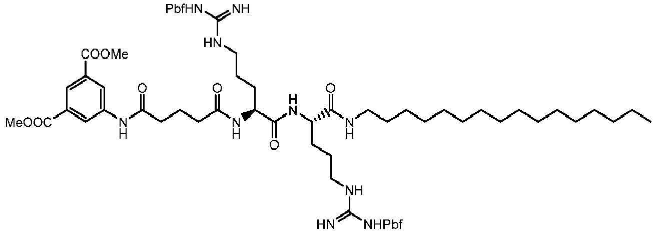

Le peptide est clivée dans un mélange DCM/ TFA/TIS/H2O 50/47.5/2.5/2.5 pendant 2x1 h. La solution de « clivage » est concentré sous pression réduite puis le peptide antibactérien est précipité dans l'éther diéthylique et enfin purifié par HPLC préparative (Colonne Vydac Denali 10 µm C18 120 Å (310 x 25 mm) utilisant un gradient de 20 à 60% de tampon B en 40 min.). **ESI-MS** (*m*/z): [M+H]⁺ théorique C₄₃H₇₅N₁₀O₈: 860,13 expérimental: 859.80.

### Exemple 2 : Préparation de monomères diméthyl glutamate substitués par un peptide

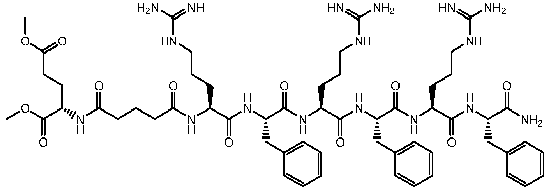

Le peptide antibactérien H-(RF)3-NH₂ est synthétisé sur une résine Rink (charge 0,52 mmol/g, échelle de synthèse : 0.25 mmol) en stratégie Fmoc/tBu. Chaque couplage est suivi d'une déprotection du groupement Fmoc en N-ter. Le peptide est ensuite fonctionnalisé sur support après introduction d'un espaceur glutarique dans le DCM pendant 15 minutes en utilisant l'anhydride glutarique (5éq) en présence de DIEA (7 éq). la résine est lavée (3*DMF, 1 *MeOH et 1 *DCM).Le diméthyl glutamate est couplé dans le DMF en présence de 3éq. d'EDC.HCl et 1éq. de DMAP. La réaction est laissée sous agitation pendant une nuit puis la résine est lavée (3*DMF, 1 *MeOH et 1 *DCM) et clivée dans un mélange DCM/ TFA/TIS/H2O 50/47.5/2.5/2.5 pendant 2x1 h. La solution de « clivage » est concentré sous pression réduite puis le peptide antibactérien est précipité dans l'éther diéthylique et enfin purifié par HPLC préparative (Colonne Vydac Denali 10 µm C18 120 Å (310 x 25 mm) utilisant un gradient de 20 à 60% de tampon B en 40 min.).
**ESI-MS** (*m*/z): [M+H]⁺ théorique C₅₇H₈₄N₁₇O₁₂: 1199.40, expérimental: 1199.70
**HPLC** (temps de rétention, min) : 3,64

### Exemple 3 : Préparation de monomères bis(2-hydroxyéthyl) isophthalate substitués par un peptide

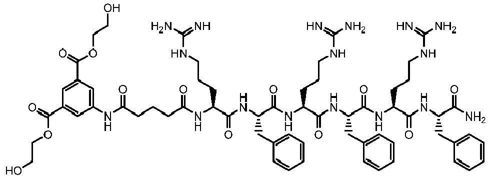

Le peptide antibactérien H-(RF)3-NH₂ est synthétisé sur une résine Rink Amide (charge 0,52 mmol/g, échelle de synthèse : 0.25 mmol) en stratégie Fmoc/tBu. Chaque couplage est suivi d'une déprotection du groupement Fmoc en N-ter. Le peptide est ensuite fonctionnalisé sur support après introduction d'un espaceur glutarique dans le DCM pendant 15 minutes en utilisant l'anhydride glutarique (5éq) en présence de DIEA (7 éq). la résine est lavée (3*DMF, 1 *MeOH et 1 *DCM). Le 5-aminoisophthalic acid (3éq.) est couplé dans le DMF après préactivation de la résine au HATU (3éq.) en présence de DIEA (6éq.) durant 15 minutes. L'éthylène glycol (12éq.) est couplé dans le DMF en présence de 6 éq. d'EDC.HCl et 1éq. de DMAP. La réaction est laissée sous agitation pendant une nuit. puis la résine est lavée (3*DMF, 1 *MeOH et 1 *DCM) et clivée dans un mélange DCM/ TFA/TIS/H2O 50/47.5/2.5/2.5 pendant 2X1 h. La solution de « clivage » est concentrée sous pression réduite puis le peptide antibactérien est précipité dans l'éther diéthylique et enfin purifié par HPLC (Colonne Vydac Denali 10 µm C18 120 Å (310 x 25 mm) utilisant un gradient de 20 à 60% de tampon B en 40 min.).
**ESI-MS** (*m*/z): [M+H]⁺ théorique C₆₂H₈₆N₁₇O₁₄: 1293.47, expérimental: 1293.70
**HPLC** (temps de rétention, min) : 2,71

### Exemple 4 : Préparation de monomères bis(4-hydroxybutyl) isophthalate substitués par un peptide

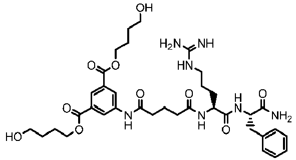

### Méthode A :

Le peptide antibactérien H-(RF)-NH₂ est synthétisé sur une résine Rink Amide (charge 0,52 mmol/g, échelle de synthèse : 0.25 mmol) en stratégie Fmoc/tBu. Chaque couplage est suivi d'une déprotection du groupement Fmoc en N-ter. Le peptide est ensuite fonctionnalisé sur support après introduction d'un espaceur glutarique dans le DCM pendant 15 minutes en utilisant l'anhydride glutarique (5éq) en présence de DIEA (7 éq). la résine est lavée (3*DMF, 1 *MeOH et 1 *DCM). L'acide 5-aminoisophthalique (3éq.) est couplé dans le DMF après préactivation de la résine au HATU (3éq.) en présence de DIEA (6éq.) durant 15 minutes. Le butylène glycol (12éq.) est couplé dans le DMF en présence de 6éq. d'EDC.HCl et 1éq. de DMAP. La réaction est laissée sous agitation pendant une nuit. puis la résine est lavée (3*DMF, 1 *MeOH et 1 *DCM) et clivée dans un mélange DCM/ TFA/TIS/H2O 50/47.5/2.5/2.5 pendant 2X1 h. La solution de « clivage » est concentré sous pression réduite puis le peptide antibactérien est précipité dans l'éther diéthylique et enfin purifié par HPLC préparative. (Colonne Vydac Denali 10 µm C18 120 Å (310 x 25 mm) utilisant un gradient de 20 à 60% de tampon B en 40 min.).
**ESI-MS** (*m*/z): [M+H]⁺ théorique C₃₆H₅₂N₇O₁₀: 742.84, expérimental: 742.54
**HPLC** (temps de rétention, min) : 3,29

### Méthode B :

Le peptide antibactérien H-(RF)-NH₂ est synthétisé sur une résine Rink Amide (charge 0,52 mmol/g, échelle de synthèse : 0.25 mmol) en stratégie Fmoc/tBu. Chaque couplage est suivi d'une déprotection du groupement Fmoc en N-ter. Le peptide est ensuite fonctionnalisé sur support après introduction d'un espaceur (ou « linker ») glutarique dans le DCM pendant 15 minutes en utilisant l'anhydride glutarique (5éq) en présence de DIEA (7 éq). La résine est lavée (3*DMF, 1 *MeOH et 1 *DCM). L'amino diméthylisophthalate est couplé dans le DMF en présence de 3éq. d'EDC.HCl et 1éq. de DMAP. La réaction est laissée sous agitation pendant une nuit puis la résine est lavée (3*DMF, 1*MeOH et 1*DCM) et clivée dans un mélange DCM/ TFA/TIS/H2O 50/47.5/2.5/2.5 pendant 2X1 h. La solution de « clivage » est concentré sous pression réduite puis le peptide antibactérien est précipité dans l'éther diéthylique et enfin purifié par HPLC préparative. (Colonne Vydac Denali 10 µm C18 120 Å (310 x 25 mm) utilisant un gradient de 20 to 60% de tampon B en 40 min.).

Peptide diméthyl isophthalate :
**ESI-MS** (*m*/z): [M+H]⁺ théorique C₃₀H₄₀N₇O₈: 626.68, expérimental: 626.32
**HPLC** (temps de rétention, min) : 3,38

Le peptide dimethyl isophthalate est ensuite transestérifié en solution dans le 1,4 butanediol en présence de catalyseur pendant 2h entre 150°C et 180°C.
**ESI-MS** (*m*/z): [M+H]⁺ théorique C₃₆H₅₂N₇O₁₀: 742.84, expérimental: 742.33
**HPLC** (temps de rétention, min) : 3,25

Il a également pu être observé que la transestérification (dans les mêmes conditions que pour un composé diester, à savoir solution dans le 1,4 butanediol en présence de catalyseur pendant 2h entre 150°C et 180°C) à partir d'un composé peptidique diacide conduisait à une dégradation du peptide.

### Exemple 5 : Préparation de monomères de type amino ester monoamide substitués par un peptide (peptide (FR)4))

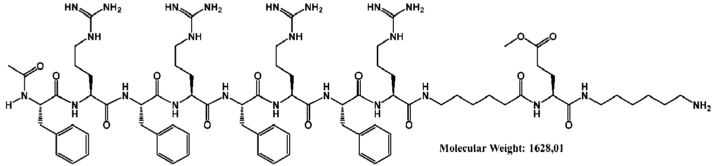

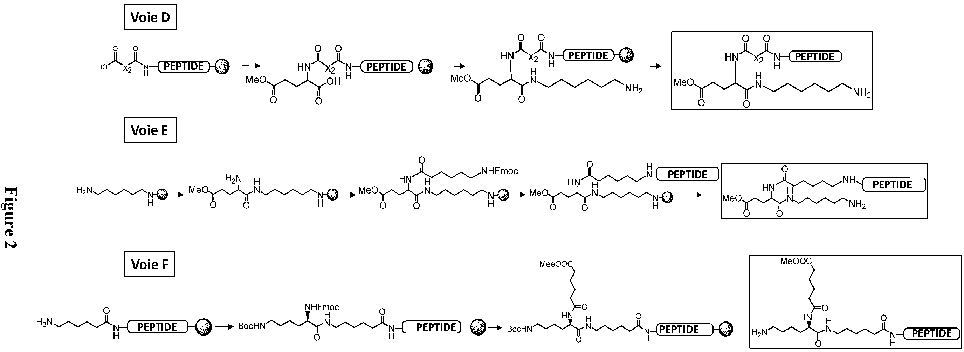

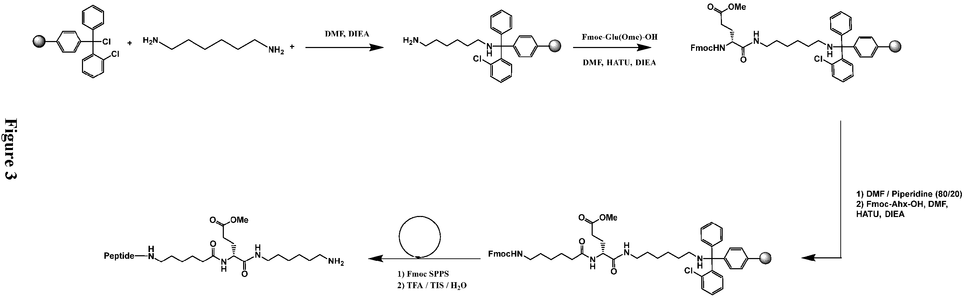

Le bloc élémentaire est synthétisé sur une résine chlorure de 2-chlorotrityle (charge 0,28 mmol/g, échelle de synthèse : 0.25 mmol) en stratégie Fmoc/tBu (Figure 2 - Voie E, et Figure 3). Après avoir fait préalablement gonflé la résine dans le DCM, l'hexaméthylene diamine (5 éq.) est introduite sur la résine dans le DMF en présence de DIEA (11 éq.) pendant 24h. La réaction est laissée sous agitation pendant une nuit, puis la résine est lavée (3*DMF, 1 *MeOH et 1 *DCM). Après le couplage de l'acide aminé Fmoc-Glu(Ome)-OH en présence de 3 équivalents d'HATU et de 6 équivalents de DIEA, la résine est lavée (3*DMF, 1 *MeOH et 1 *DCM). L'espaceur Fmoc-Ahx-OH est introduit dans le DMF en présence de 3 équivalents d'HATU et de 6 équivalents de DIEA, la résine est lavée (3*DMF, 1 *MeOH et 1 *DCM). La synthèse du peptide est alors réalisée classiquement en utilisant un synthétiseur de peptide de type Symphony X (Protein Technologies, Inc., USA) en stratégie Fmoc/tert-butyl utilisant le bullage d'azote comme méthode d'agitation pour les cycles de couplages et de déprotection des groupements Fmoc en position N-terminal.
Le clivage du peptide de la résine est réalisé selon 2 méthodes :

### Méthode A (le peptide est clivé et déprotégé) :

La résine est clivée dans un mélange DCM/ TFA/TIS/H2O 50/47.5/2.5/2.5 pendant 2X1 h. La solution de « clivage » est concentré sous pression réduite puis le peptide antibactérien est précipité dans l'éther diéthylique et enfin purifié par HPLC préparative (Colonne Vydac Denali 10 µm C18 120 Å (310 x 25 mm) utilisant un gradient de 20 to 60% de tampon B en 40 min.).
**ESI-MS** (*m*/z): [M+H]⁺ théorique C₈₀H₁₂₃N₂₄O₁₃: 1629.01, expérimental: 1629.37
**HPLC** (temps de rétention, min) : 2,63

### Méthode B (le peptide est clivé sous sa forme protégée) :

La résine est clivée dans un mélange AcOH/TFE/DCM (10/20/70) pendant 2X1 h. La solution de « clivage » est concentré sous pression réduite puis le peptide antibactérien est précipité dans l'éther diéthylique et enfin purifié par HPLC préparative(Colonne Vydac Denali 10 µm C18 120 Å (310 x 25 mm) utilisant un gradient de 20 to 60% de buffer B en 40 min.). 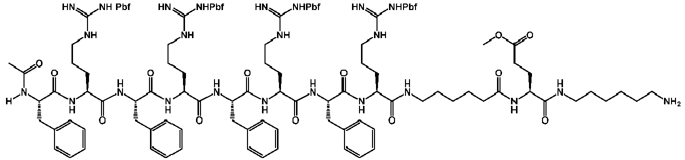 **ESI-MS** (*m*/z): [M+H]⁺ théorique C₁₃₂H₁₈₇N₂₄O₂₅S₄: 2638.32, expérimental: 2638.28
**HPLC** (temps de rétention, min) : 5.97

### B. Préparation des polymères

### Exemple 6 : Préparation des copolymères de poly(butylène adipate-co-téréphthalate) (PBAT)

La synthèse des copolymères de poly(butylène adipate-co-téréphthalate) est réalisée par polycondensation en deux étapes en présence de catalyseur (Ti(OBu)₄).
La première étape est une transestérification entre les diesters et le 1,4- butanediol en excès pour obtenir des oligomères, la deuxième étape est la polycondensation de ces oligomères pour éliminer le 1,4-butanediol en excès pour former des polyesters à plus haut poids moléculaire (Mw) (schéma 1).

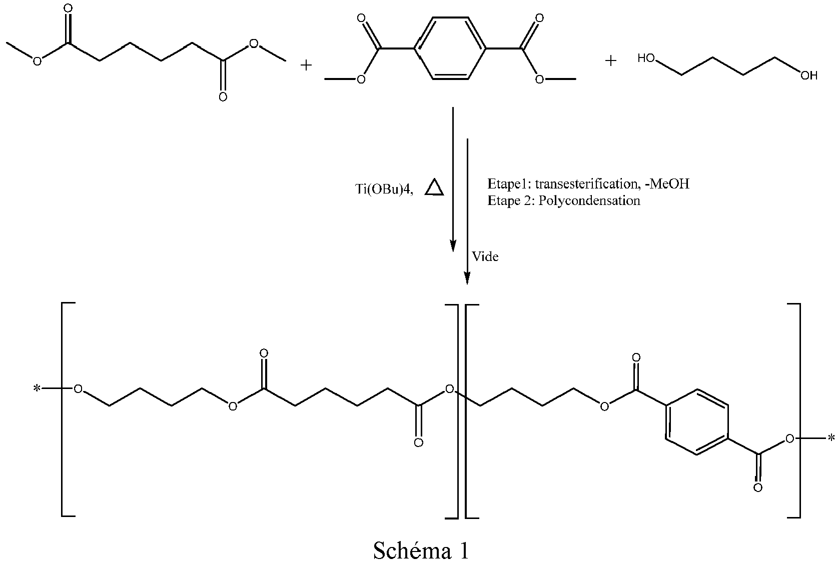

Lors de la première étape qui consiste en une transesterification, les éléments suivants, diméthyl adipate (DMA, 12.66 g (0.5 eq.)) et diméthyl téréphthalate (DMT, 14.11 g (0.5 eq.)) en quantité stoechiométrique (total en fonctions diester représentant 1 équivalent (DMA+DMT=1éq.)) sont ajoutés au 1,4-butanediol en léger excès (15.07g1.15éq.) et au catalyseur (0.1%) dans un ballon tricol de 250 ml équipé d'un agitateur mécanique étanche, d'une entrée d'azote et d'une tête de distillation relié à un tube gradué pour recueillir le méthanol distillé.
Le réacteur est purgé à l'argon par 3 cycles consécutifs et l'étape de transesterification est réalisée sous atmosphère d'argon à 160°C durant 1h jusqu'à élimination du méthanol sous-produit de réaction. La polycondensation est réalisée en passant le système sous vide (20mbar) durant 5h entre 160°C et 180°C.
Après refroidissement, le milieu réactionnel est dissout dans du dichlorométhane et précipité dans le méthanol, filtré et séché.

### Exemple 7 : Préparation des copolymères de poly(butylène succinate-co-téréphthalate)

La synthèse des copolymères de poly(butylène succinate-co- téréphthalate) avec x% d'unités aromatiques sont préparés par polycondensation en deux étapes en présence de catalyseur (Ti(OBu)₄).
La première étape est une transestérification entre les diesters et le 1,4- butanediol en excès pour obtenir des oligomères, la deuxième étape est la polycondensation de ces oligomères pour éliminer le 1,4-butanediol en excès pour former des polyesters à plus haut poids moléculaire (Mw) (Schéma 2).

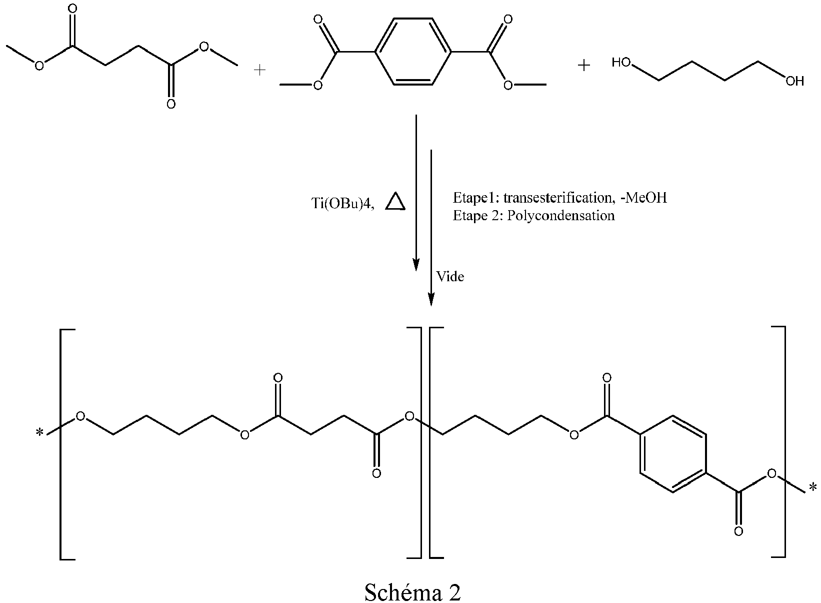

Lors de la première étape qui consiste en une transesterification, les éléments suivants, diméthyl succinate (DMS 15.94g 0.75 éq.) et diméthyl téréphthalate (DMT 7.06g 0.25éq.) en quantité stoechiométrique (total en fonctions diester représentant 1 équivalent (DMS+DMT=1éq.)) sont ajoutés au 1,4-butanediol en léger excès (BD 15.07g 1.15éq.) et au catalyseur (0.1%) dans un ballon tricol de 250 ml équipé d'un agitateur mécanique étanche, d'une entrée d'azote et d'une tête de distillation relié à un tube gradué pour recueillir le méthanol distillé.
Le réacteur est purgé à l'argon par 3 cycles consécutifs et l'étape de transesterification est réalisée sous atmosphère d'argon à 160°C durant 1h jusqu'à élimination du méthanol sous-produit de réaction. La polycondensation est réalisée en passant le système sous vide (20mbar) durant 5h entre 160°C et 180°C.
Après refroidissement, le milieu réactionnel est dissout dans du dichlorométhane et précipité dans le méthanol, filtré et séché.

### Exemple 8 : Préparation des copolymères de poly(butylène adipate-co-téréphthalate-co-isophthalatopeptide) (PBAT-co-peptide)

La synthèse des copolymères de poly(butylène adipate-co-téréphthalate-co-isophthalatopeptide) avec 50% molaire d'unités aromatiques et avec 0.5% massique d'unités peptidiques sont préparés par polycondensation en deux étapes en présence de catalyseur (Ti(OBu)₄).
La première étape est une transestérification entre les diesters et le 1,4- butanediol en excès pour obtenir des oligomères, la deuxième étape est la polycondensation de ces oligomères pour éliminer le 1,4-butanediol en excès pour former des polyesters à plus haut poids moléculaire (Mw) (Schéma 3).

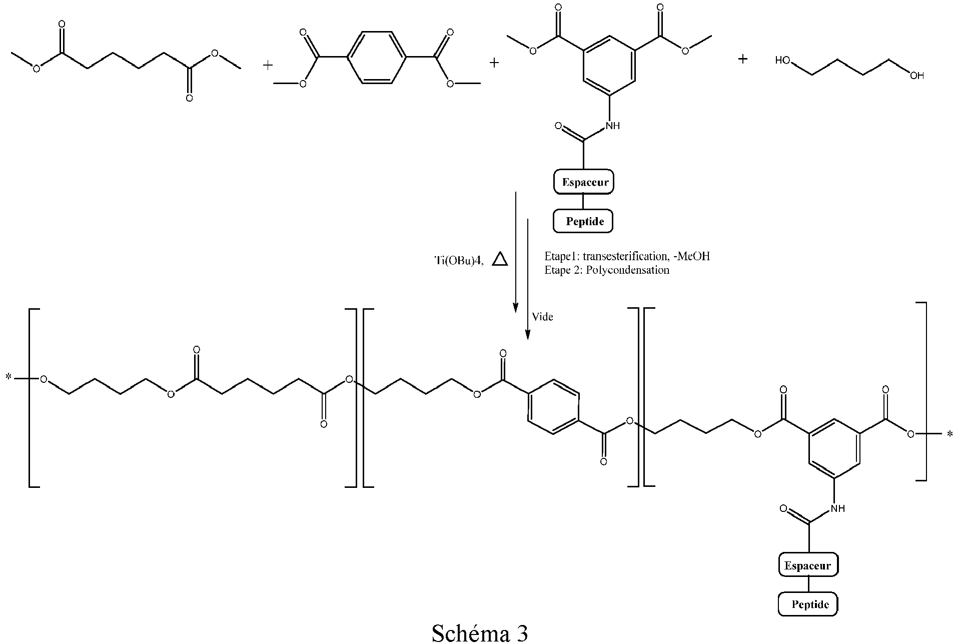

Lors de la première étape qui consiste en une transesterification, les éléments suivants, diméthyl adipate (DMA, 12.16g 0.5 éq.), diméthyl téréphthalate (DMT 14.11g 0.5 éq) et isophthalatopeptide (DMIP peptide = monomère de l'Exemple la, 255mg 0.5% massique ) en quantité stoechiométrique (total en fonctions diester représentant 1 équivalent (DMA+DMT+DMIPpeptide=1éq.)) sont ajoutés au 1,4-butanediol en léger excès (BD 15.07g 1.15éq.) et au catalyseur (0.1%) dans un ballon tricol de 250 ml équipé d'un agitateur mécanique étanche, d'une entrée d'azote et d'une tête de distillation relié à un tube gradué pour recueillir le méthanol distillé.
Le réacteur est purgé à l'argon par 3 cycles consécutifs et l'étape de transesterification est réalisée sous atmosphère d'argon à 160°C durant 1h jusqu'à élimination du méthanol sous-produit de réaction. La polycondensation est réalisée en passant le système sous vide (20mbar) durant 5h entre 160°C et 180°C.
Après refroidissement, le milieu réactionnel est dissout dans du dichlorométhane et précipité dans le méthanol, filtré et séché.

### Exemple 9: Préparation des copolymères de poly(butylène succinate-co-téréphthalate-co-isophthalatopeptide)

La synthèse des copolymères de poly(butylène succinate-co-téréphthalate-co-isophthalatopeptide) avec 50% molaire d'unités aromatiques et avec 0,5% massique d'unités peptidiques sont préparés par polycondensation en deux étapes en présence de catalyseur (Ti(OBu)₄).
La première étape est une transestérification entre les diesters et le 1,4- butanediol en excès pour obtenir des oligomères, la deuxième étape est la polycondensation de ces oligomères pour éliminer le 1,4-butanediol en excès pour former des polyesters à haut poids moléculaire (Mw) (Schéma 4).

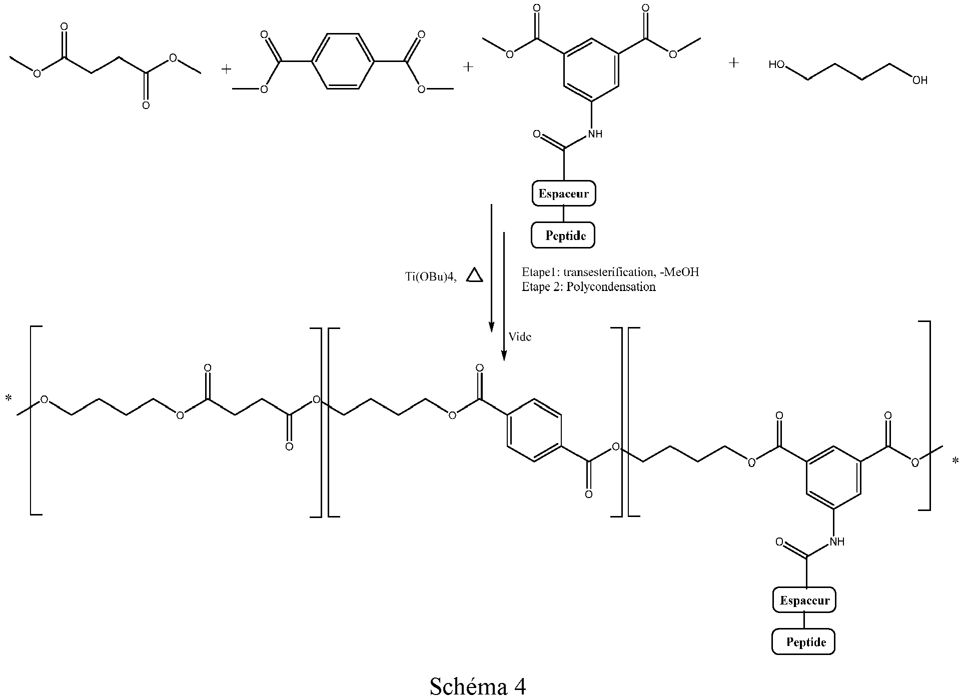

Lors de la première étape qui consiste en une transesterification, les éléments suivants, diméthyl succinate (DMS 15.94g 0.75 éq.), diméthyl téréphthalate (DMT 7.06g 0.25 éq.) et isophthalatopeptide (DMIP peptide = monomère de l'Exemple la, 235mg 0.5% massique) en quantité stoechiométrique (total en fonctions diester représentant 1 équivalent (DMS+DMT+DMIPpeptide=1éq.)) sont ajoutés au 1,4-butanediol en léger excès (BD 15.07 g 1.15éq.) et au catalyseur (0.1%) dans un ballon tricol de 250 ml équipé d'un agitateur mécanique étanche, d'une entrée d'azote et d'une tête de distillation relié à un tube gradué pour recueillir le méthanol distillé.
Le réacteur est purgé à l'argon par 3 cycles consécutifs et l'étape de transesterification est réalisée sous atmosphère d'argon à 160°C durant 1h jusqu'à élimination du méthanol sous-produit de réaction. La polycondensation est réalisée en passant le système sous vide (20mbar) durant 5h entre 160°C et 180°C.
Après refroidissement, le milieu réactionnel est dissout dans du dichlorométhane et précipité dans le méthanol, filtré et séché.

### Exemple 10 : Préparation des polymères de poly(butylène adipate)

Les synthèse des polymères de poly(butylène adipate) sont préparées par polycondensation en deux étapes en présence de catalyseur (Ti(OBu)₄).
La première étape est une transestérification entre le diester et le 1,4- butanediol en excès pour obtenir des oligomères, la deuxième étape est la polycondensation de ces oligomères pour éliminer le 1,4-butanediol en excès pour former des polyesters à plus haut poids moléculaire (Mw) (Schéma 5).

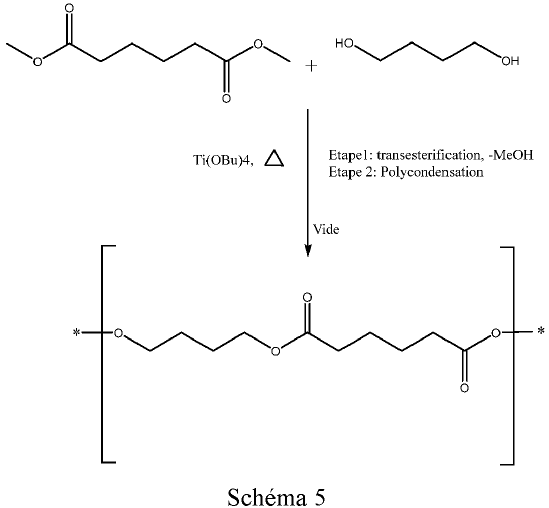

Lors de la première étape qui consiste en une transesterification, les éléments suivants, diméthyl adipate (DMA 25.33g 1éq.), sont ajoutés au 1,4-butanediol en léger excès (BD 15.07g 1.15éq.) et au catalyseur (0.1%) dans un ballon tricol de 250 ml équipé d'un agitateur mécanique étanche, d'une entrée d'azote et d'une tête de distillation relié à un tube gradué pour recueillir le méthanol distillé.
Le réacteur est purgé à l'argon par 3 cycles consécutifs et l'étape de transesterification est réalisée sous atmosphère d'argon à 160°C durant 1h jusqu'à élimination du méthanol sous-produit de réaction. La polycondensation est réalisée en passant le système sous vide (20mbar) durant 5h entre 160°C et 180°C.

Après refroidissement, le milieu réactionnel est dissout dans du dichlorométhane et précipité dans le méthanol, filtré et séché.

### Exemple 11 : Préparation des polymères de poly(butylène succinate) (PBS)

Les synthèse des polymères de poly(butylène succinate) sont préparées par polycondensation en deux étapes en présence de catalyseur (Ti(OBu)₄).
La première étape est une transestérification entre le diester et le 1,4- butanediol en excès pour obtenir des oligomères, la deuxième étape est la polycondensation de ces oligomères pour éliminer le 1,4-butanediol en excès pour former des polyesters à haut poids moléculaire (Mw) (Schéma 6).

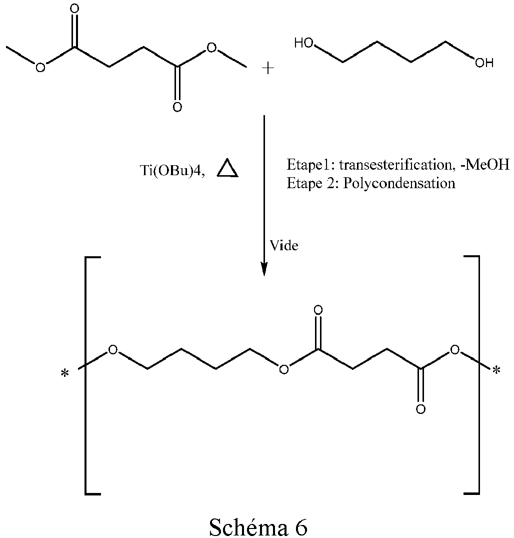

Lors de la première étape qui consiste en une transesterification, les éléments suivants, diméthyl succinate (DMS 21.25g léq), sont ajoutés au 1,4-butanediol en léger excès (BD 15.07g 1.15éq ) et au catalyseur (0.1%) dans un ballon tricol de 250 ml équipé d'un agitateur mécanique étanche, d'une entrée d'azote et d'une tête de distillation relié à un tube gradué pour recueillir le méthanol distillé.
Le réacteur est purgé à l'argon par 3 cycles consécutifs et l'étape de transesterification est réalisée sous atmosphère d'argon à 160°C durant 1h jusqu'à élimination du méthanol sous-produit de réaction. La polycondensation est réalisée en passant le système sous vide (20mbar) durant 5h entre 160°C et 180°C.
Après refroidissement, le milieu réactionnel est dissout dans du dichlorométhane et précipité dans le méthanol filtré et séché.

### Exemple 12 : Préparation des copolymères de poly(butylène adipate-co-isophthalatopeptide) (PBA-co-peptide)

La synthèse des copolymères de poly(butylène adipate-co-isophthalatopeptide) avec 0.5% massique d'unités peptidiques sont préparés par polycondensation en deux étapes en présence de catalyseur (Ti(OBu)₄).
La première étape est une transestérification entre les diesters et le 1,4- butanediol en excès pour obtenir des oligomères, la deuxième étape est la polycondensation de ces oligomères pour éliminer le 1,4-butanediol en excès pour former des polyesters à plus haut poids moléculaire (Mw) (Schéma 7).

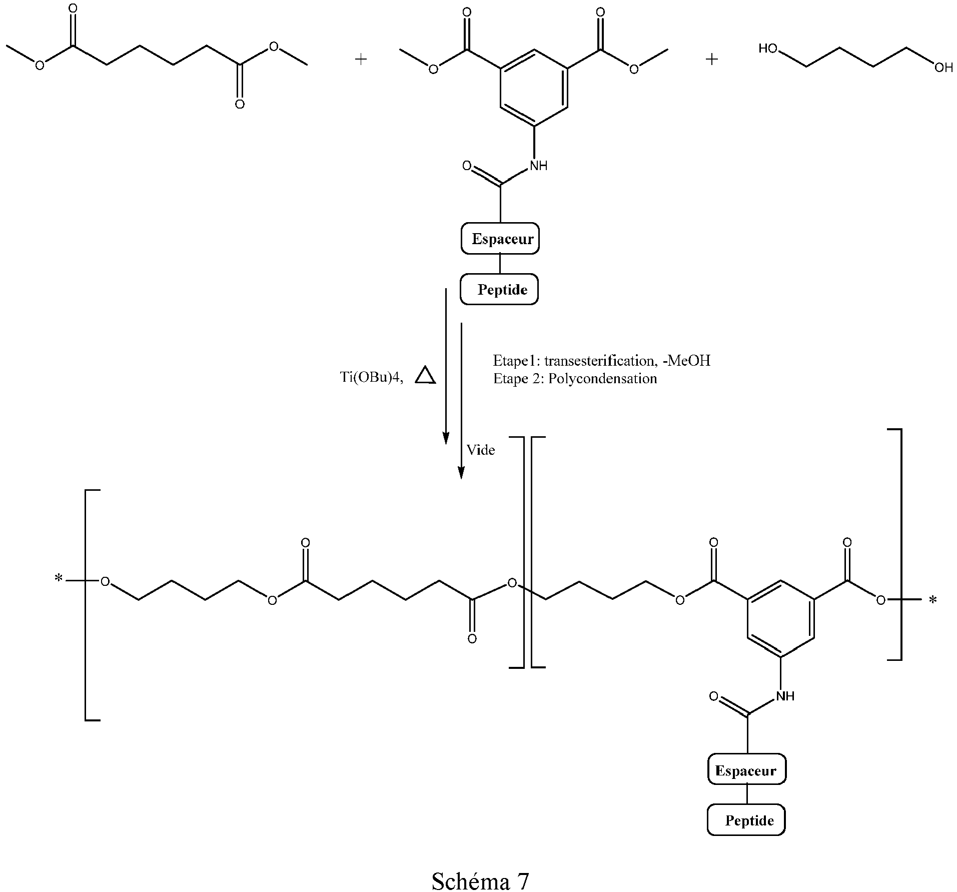

Lors de la première étape qui consiste en une transesterification, les éléments suivants, diméthyl adipate (DMA, 25.33g 1 éq.) et isophthalatopeptide (DMIP peptide = monomère de l'Exemple la, 249mg 0.5% massique ) en quantité stoechiométrique (total en fonctions diester représentant 1 équivalent (DMA +DMIPpeptide=1éq.)) sont ajoutés au 1,4-butanediol en léger excès (BD 15.07g 1.15éq.) et au catalyseur (0.1%) dans un ballon tricol de 250 ml équipé d'un agitateur mécanique étanche, d'une entrée d'azote et d'une tête de distillation relié à un tube gradué pour recueillir le méthanol distillé.
Le réacteur est purgé à l'argon par 3 cycles consécutifs et l'étape de transesterification est réalisée sous atmosphère d'argon à 160°C durant 1h jusqu'à élimination du méthanol sous-produit de réaction. La polycondensation est réalisée en passant le système sous vide (20mbar) durant 5h entre 160°C et 180°C.
Après refroidissement, le milieu réactionnel est dissout dans du dichlorométhane et précipité dans le méthanol, filtré et séché.

### Exemple 13: Préparation des copolymères de poly(butylène succinate-co-téréphthalate-co-isophthalatopeptide)

La synthèse des copolymères de poly(butylène succinate-co-téréphthalate-co-isophthalatopeptide) sont préparés par polycondensation en deux étapes en présence de catalyseur (Ti(OBu)₄).
La première étape est une transestérification entre les diesters et le 1,4- butanediol en excès pour obtenir des oligomères, la deuxième étape est la polycondensation de ces oligomères pour éliminer le 1,4-butanediol en excès pour former des polyesters à haut poids moléculaire (Mw) (Schéma 8).

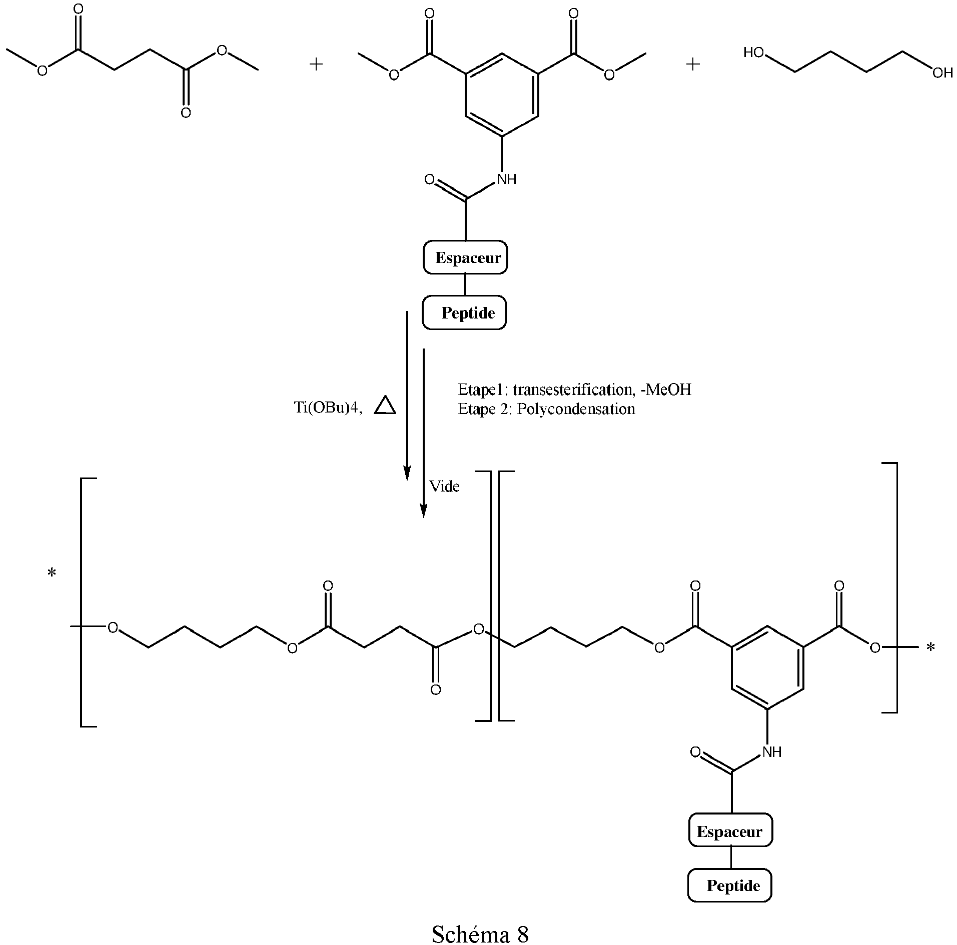

Lors de la première étape qui consiste en une transesterification, les éléments suivants, diméthyl succinate (DMS, 21.25g 1 éq.) et isophthalatopeptide (DMIP peptide= monomère de l'Exemple la, 223mg 0.5% massique ) en quantité stoechiométrique (total en fonctions diester représentant 1 équivalent (DMS +DMIPpeptide=1éq.)) sont ajoutés au 1,4-butanediol en léger excès (BD 15.07g 1.15éq.) et au catalyseur (0.1%) dans un ballon tricol de 250 ml équipé d'un agitateur mécanique étanche, d'une entrée d'azote et d'une tête de distillation relié à un tube gradué pour recueillir le méthanol distillé.
Le réacteur est purgé à l'argon par 3 cycles consécutifs et l'étape de transesterification est réalisée sous atmosphère d'argon à 160°C durant 1h jusqu'à élimination du méthanol sous-produit de réaction. La polycondensation est réalisée en passant le système sous vide (20mbar) durant 5h entre 160°C et 180°C.

Après refroidissement, le milieu réactionnel est dissout dans du dichlorométhane et précipité dans le méthanol, filtré et séché.

### Exemple 14: Préparation de polymères polyuréthane

Le polyuréthane est produit ici par polyaddition en faisant réagir des diisocyanates avec des diols.
Dans un réacteur sous atmosphère d'argon sont introduit le 4,4'-diisocyanate de diphénylméthylène (4.4 MDI 10g 1.05 éq.) et le catalyseur dilaurate de dibutylétain (DBTL 126 mg 0.05%) dans 50mml de diméthylacétamide anhydre à 0°C. Le bis(4-hydroxybutyl) terephthalate (278mg) (optionnel) et le 1,4 butanediol (1.71g 0.5 éq.) en solution dans 10 ml de DMAc anhydre sont introduit goutte à goutte et la réaction est agitée à température ambiante pendant 30 minutes. Le polyytetrahydrofuran 1000 (19g 0.5 éq.) est alors introduit goutte à goutte et la réaction est agitée à 50°C pendant 2 heures. 2 ml d'éthanol sont introduit dans le milieu réactionnel et la solution est agitée 1h supplémentaire. Après refroidissement, le milieu réactionnel est précipité dans l'éther diéthylique, filtré et séché.

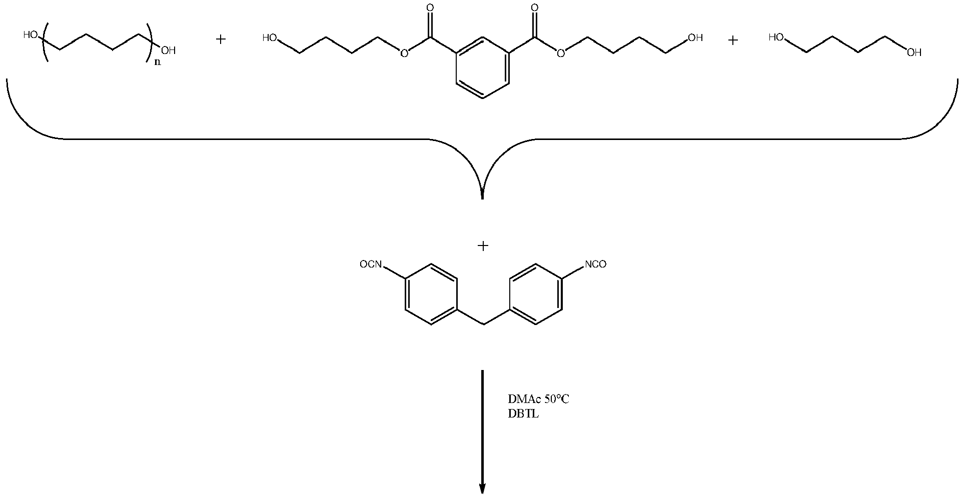

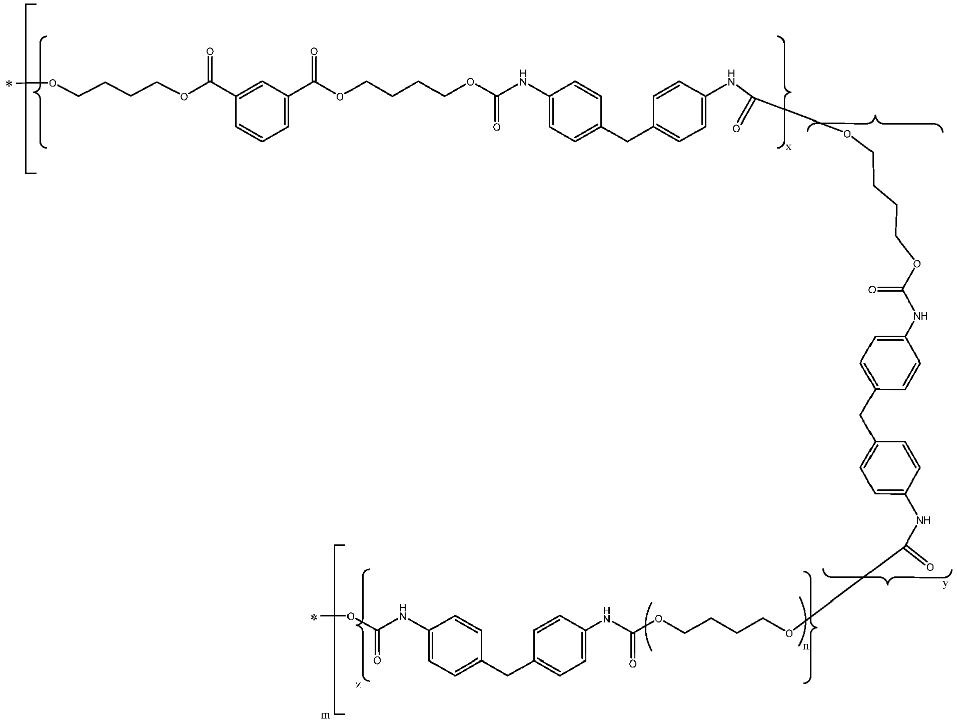

### Exemple 15: Préparation de polymères polyuréthane peptidique

Le polyuréthane est produit par polyaddition en faisant réagir des diisocyanates avec des diols. Dans un réacteur sous atmosphère d'argon sont introduit le 4,4'-diisocyanate de diphénylméthylène (4.4 MDI 10g 1.05 éq) et le catalyseur dilaurate de dibutylétain (DBTL 126 mg 0.05%) dans 50mml de diméthylacétamide anhydre à 0°C. Le bis(4-hydroxybutyl) isophthalato-peptide (exemple 4) (767 mg 0.5% massique) et le 1,4 butanediol en solution dans 10 ml de DMAc anhydre sont introduit goutte à goutte et la réaction est agitée à température ambiante pendant 30 minutes. Le polyytetrahydrofurane 1000 est alors introduit goutte à goutte et la réaction est agitée à 50°C pendant 2 heures. 2 ml d'ethanol sont introduit dans le milieu réactionnel et la solution est agitée 1h supplémentaire. Après refroidissement, le milieu réactionnel est précipité dans l'éther diéthylique, filtré et séché.

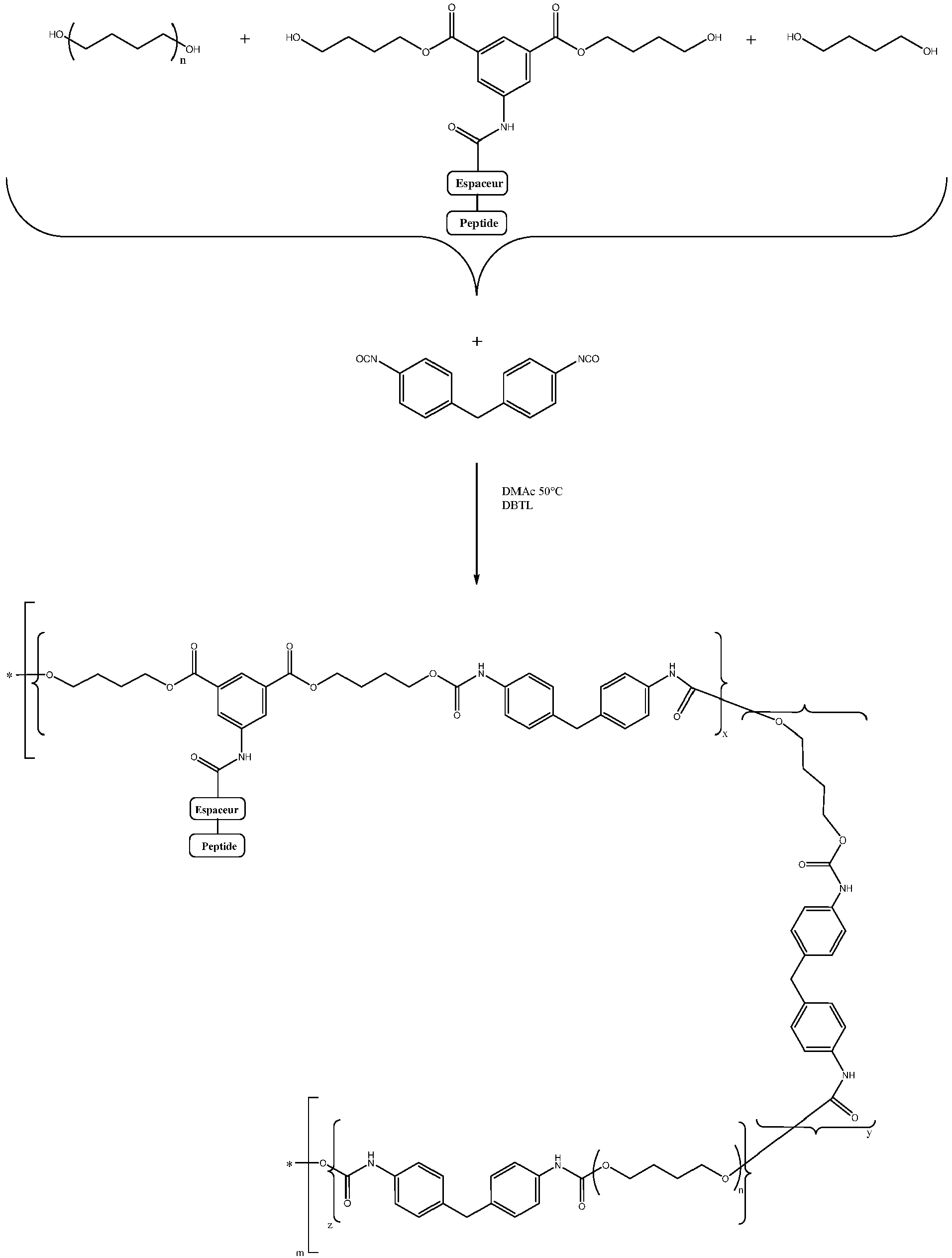

### Exemple 16: Utilisation d'un polymère selon l'invention dans des applications antimicrobiennes

### • Mise en forme de disques et d'haltères

La mise en forme des disques et d'haltère a été réalisée via une micro presse de la marque Zamak Mercator (IM15). Les matériaux sont introduits de façon manuelle dans un fourreau thermo-régulé. Un piston est ensuite introduit dans ce fourreau puis placé dans l'axe du piston pneumatique. Un temps de chauffe est respecté en fonction des caractéristiques de chaque matériau. Puis le matériau est injecté à travers un moule conique pour former les éprouvettes souhaitées. Une fois refroidit le moule est ensuite retiré et les éprouvettes démoulées.

PBS : Après un étuvage à 60°C, des pastilles ont été injectées à 110-115°C avec une température de moule de 60°C, une pression de 6 bars et des temps de chauffe et de refroidissement de 50s et 10s respectivement.

PBAT: Après un étuvage à 60°C, des pastilles de PBAT ont été injectées à 100°C ou 80°C avec une température de moule de 20°C, une pression de 4 bars et des temps de chauffe et de refroidissement de 60s et 10s respectivement.

### • Tests antibactériens selon la norme ISO 22196:2011

L'activité antibactérienne selon la norme ISO 22196:2011 est destinée à évaluer l'activité antimicrobienne de produits plastiques ou de surfaces non poreuses traités par des agents antimicrobiens. La méthode employée dans cette étude se focalise uniquement sur l'évaluation de l'activité antibactérienne.

Cette étude est destinée à évaluer l'activité antibactérienne du matériau antibactérien PBAT-co-peptide vis-à-vis de sa référence sans traitement selon les recommandations de la norme ISO 22196: 20111 pour un temps de contact de 24 heures:

### - Matériau PE (disque de 2,2 cm de diamètre)

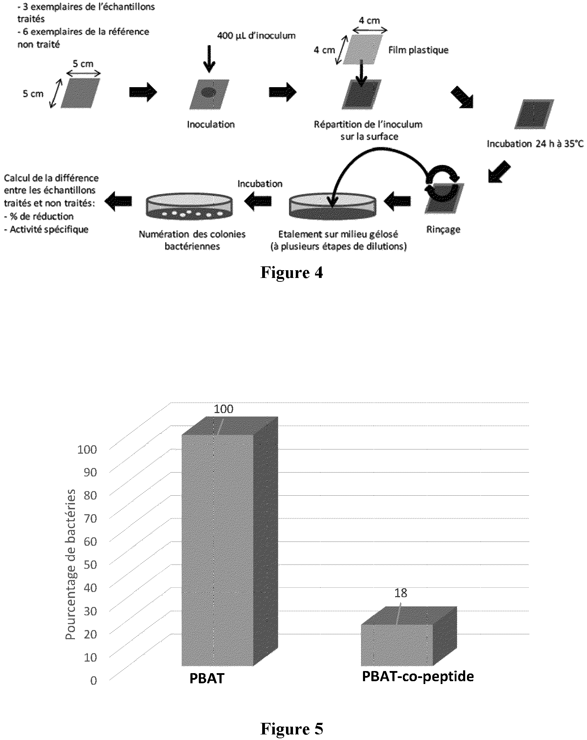

Cette étude utilise idéalement des échantillons de 5 cm x 5 cm sur lesquels une concentration connue du microorganisme à tester est déposée. Après une incubation de 24 heures à 35°C, la quantité de microorganismes viables est évaluée par la technique de dénombrement sur milieu gélosé. La comparaison des concentrations bactériennes obtenues entre le matériau traité et le matériau non traité permet de définir l'activité antibactérienne de la formulation testée. La Figure 4 schématise le principe des différentes étapes de l'analyse sur un échantillon de 5 cm x 5 cm.

L'étude menée ici a été réalisée sur des échantillons sous forme de disques d'un diamètre de 2.2 cm ; Les tests ont été menés vis-à-vis des souches bactériennes prescrites par la norme et très fréquemment impliquées dans des infections, *Escherichia coli* ATCC 8739 et *Staphylococcus aureus* ATCC 6538P.

Les résultats ont pu montrer une inhibition significative avec une réduction de la croissance *Staphylococcus aureus* de 81% (Tableau 1 et Figure 5).

**Tableau 1**

| | | |
|---|---|---|
| Bacterial strain | PBAT | PBAT-co-peptide |
| *Staphylococcus aureus* | 2.73.10⁵ | 5.06.10⁴ |

## Revendications

1. Composé polymère comprenant :
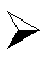 des motifs répétitifs de formule (I) : 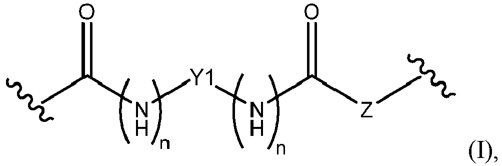 dans lequel
o n vaut 0 ou 1 ;
o Y1 représente un radical choisi parmi un (C₁-C4₁₂)alkyle, un aryle, et un aryle-(Ci-C₁₂)alkyle-aryle ; et
o Z représente un radical choisi parmi :
▪ un groupe représenté par la formule (la) suivante : 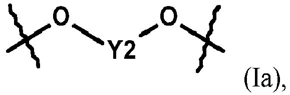 dans lequel Y2 représente un (C₁-C4₁₂)alkyle, aryle, ou polyalkylène glycol ; et
▪ un groupe représenté par la formule (Ib) suivante : 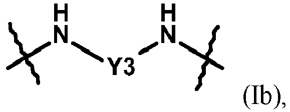 dans lequel Y3 représente un (C₁-C4₁₂)alkyle, aryle, ou polyalkylène glycol ;
et dans lequel au moins un de Y1, Y2, et Y3 est relié à un résidu peptidique via un résidu fonctionnel, le résidu fonctionnel et le résidu peptidique étant éventuellement séparés par un fragment espaceur ;
et 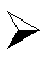 des motifs répétitifs de formule (II) : 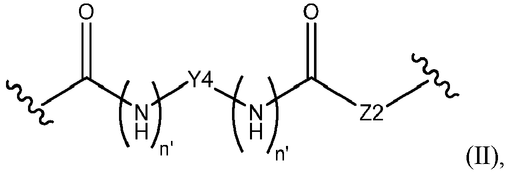 dans lequel
o n' vaut 0 ou 1 ;
o Y4 représente un radical choisi parmi un (C₁-C4₁₂)alkyle, un aryle, et un aryle-(Ci-C₁₂)alkyle-aryle ; et
o Z2 représente un radical choisi parmi :
▪ un groupe représenté par la formule (IIa) suivante : 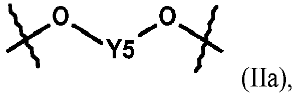 dans lequel Y5 représente un (C₁-C4₁₂)alkyle, aryle, ou polyalkylène glycol ; et
▪ un groupe représenté par la formule (IIb) suivante : 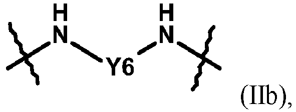 dans lequel Y6 représente un (C₁-C4₁₂)alkyle, aryle ou polyalkylène glycol,
sous réserve que, lorsque n et n' valent 0 et que Y3 est relié à un résidu peptidique via un résidu fonctionnel, le polymère présente au moins une extrémité de formule (E10) ou (E40) : 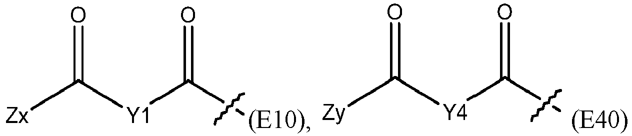 dans lesquelles, Zx et Zy représentent indépendamment un (C₁-C₁₂)alkyle-O- ou aryle-O-.

2. Composé polymère selon la revendication 1, dans lequel :
∘ n = 0;
o Z est un groupe représenté par la formule (la) suivante : 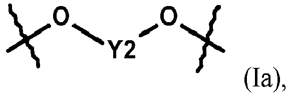 dans lequel Y2 représente un (C₁-C4₁₂)alkyle, aryle, ou polyalkylène glycol ; et
∘ n' =0;
∘ Z2 est un groupe représenté par la formule (IIa) suivante : 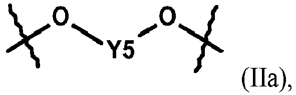 dans lequel Y5 représente un (C₁-C4₁₂)alkyle, aryle, ou polyalkylène glycol ;
et dans lequel au moins un de Y1 et Y2 est relié à un résidu peptidique via un résidu fonctionnel, le résidu fonctionnel et le résidu peptidique étant éventuellement séparés par un fragment espaceur.

3. Composé polymère selon la revendication 1, dans lequel :
∘ n = 0 ;
o Z est un groupe représenté par la formule (Ib) suivante : 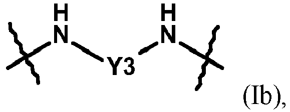 dans lequel Y3 représente un (C₁-C4₁₂)alkyle, aryle ou polyalkylène glycol ; et
∘ n' = 0 ;
o Z2 est un groupe représenté par la formule (IIb) suivante : 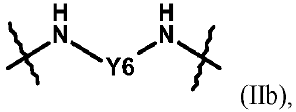 dans lequel Y6 représente un (C₁-C4₁₂)alkyle, aryle, ou polyalkylène glycol, et
dans lequel au moins un de Y1 et Y3 est relié à un résidu peptidique via un résidu fonctionnel, le résidu fonctionnel et le résidu peptidique étant éventuellement séparés par un fragment espaceur.

4. Composé polymère selon l'une quelconque des revendications 1 à 3, dans lequel le résidu peptidique est un résidu du peptide Arg-Phe-Arg-Phe-Arg-Phe-Arg-Phe, Phe-Arg-Phe-Arg-Phe-Arg-Phe-Arg, ou Arg-Arg-Palm, où Palm désigne un groupe palmityle.

5. Composé polymère selon l'une quelconque des revendications 1 à 4, ledit fragment espaceur est de formule -(X1)-(X2)-(X3)-, dans laquelle X1 et X3 représentent chacun indépendamment -NH- ou -CO-, et X2 représente un (C₁-C4₁₂)alkyle, aryle ou un polyalkylène glycol.

6. Composé monomère représenté par la formule (III-1) suivante : 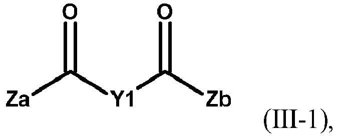 dans lequel
o Y1 représente un radical choisi parmi un (C₁-C4₁₂)alkyle, un aryle, et un aryle-(Ci-C₁₂)alkyle-aryle ;
o Za représente un radical choisi parmi :
▪ un (C₁-C₁₂)alkyle-O-, un aryle-O-, un chlore ;
▪ un groupe représenté par la formule (IIIa) suivante : 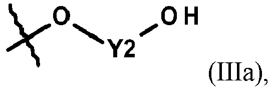 dans lequel Y2 représente un (C₁-C4₁₂)alkyle, aryle, ou un polyalkylène glycol ; et
o Zb représente un radical choisi parmi :
▪ un (C₁-C₁₂)alkyle-O-, un aryle-O-, un chlore ;
▪ un groupe représenté par la formule (IIIa) suivante : 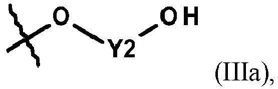 dans lequel Y2 représente un (C₁-C4₁₂)alkyle, aryle, ou un polyalkylène glycol ; et
▪ un groupe représenté par la formule (IIIb) suivante : 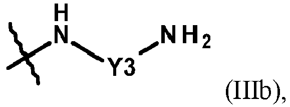 dans lequel Y3 représente un (C₁-C4₁₂)alkyle, aryle, ou un polyalkylène glycol ;
et dans lequel au moins un de Y1, Y2, et Y3 est relié à un résidu peptidique via un résidu fonctionnel, le résidu fonctionnel et le résidu peptidique étant éventuellement séparés par un fragment espaceur,
sous réserve que, lorsque Y3 est relié à un résidu peptidique via un résidu fonctionnel, le résidu fonctionnel et le résidu peptidique étant éventuellement séparés par un fragment espaceur, Za est choisi parmi un (C₁-C₁₂)alkyle-O- et un aryle-O-.

7. Composé monomère selon la revendication 6, dans lequel Za et Zb représentent chacun indépendamment un chlore, (C₁-C₁₂)alkyle-O-, aryle-O-, et dans lequel Y1 est relié à un résidu peptidique via un résidu fonctionnel, le résidu fonctionnel et le résidu peptidique étant éventuellement séparés par un fragment espaceur.

8. Composé monomère selon la revendication 6, dans lequel Za et Zb sont un groupe représenté par la formule (IIIa) suivante : 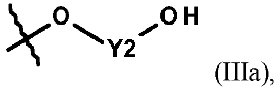 dans lequel Y2 représente un (C₁-C4₁₂)alkyle, aryle, ou polyalkylène glycol, et dans lequel au moins un de Y1 et Y2 est relié à un résidu peptidique via un résidu fonctionnel, le résidu fonctionnel et le résidu peptidique étant éventuellement séparés par un fragment espaceur.

9. Composé monomère selon la revendication 6, dans lequel :
∘ Za est un (C₁-C₁₂)alkyle-O-, un aryle-O-, ou un chlore ; et
o Zb est un groupe représenté par la formule (IIIb) suivante : 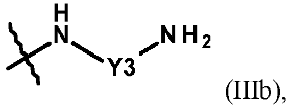 dans lequel Y3 représente un (C₁-C4₁₂)alkyle, aryle, ou polyalkylène glycol, et dans lequel au moins un de Y1 et Y3 est substitué par un peptide et sous réserve que, lorsque Y3 est relié à un résidu peptidique via un résidu fonctionnel, le résidu fonctionnel et le résidu peptidique étant éventuellement séparés par un fragment espaceur, Za est choisi parmi un (Ci-C₁₂)alkyle-O- et un aryle-O-.

10. Composé monomère représenté par la formule (III-2) suivante :
HO-Y2-OH (111-2),
où Y2 représente un (C₁-C4₁₂)alkyle, aryle, ou polyalkylène glycol, où Y2 est relié à un résidu peptidique via un résidu fonctionnel, le résidu fonctionnel et le résidu peptidique étant éventuellement séparés par un fragment espaceur.

11. Composé monomère selon l'une quelconque des revendications 6 à 10, dans lequel le résidu peptidique est un résidu du peptide Arg-Phe-Arg-Phe-Arg-Phe-Arg-Phe, Phe-Arg-Phe-Arg-Phe-Arg-Phe-Arg, ou Arg-Arg-Palm, où Palm désigne un groupe palmityle.

12. Composé monomère selon l'une quelconque des revendications 6 à 11, dans lequel ledit fragment espaceur est de formule -(X1)-(X2)-(X3)-, dans laquelle X1 et X3 représentent chacun indépendamment -NH- ou -CO-, et X2 représente un (C₁-C4₁₂)alkyle, aryle, ou polyalkylène glycol.

13. Procédé de préparation d'un composé polymère tel que défini dans l'une quelconque des revendications 1 à 5, comprenant une réaction de polycondensation en milieu fondu, mettant en jeu :
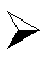 au moins un composé monomère choisi parmi :
∘ un composé monomère de formule Za-C(O)-Y1-C(O)-Zb, où :
- Y1 représente un radical choisi parmi un (C₁-C4₁₂)alkyle, un aryle, et un aryle-(C₁-C₁₂)alkyle-aryle ;
- Za représente un radical choisi parmi un (C₁-C₁₂)alkyle-O-, un aryle-O-, un chlore, et un groupe de formule (IIIa) : 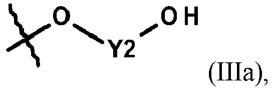 où Y2 représente un (C₁-C4₁₂)alkyle, aryle, ou polyalkylène glycol ;
- Zb représente un radical choisi parmi un (C₁-C₁₂)alkyle-O-, un aryle-O-, un chlore, un groupe de formule (IIIa) et un groupe de formule (IIIb) ; 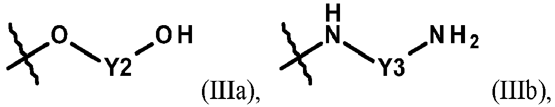 où Y2 représente un (C₁-C4₁₂)alkyle, aryle ou polyalkylène glycol, et Y3 représente un (Ci-C₁₂)alkyle, aryle ou polyalkylène glycol ;
∘ OCN-Y1-NCO, où Y1 représente un (C₁-C4₁₂)alkyle, aryle, ou un aryle-(C₁-C₁₂)alkyle-aryle ;
∘ HO-Y2-OH, où Y2 représente un (C₁-C4₁₂)alkyle, aryle, ou polyalkylène glycol, et
∘ H₂N-Y3-NH₂, où Y3 représente un (C₁-C4₁₂)alkyle, aryle ou polyalkylène glycol ;
où au moins un de Y1, Y2, et Y3 est relié à un résidu peptidique via un résidu fonctionnel, le résidu fonctionnel et le résidu peptidique étant éventuellement séparés par un fragment espaceur;
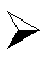 et au moins un composé monomère choisi parmi :
∘ un composé monomère représenté par la formule (V) suivante : 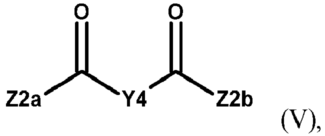 dans lequel
- Y4 représente un radical choisi parmi un (C₁-C4₁₂)alkyle, un aryle, et un aryle-(C₁-C₁₂)alkyle-aryle ;
- Z2a représente un radical choisi parmi :
▪ un (C₁-C₁₂)alkyle-O-, un aryle-O-, un chlore ; et
▪ un groupe représenté par la formule (Va) suivante : 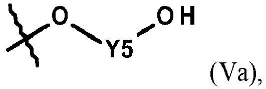 dans lequel Y5 représente un (C₁-C4₁₂)alkyle, aryle, ou polyalkylène glycol ; et
- Z2b représente un radical choisi parmi :
▪ un (C₁-C₁₂)alkyle-O-, un aryle-O-, un chlore ;
▪ un groupe représenté par la formule (Va) suivante : 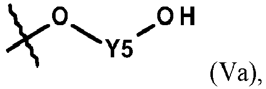 dans lequel Y5 représente un (C₁-C4₁₂)alkyle, aryle, ou polyalkylène glycol ; et
▪ un groupe représenté par la formule (Vb) suivante : 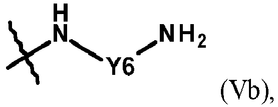 dans lequel Y6 représente un (C₁-C4₁₂)alkyle, aryle, ou polyalkylène glycol,
∘ OCN-Y4-NCO, où Y4 représente un (C₁-C4₁₂)alkyle, aryle, ou un aryle-(C₁-C₁₂)alkyle-aryle,
∘ HO-Y5-OH, où Y5 représente un (C₁-C4₁₂)alkyle, aryle, ou polyalkylène glycol et
∘ H₂N-Y6-NH₂, où Y6 représente un (C₁-C4₁₂)alkyle, aryle, ou polyalkylène glycol.

14. Utilisation d'un composé polymère tel que défini dans l'une quelconque des revendications 1 à 5, dans la fabrication d'un matériau ou d'un article.

15. Matériau comprenant un composé polymère tel que défini dans l'une quelconque des revendications 1 à 5.

16. Article comprenant un composé polymère tel que défini dans l'une quelconque des revendications 1 à 5.
